# EUROPEAN PATENT APPLICATION

(11) **EP 2 518 059 A1**
(43) Date of publication of application: **31.10.2012**
(21) Application number: 10838964.4
(22) Date of filing: 22.12.2010
(51) Int. Cl.: C07D 265/20, A61K 31/536, A61K 31/54, A61K 31/541, A61K 31/5415, A61P 25/28, A61P 43/00, C07D 279/06, C07D 413/12, C07D 417/12

(54) **4-AMINO-1,3-THIAZINE OR OXAZINE DERIVATIVE**

(30) Priority: 24.12.2009 JP 2009293420
(71) Applicant: Shionogi & Co., Ltd., Osaka-shi, Osaka 5410045 (JP)
(72) Inventor: SUZUKI, Shinji, Osaka 561-0825 (JP); KOORIYAMA, Yuuji, Osaka-shi Osaka 541-0045 (JP)
(74) Representative: Vossius & Partner
(86) International application number: PCT/JP2010/007449
(87) International publication number: WO 2011/077726

(57) **Abstract**

The present invention provide a medicament for treating the diseases induced by production, secretion or deposition of amyloid-β proteins, for example, a compound of the following formula (I) wherein R¹ R^{2a}, R^{2b}, R^{3a}, R^{3b} R^{4a}, R^{4b}, ring A, Y and the dotted line are defined in the specification, its pharmaceutically acceptable salt or a solvate thereof.

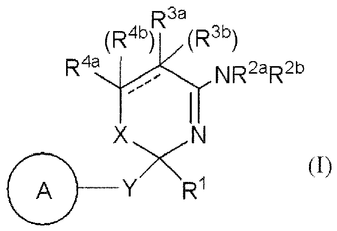

## Description

### [Technical field]

The present invention relates to a compound which has amyloid β production inhibitory activity, and is useful as an agent for treating or preventing disease induced by production, secretion and/or deposition of amyloid β protein.

### [Background Art]

In the brain of Alzheimer's patient, the peptide composed of about 40 amino acids residue as is called amyloid β protein, that accumulates to form insoluble specks (senile specks) outside nerve cells is widely observed. It is concerned that this senile specks kill nerve cells to cause Alzheimer's disease, so the therapeutic agents for Alzheimer's disease, such as decomposition agents of amyloid β protein and amyloid vaccine, are under investigation.

Secretase is an enzyme which cleaves a protein called amyloid β precursor protein (APP) in cell and produces amyloid β protein. The enzyme which controls the production of N terminus of amyloid β protein is called as β-secretase (beta-site APP-cleaving enzyme 1, BACE1). It is thought that inhibition of this enzyme leads to reduction of producing amyloid β protein and that the therapeutic agent for Alzheimer's disease will be created due to the inhibition.

Patent Literatures 1 to 11 and Non-patent Literatures 5 and 6 describe the compounds which are structurally similar to the compounds of the present invention, and the compounds can be the therapeutic agent for Alzheimer's disease or Alzheimer's disease-related symptom. However, all compounds substantively disclosed in these literatures have different core structures from the present invention. Patent Literature 12 describes the compounds which are structurally similar to the compounds of the present invention, however, the effect for Alzheimer's disease is not suggested. Non-patent Literatures 1 to 4 describe the compounds which are structurally similar to the compounds of the present invention, however, there is no suggestion that these compounds have a medical use.

### [Prior Art Literatures]

### [Patent Literatures]

[Patent Literature 1] International Patent Application Publication WO2007/058583
[Patent Literature 2] International Patent Application Publication WO2007/049532
[Patent Literature 3] International Patent Application Publication WO2008/133273
[Patent Literature 4] International Patent Application Publication WO2008/133274
[Patent Literature 5] International Patent Application Publication WO2009/103626
[Patent Literature 6] International Patent Application Publication WO2009/134617
[Patent Literature 7] International Patent Application Publication WO2010/019392
[Patent Literature 8] International Patent Application Publication WO2010/019393
[Patent Literature 9] International Patent Application Publication WO2009/151098
[Patent Literature 10] International Patent Application Publication WO2010/047372
[Patent Literature 11] US Patent Application Publication US 2010/0261727
[Patent Literature 12] US Patent 5328915

### [Non-Patent Literatures]

[Non-Patent Literature 1] Chemistry of Heterocyclic Compounds (2001),37(4),522-523
[Non-Patent Literature 2] Russian Journal of Organic Chemistry (2000),36(12), 1739-1742
[Non-Patent Literature 3] Russian Journal of Organic Chemistry (1997),33(1),96-102
[Non-Patent Literature 4] Journal of Organic Chemistry (1983),48(4),623-625
[Non-Patent Literature 5] Bioorganic & Medicinal Chemistry Letters (2010),20(3),1269-1271
[Non-Patent Literature 6] Bioorganic & Medicinal Chemistry Letters (2010), 20(7), 2279-2282

### [Disclosure of Invention]

### [Problems to be solved by the Invention]

The present invention provides compounds which have reducing effects to produce amyloid β protein, especially β secretase inhibitory activity, and are useful as an agent for treating or preventing disease induced by production, secretion and/or deposition of amyloid β protein.

### [Means to Solve the Problems]

The present invention, for example, provides the inventions described in the following items.

(1) A compound of formula (I): 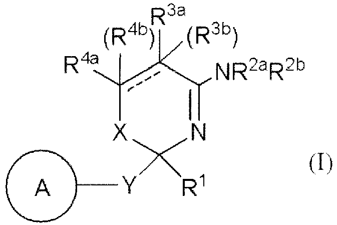 wherein
   -X- is -O-, -S-, -SO-, -SO₂- or -N(R^{x})-,
   R^{x} is hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl,
   ring A is a substituted or unsubstituted carbocycle or a substituted or unsubstituted heterocycle, -Y- is substituted or unsubstituted alkenylene, substituted or unsubstituted alkynylene, - (CR⁵R⁶)ₙ-, -(CR⁵R⁶)ₙO(CR⁵R⁶)ₘ-, -(CR⁵R⁶)ₙS(CR⁵R⁶)ₘ-, -(CR⁵R⁶)ₙSO₂(CR⁵R⁶)ₘ-, - (CR⁵R⁶)ₙSO(CR⁵R⁶)ₘ-, -(CR⁵R⁶)ₙC(=O)(CR⁵R⁶)ₘ-, -(CR⁵R⁶)ₙN(R⁷)(CR⁵R⁶)ₘ-, - (CR⁵R⁶)ₙC(=O)N(R⁷)(CR⁵R⁶)ₘ-, or -(CR⁵R⁶)ₙN(R⁷)C(=O)(CR⁵R⁶)ₘ-,
   R⁵ and R⁶ are each independently, hydrogen, halogen, hydroxy, nitro, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted carbamoyloxy, substituted or unsubstituted thiocarbamoyl, substituted or unsubstituted sulfamoyl, substituted or unsubstituted aminosulfinyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl,
   R⁷ is hydrogen, substituted or unsubstituted alkyl, substituted or unsubstituted alkenyl, substituted or unsubstituted alkynyl or substituted or unsubstituted acyl,
   n and m are each independently an integer of 0 to 3,
   R¹ is substituted or unsubstituted alkyl, substituted or unsubstituted alkenyl, substituted or unsubstituted alkynyl, substituted or unsubstituted acyl, cyano, carboxy, substituted or unsubstituted alkoxycarbonyl, substituted or unsubstituted alkenyloxycarbonyl, substituted or unsubstituted alkynyloxycarbonyl, substituted or unsubstituted carbamoyl, substituted or unsubstituted thiocarbamoyl, a substituted or unsubstituted carbocyclic group or a substituted or unsubstituted heterocyclic group,
   R^{2a} and R^{2b} are each independently hydrogen, substituted or unsubstituted alkyl, substituted or unsubstituted acyl, substituted or unsubstituted alkoxycarbonyl or substituted or unsubstituted carbamoyl,
   R^{3a}, R^{3b}, R^{4a} and R^{4b} are each independently
   hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl,
   R^{3a} and R^{3b} together with the carbon atom to which they are attached may form a substituted or unsubstituted carbocycle or a substituted or unsubstituted heterocycle,
   R^{4a} and R^{4b} together with the carbon atom to which they are attached may form a substituted or unsubstituted carbocycle or a substituted or unsubstituted heterocycle,
   R^{3a} and R^{4a} together with the carbon atom to which they are attached may form a substituted or unsubstituted carbocycle or a substituted or unsubstituted heterocycle,
   dashed line means the presence or absence of a bond,
   when dashed line means the presence of a bond, then R^{3b} and R^{4b} are absent,
   provided that when -X- is -O- or -N(R^{x})-, and R^{3a}, and R^{4a} together with the carbon atom to which they are attached form a substituted or unsubstituted benzene ring, then ring A is not (i) phenyl substituted only with halogen or (ii) phenyl substituted with substituted or unsubstituted phenyl at m-position,
   its pharmaceutically acceptable salt or a solvate thereof.
(1') A compound of formula (I): 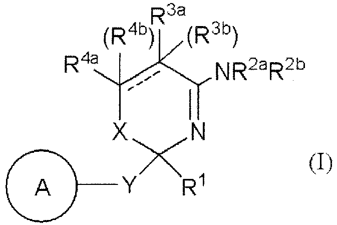 wherein
   -X- is -O-, -S-, -SO-, -SO₂- or -N(R^{x})-,
   R^{x} is hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclyloxycarbonyl, substituted or unsubstituted heterocyclylsulfinyl or substituted or unsubstituted heterocyclylsulfonyl,
   ring A is a substituted or unsubstituted carbocycle or a substituted or unsubstituted heterocycle, -Y- is substituted or unsubstituted alkenylene, substituted or unsubstituted alkynylene, - (CR⁵R⁶)ₙ-, -(CR⁵R⁶)ₙO(CR⁵R⁶)ₘ-, -(CR⁵R⁶)ₙS(CR⁵R⁶)ₘ-, -(CR⁵R⁶)ₙSO₂(CR⁵R⁶)ₘ-, - (CR⁵R⁶)ₙSO(CR⁵R⁶)ₘ-, -(CR⁵R⁶)ₙC(=O)(CR⁵R⁶)ₘ-, -(CR⁵R⁶)ₙN(R⁷)(CR⁵R⁶)ₘ-, - (CR⁵R⁶)ₙC(=O)N(R⁷)(CR⁵R⁶)ₘ-, or -(CR⁵R⁶)ₙN(R⁷)C(=O)(CR⁵R⁶)ₘ-,
   R⁵ and R⁶ are each independently, hydrogen, halogen, hydroxy, nitro, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted carbamoyloxy, substituted or unsubstituted thiocarbamoyl, substituted or unsubstituted sulfamoyl, substituted or unsubstituted aminosulfinyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclyloxycarbonyl, substituted or unsubstituted heterocyclylthio, substituted or unsubstituted heterocyclylsulfinyl or substituted or unsubstituted heterocyclylsulfonyl,
   R⁷ is hydrogen, substituted or unsubstituted alkyl, substituted or unsubstituted alkenyl, substituted or unsubstituted alkynyl or substituted or unsubstituted acyl,
   n and m are each independently an integer of 0 to 3,
   R¹ is substituted or unsubstituted alkyl, substituted or unsubstituted alkenyl, substituted or unsubstituted alkynyl, substituted or unsubstituted acyl, cyano, carboxy, substituted or unsubstituted alkoxycarbonyl, substituted or unsubstituted alkenyloxycarbonyl, substituted or unsubstituted alkynyloxycarbonyl, substituted or unsubstituted carbamoyl, substituted or unsubstituted thiocarbamoyl, a substituted or unsubstituted carbocyclic group or a substituted or unsubstituted heterocyclic group,
   R^{2a} and R^{2b} are each independently hydrogen, substituted or unsubstituted alkyl, substituted or unsubstituted acyl, substituted or unsubstituted alkoxycarbonyl or substituted or unsubstituted carbamoyl,
   R^{3a}, R^{3b}, R^{4a} and R^{4b} are each independently
   hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclyloxycarbonyl, substituted or unsubstituted heterocyclylsulfinyl or substituted or unsubstituted heterocyclylsulfonyl,
   R^{3a} and R^{3b} together with the carbon atom to which they are attached may form a substituted or unsubstituted carbocycle or a substituted or unsubstituted heterocycle,
   R^{4a} and R^{4b} together with the carbon atom to which they are attached may form a substituted or unsubstituted carbocycle or a substituted or unsubstituted heterocycle,
   R^{3a} and R^{4a} together with the carbon atom to which they are attached may form a substituted or unsubstituted carbocycle or a substituted or unsubstituted heterocycle,
   dashed line means the presence or absence of a bond,
   when dashed line means the presence of a bond, then R^{3b} and R^{4b} are absent,
   provided that when X- is -O- or -N(R^{x})-, and R^{3a} and R^{4a} together with the carbon atom to which they are attached form a substituted or unsubstituted benzene ring, then ring A is not (i) phenyl substituted only with halogen or (ii) phenyl substituted with substituted or unsubstituted phenyl at m-position,
   its pharmaceutically acceptable salt or a solvate thereof.
(2) The compound according to the item (1) or (1') wherein Y is -(CR⁵R⁶)ₙ- and n is 0, its pharmaceutically acceptable salt or a solvate thereof.
(3) The compound according to the item (2) wherein -X- is -O- or -S-, its pharmaceutically acceptable salt or a solvate thereof.
(4) The compound according to any one of the items (1), (1'), (2) and (3) wherein ring A is a group of formula: 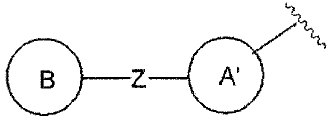
   wherein ring A' and ring B are each independently a substituted or unsubstituted carbocycle or a substituted or unsubstituted heterocycle, -Z- is -L¹-C(=O)N(R⁸)-L²-, -L¹-N(R⁸)C(=O)-L²- or -L¹-N(R⁸)-L²-,
   L¹ and L² are each independently a bond, substituted or unsubstituted alkylene, substituted or unsubstituted alkenylene or substituted or unsubstituted alkynylene,
   R⁸ is hydrogen, substituted or unsubstituted alkyl, substituted or unsubstituted alkenyl, substituted or unsubstituted alkynyl or substituted or unsubstituted acyl,
   provided that -Z- is -L¹-C(=O)N(R⁸)-L²-, -L¹-N(R⁸)C(=O)-L²- or -L¹-N(R⁸)-L²-, then L¹ bonds to the ring B, and, L² bonds to the ring A',
   its pharmaceutically acceptable salt or a solvate thereof.
(5) The compound according to the item (4) wherein -Z- is -C(=O)N(R⁸)-, its pharmaceutically acceptable salt or a solvate thereof.
(6) The compound according to the item (4) or (5) wherein ring A' is substituted or unsubstituted benzene, its pharmaceutically acceptable salt or a solvate thereof.
(7) The compound according to any one of the items (4) to (6) wherein ring B is substituted or unsubstituted pyridine, substituted or unsubstituted pyrimidine or substituted or unsubstituted pyrazine, its pharmaceutically acceptable salt or a solvate thereof.
(8) The compound according to any one of the items (1), (1') and (2) to (7) wherein R^{3a}, R^{3b}, R^{4a} and R^{4b} are all hydrogen, its pharmaceutically acceptable salt or a solvate thereof.
(9) The compound according to any one of the items (1), (1') and (2) to (7) wherein R^{3a} and R^{4a} together with the carbon atom to which they are attached form a substituted or unsubstituted benzene ring, its pharmaceutically acceptable salt or a solvate thereof.
(10) The compound according to any one of the items (1), (1') and (2) to (9) wherein R¹ is C1 to C3 unsubstituted alkyl, its pharmaceutically acceptable salt or a solvate thereof.
(11) The compound according to any one of the items (1), (1') and (2) to (10) wherein R^{2a} and R^{2b} are both hydrogen, its pharmaceutically acceptable salt or a solvate thereof.
(12) A pharmaceutical composition comprising the compound according to any one of the items (1), (1') and (2) to (11), its pharmaceutically acceptable salt or a solvate thereof as an active ingredient.
(13) A pharmaceutical composition having BACE1 inhibitory activity comprising the compound according to any one of the items (1), (1') and (2) to (11), its pharmaceutically acceptable salt or a solvate thereof as an active ingredient.
(14) The pharmaceutical composition according to the item (12) or (13), which is a medicament for treating or preventing the diseases induced by production, secretion or deposition of amyloid-β proteins.
(15) The pharmaceutical composition according to the items (12) or (13), which is a medicament for treating or preventing Alzheimer's disease.
(16) A method for inhibiting BACE1 activity comprising administering the compound according to any one of the items (1), (1') and (2) to (11), its pharmaceutically acceptable salt or a solvate thereof.
(17) The compound according to any one of items (1), (1') and (2) to (11), its pharmaceutically acceptable salt or a solvate thereof for use in a method for inhibiting BACE1 activity.
(18) A method for treating or preventing diseases induced by production, secretion or deposition of amyloid-β proteins comprising administering the compound according to any one of items (1) to (11), its pharmaceutically acceptable salt or a solvate thereof.
(19) The compound according to any one of the items (1), (1') and (2) to (11), its pharmaceutically acceptable salt or a solvate thereof for use in a method for treating or preventing diseases induced by production, secretion or deposition of amyloid-β proteins.
(20) A method for treating or preventing Alzheimer's disease comprising administering the compound according to any one of the items (1), (1') and (2) to (11), its pharmaceutically acceptable salt or a solvate thereof.
(21) The compound according to any one of the items (1), (1') and (2) to (11), its pharmaceutically acceptable salt or a solvate thereof for treating or preventing Alzheimer's disease.
(22) Use of the compound according to any one of the items (1), (1') and (2) to (11), or its pharmaceutically acceptable salt or a solvate thereof in the manufacture of a medicament for inhibiting β secretase activity,
(23) Use of the compound according to any one of the items (1), (1') and (2) to (11), or its pharmaceutically acceptable salt or a solvate thereof in the manufacture of a medicament for treating or preventing disease induced by production, secretion or deposition of amyloid β protein,
(24) Use of the compound according to any one of the items (1), (1') and (2) to (11), or its pharmaceutically acceptable salt or a solvate thereof in the manufacture of a medicament for treating or preventing Alzheimer's disease.
(25) A method, a system, an apparatus, a kit or the like for preparing the compound according to any one of the item (1), (1') and (2) to (11), or its pharmaceutically acceptable salt or a solvate thereof.
(26) A method, a system, an apparatus, a kit or the like for preparing the pharmaceutical composition comprising the compound according to any one of the item (1), (1') and (2) to (11), or its pharmaceutically acceptable salt or a solvate thereof.
(27) A method, a system, an apparatus, a kit or the like for using the compound according to any one of the item (1), (1') and (2) to (11), or its pharmaceutically acceptable salt or a solvate thereof.

### [Effect of the Invention]

The compounds of the present invention are useful as an agent for treating or preventing disease induced by production, secretion or deposition of amyloid β protein such as Alzheimer's disease and the like.

### [Best Mode for Carrying Out the Invention]

As used herein, the "halogen" includes fluorine, chlorine, bromine, and iodine.

As used herein, the "alkyl" includes straight or branched alkyl of a carbon number of 1 to 15, for example, a carbon number of 1 to 10, for example, a carbon number of 1 to 6, and for example, a carbon number of 1 to 3. Examples include methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl, and n-decyl.

In the specification, an alkyl part of the "alkoxy", the "halogenoalkyl", the "hydroxyalkyl", the "halogenoalkoxy", the "alkylcarbonyl", the "hydroxyalkoxy", "alkoxycarbonyl", the "halogenoalkoxycarbonyl", the "alkylamino", the "aminoalkyl", the "alkoxyalkoxy", the "alkoxyalkenyloxy", the "alkoxyalkynyl", the "alkylcarbamoyl", the "hydroxyalkylcarbamoyl", the "alkoxyimino", the "alkylthio", the "alkylsulfonyl", the "alkylsulfonylamino", the "alkylsulfonylalkylamino", the "alkylsulfonylimino", the "alkylsulfinylamino", the "alkylsulfinylalkylamino", the "alkylsulfinylimino", the "alkylsulfamoyl", the "alkylsulfinyl", the "carbocyclylalkyl", the "carbocyclylalkoxy", the "carbocyclylalkoxycarbonyl", the "carbocyclylalkylamino", the "carbocyclylalkylcarbamoyl", the "cycloalkylalkyl", the "cycloalkylalkoxy", the "cycloalkylalkylamino", the "cycloalkylalkoxycarbonyl", the "cycloalkylalkylcarbamoyl", the "arylalkyl", the "arylalkoxy", the "arylalkylamino", the "arylalkoxycarbonyl", the "arylalkylcarbamoyl", the "heterocyclylalkyl", the "heterocyclylalkoxy", the "heterocyclylalkylamino", the "heterocyclylalkoxycarbonyl" and "heterocyclylalkylcarbamoyl" is the same as the above alkyl.

In the specification, the "substituted or un substituted alkyl" may be substituted with one or more substituents selected from a substituent group α.

As used herein, the substituent group α is a group consisting of halogen, hydroxy, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfonylimino, alkylsulfinylamino, alkylsulfinylalkylamino, alkylsulfinylimino, cyano, nitro, a carbocyclic group and a heterocyclic group wherein the carbocycle and the heterocycle may be each substituted with one or more substituents selected from a group consisting of halogen, alkyl, hydroxy and alkoxy.

In the specification, examples of the substituent of the "substituted or un substituted alkoxy", the "substituted or un substituted alkoxycarbonyl", the "substituted or un substituted alkylthio", the "substituted or un substituted alkylsulfonyl" and the "substituted or un substituted alkylsulfinyl" include one or more groups selected from the above substituent group α.

In the specification, examples of an embodiment of the "halogenoalkyl," include trifluoromethyl, fluoromethyl and trichloromethyl.

In the specification, examples of an embodiment of the "halogenoalkoxy" include trifluoromethoxy, fluoromethoxy and trichloromethoxy.

In the specification, the "alkylidene" includes a divalent group of the above "alkyl", and examples include methylidene, ethylidene, propylidene, isopropylidene, butylidene, pentylidene and hexylidene.

In the specification, the "alkenyl" includes straight or branched alkenyl of a carbon number of 2 to 15, for example, a carbon number of 2 to 10, for example, a carbon number of 2 to 6 for example, a carbon number of 2 to 4, having one or more double bonds at any available position. Examples include vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl and pentadecenyl.

In the specification, an alkenyl part of the "alkenyloxy", the "alkenyloxycarbonyl", the "alkenylcarbonyl", the "alkoxyalkenyloxy", the "alkenylthio", the "alkenylamino", the "alkenylsulfonyl" and the "alkenylsulfinyl" is the same as the above "alkenyl".

In the specification, the "alkynyl" includes straight or branched alkynyl of a carbon number of 2 to 10, for example, a carbon number of 2 to 8, for example, a carbon number of 3 to 6, having one or more triple bonds at any available position. Examples include ethynyl, propynyl, butynyl, pentynyl, hexynyl, heptynyl, octynyl, nonynyl and decynyl. These may further a double bond at any available position.

In the specification, an alkynyl part of the "alkoxyalkynyl", the "alkynyloxy", the "alkynyloxycarbonyl", the "alkoxyalkynyloxy", the "alkynylthio", the "alkynylsulfinyl", the "alkynylsulfonyl" and the "alkynylamino" is the same as the above "alkynyl".

In the specification, examples of the substituent of the "substituted or unsubstituted alkenyl", the "substituted or unsubstituted alkenyloxy", the "substituted or unsubstituted alkenyloxycarbony", the "substituted or unsubstituted alkenylthio", the "substituted or unsubstituted alkenylsulfinyl", the "substituted or unsubstituted alkenylsulfonyl", the "substituted or unsubstituted alkynyl", the "substituted or unsubstituted alkynyloxy", the "substituted or unsubstituted alkynylthio", the "substituted or unsubstituted alkynyloxycarbony", the "substituted or unsubstituted alkynylsulfinyl" and the "substituted or unsubstituted alkynylsulfonyl" include one or more substituents selected from the above substituent group α.

In the specification, examples of the substituents of the "substituted or unsubstituted amino", the "substituted or unsubstituted aminosulfinyl", the "substituted or unsubstituted carbamoyl", the "substituted or unsubstituted carbamoyloxy", the "substituted or unsubstituted thiocarbamoyl" and the "substituted or unsubstituted sulfamoyl" include 1 to 2 substituents selected from alkyl, acyl, hydroxy, alkoxy, alkoxycarbonyl, a carbocyclic group and a heterocyclic group.

In the specification, the "acyl" includes formyl, alkylcarbonyl of a carbon number of 1 to 10, alkenylcarbonyl of a carbon number of 2 to 10, alkynylcarbonyl of a carbon number of 2 to 20, carbocyclylcarbonyl and heterocyclylcarbonyl. Examples include formyl, acetyl, propionyl, butyryl, isobutyryl, valeryl, pivaloyl, hexanoyl, acryloyl, propioloyl, methacryloyl, crotonoyl, benzoyl, cyclohexanecarbonyl, pyridinecarbonyl, furancarbonyl, thiophenecarbonyl, benzothiazolecarbonyl, pyrazinecarbonyl, piperidinecarbonyl, and thiomorpholino.

In the specification, an acyl part of the "acyloxy" and the "acylamino "is the same as the above "acyl".

In the specification, examples of the substituent of the "substituted or unsubstituted acyl" and "substituted or unsubstituted acyloxy" include one or more substituents selected from the substituent group α. In addition, a ring part of the carbocyclylcarbonyl and the heterocyclylcarbonyl may be substituted with one or more substituents selected from alkyl, a substituent group α, and alkyl substituted with one or more groups selected from the substituent group α.

In the specification, the "carbocyclic group" includes cycloalkyl, cycloalkenyl, aryl and a non-aromatic fused carbocyclic group.

In the specification, the "cycloalkyl" is a carbocyclic group of a carbon number of 3 to 10, for example, a carbon number of 3 to 8, and for example, a carbon number of 4 to 8. Examples include cyclopropyl, cyclobutyl, cyclopentyl, cyclohexyl, cycloheptyl, cyclooctyl, cyclononyl and cyclodecyl.

In the specification, a cycloalkyl part of the "cycloalkylalkyl", the "cycloalkyloxy", the "cycloalkylalkoxy", the "cycloalkylthio", the "cycloalkylamino", the "cycloalkylalkylamino", the "cycloalkylsulfamoyl", the "cycloalkylsulfonyl", the "cycloalkylcarbamoyl", the "cycloalkylalkylcarbamoyl", the "cycloalkylalkoxycarbonyl", and the "cycloalkyloxycarbonyl" is the same as the above "cycloalkyl".

In the specification, the "cycloalkenyl" includes the cycloalkyl having one or more double bonds at any available position in the ring, and examples include cyclopropenyl, cyclobutenyl, cyclopentenyl, cyclohexenyl, cycloheptynyl, cyclooctynyl and cyclohexadienyl.

In the specification, the "aryl" includes phenyl, naphthyl, anthryl and phenanthryl, and specific example is phenyl.

In the specification, the "non-aromatic fused carbocyclic group" includes a non-aromatic group in which two or more cyclic groups selected from the above "cycloalkyl", the above "cycloalkenyl" and the above "aryl" are fused, and examples include indanyl, indenyl, tetrahydronaphthyl and fluorenyl.

In the specification, a carbocyclyl part of the "carbocycle", the "carbocyclyloxy", the "carbocyclylalkyl", the "carbocyclylalkoxy", the "carbocyclylalkoxycarbonyl", the "carbocyclylthio", the "carbocyclylamino", the "carbocyclylalkylamino", the "carbocyclylcarbonyl", the "carbocyclylsulfamoyl", the "carbocyclylsulfonyl", the "carbocyclylcarbamoyl", the "carbocyclylalkylcarbamoyl", the "carbocyclyloxycarbonyl", the "carbocyclylsulfinyl" and the "carbocyclylsulfonyl" is the same as the "carbocyclic group".

In the specification, an aryl part of the "arylalkyl", the "aryloxy", the "aryloxycarbonyl", the "arylalkoxycarbonyl", the "arylthio", the "arylamino", the "arylalkoxy", the "arylalkylamino", the "arylsulfonyl", the "arylsulfamoyl", the "arylcarbamoyl" and the "arylalkylcarbamoyl" is the same as the "aryl".

In the specification, the "heterocyclic group" includes a heterocyclic group having one or more hetero atoms optionally selected from O, S and N in a ring, and examples include 5- to 6-membered heteroaryl such as pyrrolyl, imidazolyl, pyrazolyl, pyridyl, pyridazinyl, pyrimidinyl, pyrazinyl, triazolyl, triazinyl, tetrazolyl, furyl, thienyl, isoxazolyl, oxazolyl, oxadiazolyl, isothiazolyl, thiazolyl and thiadiazolyl;
non-aromatic heterocyclic groups such as dioxanyl, thiiranyl, oxyranyl, oxetanyl, oxathioranyl, azetidinyl, thianyl, thiazolidinyl, pyrrolidinyl, pyrrolinyl, imidazolidinyl, imidazolinyl, pyrazolidinyl, pyrazolinyl, piperidyl, piperazinyl, morpholinyl, morpholino, thiomorpholinyl, thiomorpholino, dihydropyridyl, tetrahydropyridyl, tetrahydrofuryl, tetrahydropyranyl, dihydrothiazolyl, tetrahydrothiazolyl, tetrahydroisothiazolyl, dihydrooxazinyl, hexahydroazepinyl, tetrahydrodiazepinyl and tetrahydropyridazinyl;
fused bicyclic heterocyclic groups such as indolyl, isoindolyl, indazolyl, indolizinyl, indolinyl, isoindolinyl, quinolyl, isoquinolyl, cinnolinyl, phthalazinyl, quinazolinyl, naphthyridinyl, quinoxalinyl, purinyl, pteridinyl, benzopyranyl, benzimidazolyl, benzotriazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, thienopyridyl, thienopyrrolyl, thienopyrazolyl, thienopyrazinyl, furopyrrolyl, thienothienyl, imidazopyridyl, imidazopyrazolyl, pyrazolopyridyl, pyrazolopyrazinyl, thiazolopyridyl, pyrazolopyrimidinyl, pyrazolotrianidyl, pyridazolopyridyl, triazolopyridyl, imidazothiazolyl, pyrazinopyridazinyl, dihydrothiazolopyrimidinyl, tetrahydroquinolyl, tetrahydroisoquinolyl, dihydrobenzofuryl, dihydrobenzoxazinyl, dihydrobenzimidazolyl, tetrahydrobenzothienyl, tetrahydrobenzofuryl, benzodioxolyl, benzodioxonyl, chromanyl, chromenyl, octahydrochromenyl, dihydrobenzodioxinyl, dihydrobenzoxezinyl, dihydrobenzodioxepinyl and dihydrothienodioxinyl;
fused tricyclic heterocyclic groups such as carbazolyl, acridinyl, xanthenyl, phenothiazinyl, phenoxathiinyl, phenoxazinyl, dibenzofuryl, imidazoquinolyl and tetrahydrocarbazolyl. Examples include a 5- to 6-membered heteroaryl or a non-aromatic heterocyclic group.

In the specification, a heterocyclyl part of the "heterocycle", the "heterocyclylalkyl", the "heterocyclyloxy", the "heterocyclylthio", the "heterocyclycarbonyl", the "heterocyclylalkoxy", the "heterocyclylamino", the "heterocyclylsulfamoyl", the "heterocyclylsulfonyl", the "heterocyclylcarbamoyl", the "heterocyclyloxycarbonyl", the "heterocyclylalkylamino", the "heterocyclylalkoxycarbonyl", the "heterocyclylalkylcarbamoyl" and the "heterocyclylsulfinyl" is the same as the "heterocyclic group".

A bond of the above "heterocyclic group" may be situated on any ring.

In the specification, the "heteroaryl" includes an aromatic cyclic group among the "heterocyclic group".

In the specification, preferred examples of the ring A includes the groups as follows:

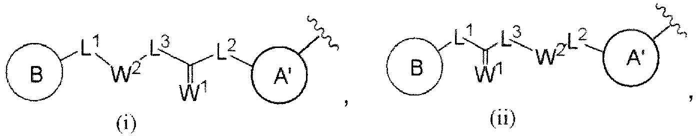

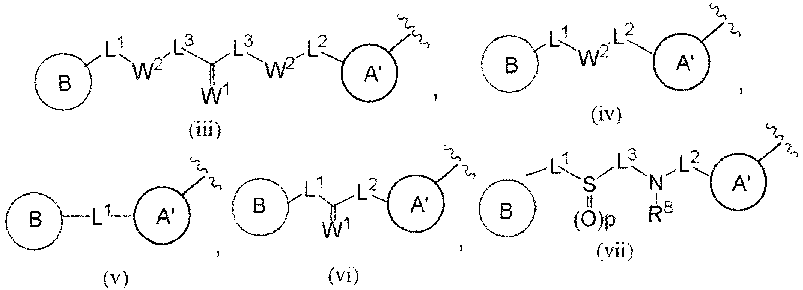

or 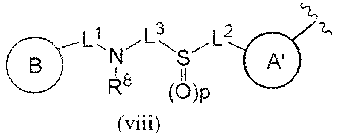
wherein the ring A' and ring B are each independently substituted or unsubstituted carbocycle or a substituted or unsubstituted heterocycle,
L¹, L² and L³ are each independently a bond, substituted or unsubstituted alkylene, substituted or unsubstituted alkenylene or substituted or unsubstituted alkynylene,
=W¹ is =O, =S or =NR⁹,
W² is O, S or N(R8),
R⁸ is hydrogen, substituted or unsubstituted alkyl, substituted or unsubstituted alkenyl, substituted or unsubstituted alkynyl or unsubstituted acyl,
R⁹ is hydrogen, hydroxy, substituted or unsubstituted alkyl, substituted or unsubstituted alkenyl, substituted or unsubstituted alkynyl or substituted or unsubstituted acyl,
when the ring A is (i), then a carbon atom constituting L¹ and a carbon atom constituting L² or an N atom of W² and a carbon atom constituting L² may bind each other via substituted or unsubstituted alkylene to form a ring,
when the ring A is (ii), then a carbon atom constituting L¹ and a carbon atom constituting L² or a carbon atom constituting L¹ and an N atom of W² may bind each other via substituted or unsubstituted alkylene to form a ring,
when the ring A is (iii), then two N atoms of W² may bind each other via substituted or unsubstituted alkylene to form a ring,
when the ring A is (vi), then a carbon atom constituting L¹ and a carbon atom constituting L² may bind each other via substituted or unsubstituted alkylene to form a ring,
p is 1 or 2, and
when there are two or more L³s, W²s, or R⁹s in the above formula, they may be each independently different from each other.

Further preferred examples of the ring A includes the groups as follows:

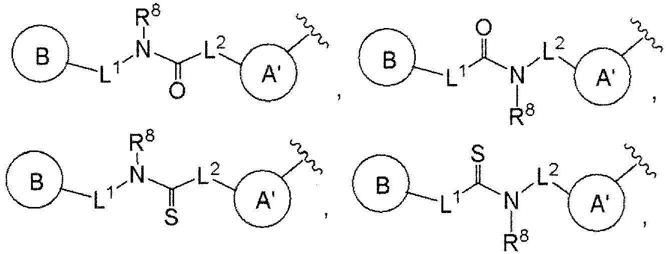

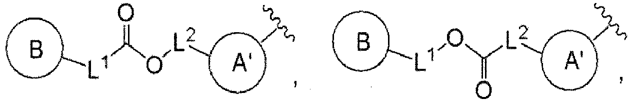

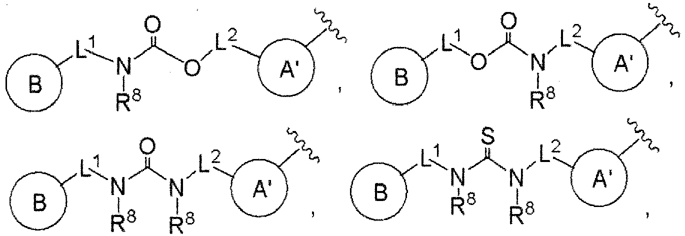

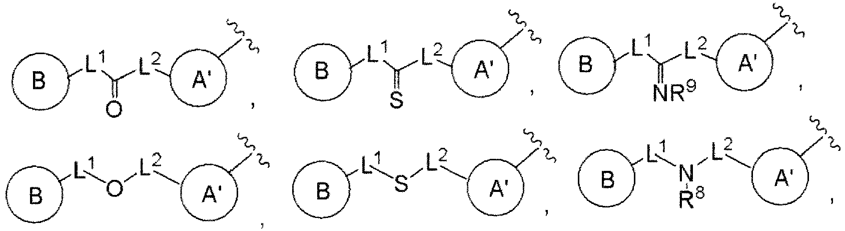

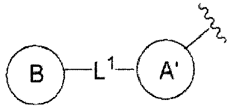

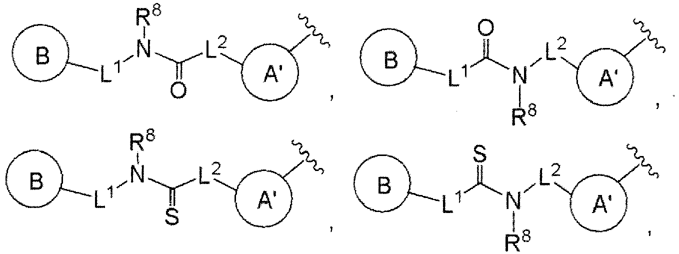

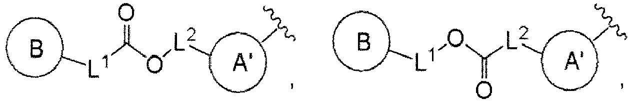

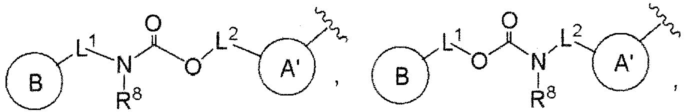

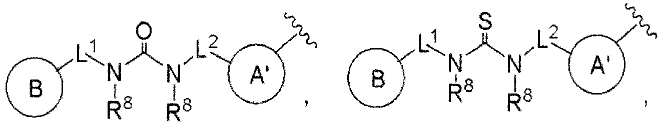

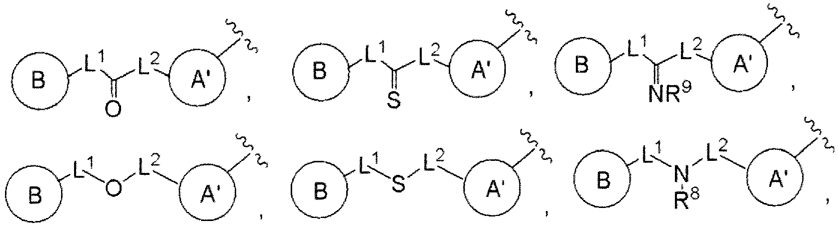

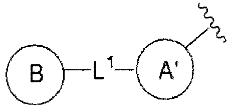

wherein L is each independently a bond, substituted or unsubstituted alkylene, substituted or unsubstituted alkenylene or substituted or unsubstituted alkynylene,
ring T is a carbocycle which may be substituted with the substituent(s) selected from the substituent group α or a heterocycle which may be substituted with the substituent(s) selected from the substituent group α, and other symbols are as defined above.

More specific examples of the ring A includes the groups as follows:

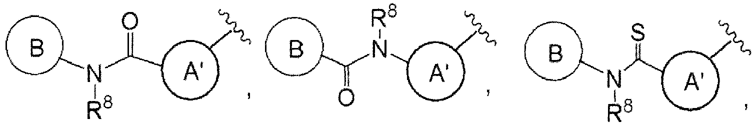

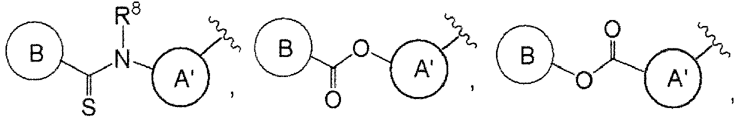

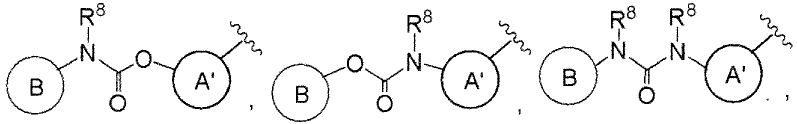

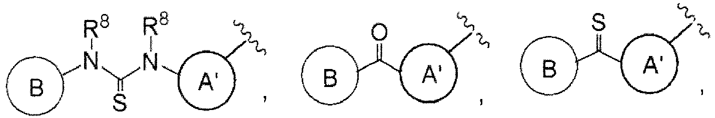

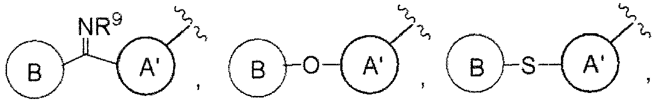

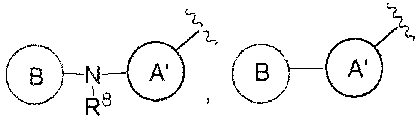

wherein symbols are as defined above.

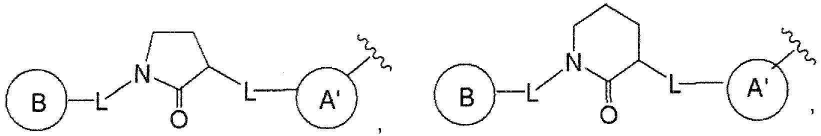

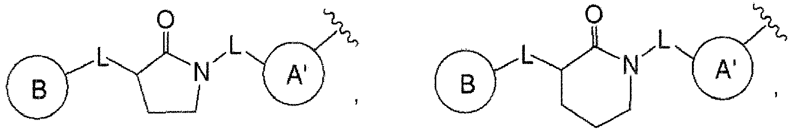

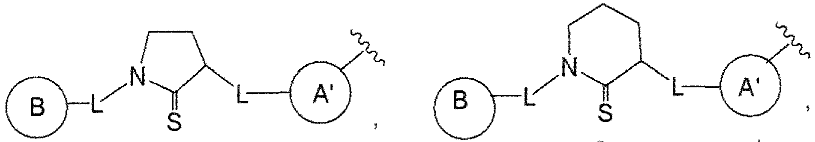

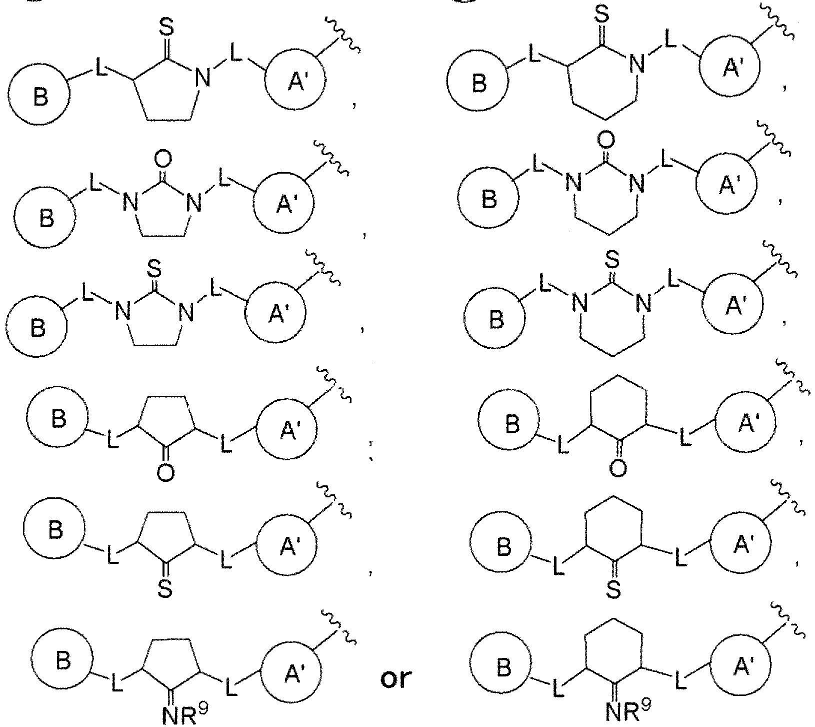

wherein symbols are as defined above.

As another embodiment of the present invention, examples of the substituent of the "substituted or unsubstituted carbocycle", the "substituted or unsubstituted heterocycle", the "substituted or unsubstituted benzene", the "substituted or unsubstituted benzene ring", the "substituted or unsubstituted pyridine", the "substituted or unsubstituted pyrimidine" and the "substituted or unsubstituted pyrazine" of the ring A, the ring A' and the ring B include:
a substituent selected from the substituent group α, for example, halogen, hydroxy, alkoxy, acyl, acyloxy, carboxy, alkoxycarbonyl, carbamoyl, amino, cyano, alkylamino and/or alkylthio etc.; alky substituted with one or more substituents selected from the substituent group α, hydroxyimino and alkoxyimino, herein, the substituent is, for example, halogen, hydroxy, alkoxy and/or alkoxycarbonyl etc. or unsubstituted alkyl;
aminoalkyl substituted with one or more substituents selected from the substituent group α; herein, the substituent is, for example, acyl, alkyl and/or alkoxy etc.;
alkenyl substituted with one or more substituents selected from the substituent group α, herein, the substituent is, for example, alkoxycarbonyl, halogen and/or halogenoalkoxycarbonyl etc. or unsubstituted alkenyl;
alkynyl substituted with one or more substituents selected from the substituent group α, herein, the substituent is, for example, alkoxycarbonyl etc. or unsubstituted alkynyl;
alkoxy substituted with one or more substituents selected from the substituent group α, herein, the substituent is, for example, halogen, carbamoyl, alkylcarbamoyl and/or hydroxyalkylcarbamoyl etc.;
alkoxyalkoxy substituted with one or more substituents selected from the substituent group α; alkenyloxy substituted with one or more substituents selected from the substituent group α, herein, the substituent is, for example, halogen, hydroxy, amino and/or alkylamino etc. or unsubstituted alkenyloxy;
alkoxyalkenyloxy substituted with one or more substituents selected from the substituent group α;
alkynyloxy substituted with one or more substituents selected from the substituent group α, herein, the substituent is, for example, halogen and/or hydroxy etc. or unsubstituted alkynyloxy, alkoxyalkynyloxy substituted with one or more groups selected from the substituent group α; alkylthio substituted with one or more substituents selected from the substituent group α or unsubstituted alkylthio;
alkenylthio substituted with one or more substituents selected from the substituent group α or unsubstituted alkenylthio;
alkynylthio substituted with one or more substituents selected from the substituent group α or unsubstituted alkynylthio;
alkylamino substituted with one or more substituents selected from the substituent group α; alkenylamino substituted with one or more substituents selected from the substituent group α; alkynylamino substituted with one or more substituents selected from the substituent group α; aminooxy substituted with one or more substituents selected from the substituent group α and alkylidene, or unsubstituted aminooxy;
acyl substituted with one or more substituents selected from the substituent group α;
alkylsulfonyl substituted with one or more substituents selected from the substituent group α or unsubstituted alkylsulfonyl;
alkylsulfinyl substituted with one or more substituents selected from the substituent group α or unsubstituted alkylsulfinyl;
alkylsulfamoyl substituted with one or more substituents selected from the substituent group α;
a carbocyclic group, e.g. cycloalkyl, aryl and the like, substituted with one or more substituents selected from the substituent group α, azide, alkyl and halogenoalkyl;
a heterocyclic group substituted with one or more substituents selected from the substituent group α, azide, alkyl and halogenoalkyl;
carbocyclylalkyl, e.g. cycloalkylalkyl, arylalkyl and the like, substituted with one or more substituents selected from the substituent group α, azide, alkyl and halogenoalkyl, or unsubstituted carbocyclylalkyl;
heterocyclylalkyl substituted with one or more substituents selected from the substituent group α, azide, alkyl and halogenoalkyl, or unsubstituted heterocyclylalkyl;
carbocyclyloxy, e.g. cycloalkoxy, aryloxy and the like, substituted with one or more substituents selected from the substituent group α, azide, alkyl and halogenoalkyl, or unsubstituted carbocyclyloxy, e.g. cycloalkylalkyl, arylalkyl and the like;
heterocyclyloxy substituted with one or more substituents selected from the substituent group α, azide, alkyl and halogenoalkyl, or unsubstituted heterocyclyloxy;
carbocyclylalkoxy, e.g. cycloalkylalkoxy, arylalkoxy and the like, substituted with one or more substituents selected from the substituent group α, azide, alkyl and halogenoalkyl, or
unsubstituted carbocyclylalkoxy, e.g. cycloalkylalkoxy, arylalkoxy and the like;
heterocyclylalkoxy substituted with one or more substituents selected from the substituent group α, azide, alkyl and halogenoalkyl, or unsubstituted heterocyclylalkoxy;
carbocyclylalkoxycarbonyl, e.g. cycloalkylalkoxycarbonyl, arylalkoxycarbonyl and the like, substituted with one or more substituents selected from the substituent group α, azide, alkyl and halogenoalkyl, or unsubstituted carbocyclylalkoxycarbonyl, e.g. cycloalkylalkoxycarbonyl, arylalkoxycarbonyl and the like;
heterocyclylalkoxycarbonyl substituted with one or more substituents selected from the substituent group α, azide, alkyl and halogenoalkyl, or unsubstituted heterocyclylalkoxycarbonyl;
carbocyclylthio, e.g. cycloalkylthio, arylthio and the like, substituted with one or more substituents selected from the substituent group α, azide, alkyl and halogenoalkyl, or unsubstituted carbocyclylthio, e.g. cycloalkylthio, arylthio and the like;
heterocyclylthio substituted with one or more substituents selected from the substituent group α, azide, alkyl and halogenoalkyl, or unsubstituted heterocyclylthio;
carbocyclylamino, e.g. cycloalkylamino, arylamino and the like, substituted with one or more substituents selected from the substituent group α, azide, alkyl and halogenoalkyl, or unsubstituted carbocyclylamino, e.g. cycloalkylamino, arylamino and the like; heterocyclylamino substituted with one or more substituents selected from the substituent group α, azide, alkyl and halogenoalkyl, or unsubstituted heterocyclylamino;
carbocyclylalkylamino, e.g. cycloalkylalkylamino, arylalkylamino and the like, substituted with one or more substituents selected from the substituent group α, azide, alkyl and halogenoalkyl, or unsubstituted carbocyclylalkylamino, e.g. cycloalkylalkylamino, arylalkylamino and the like; heterocyclylalkylamino substituted with one or more substituents selected from the substituent group α, azide, alkyl and halogenoalkyl, or unsubstituted heterocyclylalkylamino; carbocyclylsulfamoyl, e.g. cycloalkylsulfamoyl, arylsulfamoyl and the like, substituted with one or more substituents selected from the substituent group α, azide, alkyl and halogenoalkyl, or unsubstituted carbocyclylsulfamoyl;
heterocyclylsulfamoyl substituted with one or more substituents selected from the substituent group α, azide, alkyl and halogenoalkyl, or unsubstituted heterocyclylsulfamoyl; carbocyclylsulfonyl, e.g. cycloalkylsulfonyl, arylsulfonyl and the like, substituted with one or more substituents selected from the substituent group α, azide, alkyl and halogenoalkyl, or unsubstituted carbocyclylsulfonyl, e.g. cycloalkylsulfonyl, arylsulfonyl and the like; heterocyclylsulfonyl substituted with one or more substituents selected from the substituent group α**,** azide, alkyl and halogenoalkyl, or unsubstituted heterocyclylsulfonyl; carbocyclylcarbamoyl, e.g. cycloalkylcarbamoyl, arylcarbamoyl and the like, substituted with one or more substituents selected from the substituent group α, azide, alkyl and halogenoalkyl, or unsubstituted carbocyclylcarbamoyl, e.g. cycloalkylcarbamoyl, arylcarbamoyl and the like; heterocyclylcarbamoyl substituted with one or more substituents selected from the substituent group α, azide, alkyl and halogenoalkyl, or unsubstituted heterocyclylcarbamoyh carbocyclylalkylcarbamoyl, e.g. cycloalkylalkylcarbamoyl, arylalkylcarbamoyl and the like, substituted with one or more substituents selected from the substituent group α, azide, alkyl and halogenoalkyl, or unsubstituted carbocyclylalkylcarbamoyl, e.g. cycloalkylalkylcarbamoyl, arylalkylcarbamoyl and the like;
heterocyclylalkylcarbamoyl substituted with one or more substituents selected from the substituent group α, azide, alkyl and halogenoalkyl, or unsubstituted heterocyclylalkylcarbamoyl;
carbocyclyloxycarbonyl, e.g. cycloalkyloxycarbonyl, aryloxycarbonyl and the like, substituted with one or more substituents selected from the substituent group α, azide, alkyl and halogenoalkyl, or unsubstituted carbocyclyloxycarbonyl, e.g. cycloalkoxycarbonyl, aryloxycarbonyl and the like;
heterocyclyloxycarbonyl substituted with one or more substituents selected from the substituent group α, azide, alkyl and halogenoalkyl, or unsubstituted heterocyclyloxycarbonyl; alkylenedioxy substituted with halogen, or unsubstituted alkylenedioxy; oxo; and
azide. The aforementioned ring of ring A, ring A' and ring B each may be substituted with one or more substituents selected from them.

In the specification, examples of the substituent of the "substituted or unsubstituted carbocycle", the "substituted or unsubstituted benzene", the "substituted or unsubstituted heterocycle", the "substituted or unsubstituted pyridine", the "substituted or unsubstituted pyrimidine" and the "substituted or unsubstituted pyrazine" in ring A' and ring B include halogen, cyano, hydroxy, nitro, carboxy, alkyl substituted with one or more substituents selected from the substituent group α, unsubstituted alkyl, alkoxy substituted with one or more substituents selected from the substituent group α, unsubstituted alkoxy, amino substituted with one or more substituents selected from the substituent group α, unsubstituted amino, carbamoyl substituted with one or more substituents selected from the substituent group α, unsubstituted carbamoyl, alkoxycarbonyl substituted with one or more substituents selected from the substituent group α, and unsubstituted alkoxycarbonyl.

In the specification, ,examples of the substituent except "-Z-ring B" of the "substituted or unsubstituted carbocycle" or the "substituted or unsubstituted heterocycle" in ring A include halogen, hydroxy, alkyl, alkenyl, alkynyl, alkoxy, alkenyloxy, alkynyloxy, acyl, carboxy, alkoxycarbonyl, amino or cyano.

In the specification, examples of the substituent of the "substituted or unsubstituted carbocycle", the "substituted or unsubstituted benzene" or the "substituted or unsubstituted heterocycle" in ring A' include halogen.

In the specification, examples of the substituent of the "substituted or unsubstituted carbocycle", the "substituted or unsubstituted heterocycle", "substituted or unsubstituted pyridine", the "substituted or unsubstituted pyrimidine" and the "substituted or unsubstituted pyrazine" in ring B include halogen, hydroxy, alkyl, alkenyl, alkynyl, alkoxy, alkenyloxy, alkynyloxy, acyl, carboxy, alkoxycarbonyl, amino or cyano.

In the specification, examples of the substituent of the "substituted or unsubstituted carbocyclic group", the "substituted or unsubstituted carbocyclylthio", the "substituted or unsubstituted carbocyclylalkyl", the "substituted or unsubstituted carbocyclylalkoxy", the "substituted or unsubstituted carbocyclyloxycarbonyl", the "substituted or unsubstituted carbocyclyloxy" the "substituted or unsubstituted carbocyclylsulfinyl", the "substituted or unsubstituted carbocyclylsulfonyl", the "substituted or unsubstituted heterocyclic group", the "substituted or unsubstituted heterocyclyloxy", the "substituted or unsubstituted heterocyclylthio", the "substituted or unsubstituted heterocyclylalkyl", the "substituted or unsubstituted heterocyclylalkoxy", "substituted or unsubstituted heterocyclyloxycarbonyl", the "substituted or unsubstituted heterocyclylsulfinyl", the "substituted or unsubstituted heterocyclylsulfonyl" and the "substituted or unsubstituted heterocycle" in other than the above ring A, ring A' and ring B include one or more substituents selected from (i) alkyl substituted with one or more substituents selected from the substituent group α, (ii) unsubstituted alkyl and (iii) the substituent group α.

In the specification, the "alkylene" includes a straight or branched divalent carbon chain of a carbon number of 1 to 10, for example, a carbon number of 1 to 6, or a carbon number of 1 to 3. Examples include methylene, dimethylene, trimethylene, tetramethylene, and methyltrimethylene.

In the specification, an alkylene part of the "alkylenedioxy" is the same as the "alkylene".

In the specification, the "alkenylene" includes a straight or branched divalent carbon chain of a carbon number of 2 to 10, for example, a carbon number of 2 to 6, or a carbon number of 2 to 4, having a double bond at any available position. Examples include vinylene, propenylene, butenylene, butadienylene, methylpropenylene, pentenylene and hexenylene.

In the specification, the "alkynylene" includes a straight or branched divalent carbon chain of a carbon number of 2 to 10, for example, a carbon number of 2 to 6, or a carbon number of 2 to 4, having a triple bond at any available position and, further, optionally having a double bond. Examples include ethynylene, propynylene, butynylene, pentynylene and hexynylene.

Examples of the substituent of the "substituted or unsubstituted alkylene", the "substituted or unsubstituted alkenylene" and the "substituted or unsubstituted alkynylene" include a group(s) selected from the substituent group α. Examples include halogen and hydroxy.

When "R^{3a} and R^{4a} together with the carbon atom to which they are attached may form a substituted or unsubstituted carbocycle or a substituted or unsubstituted heterocycle" in the formula (I), 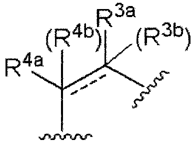 wherein each symbol is as defined above the item (I),
includes

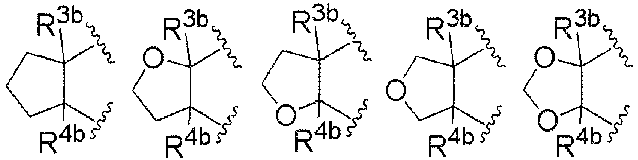

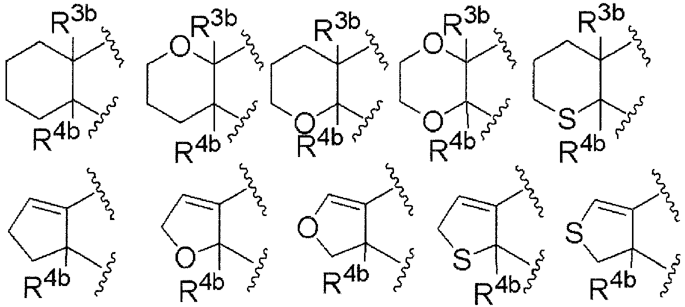

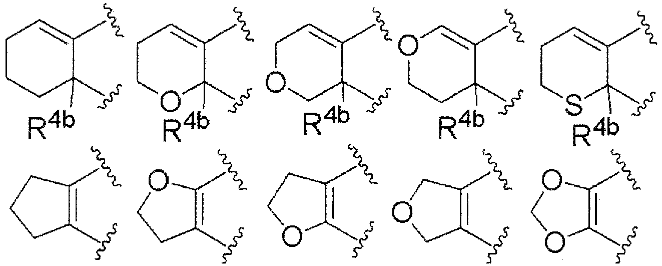

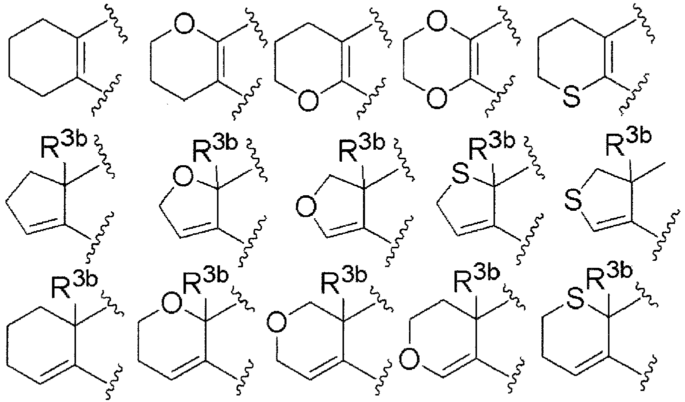

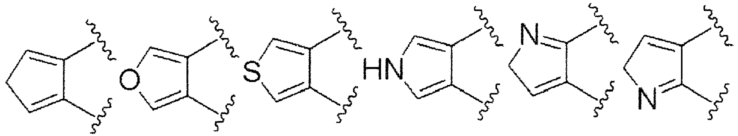

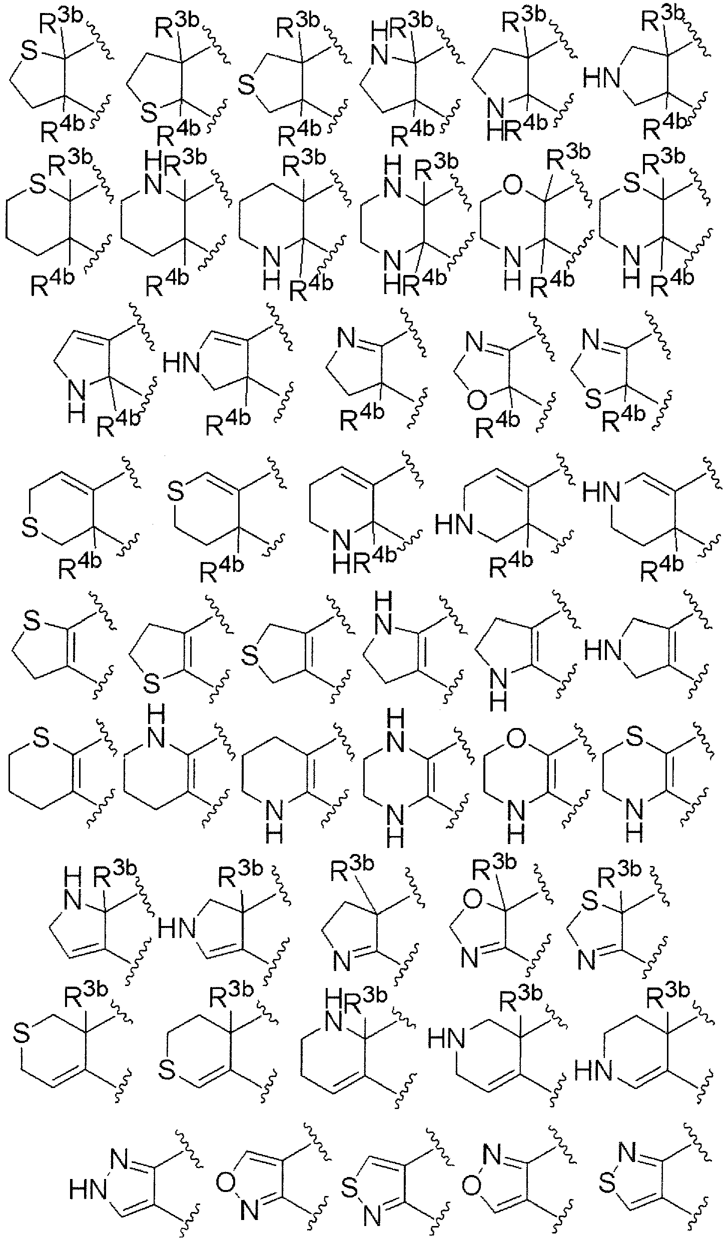

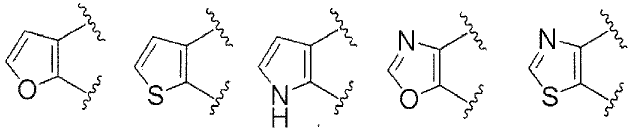

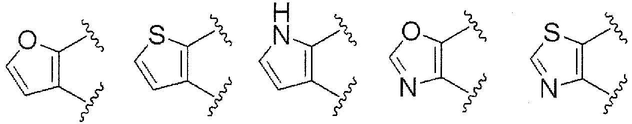

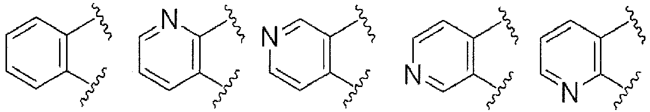

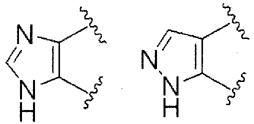

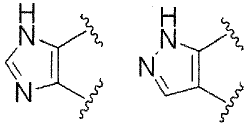

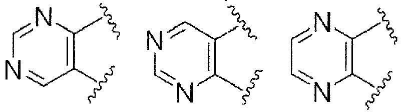

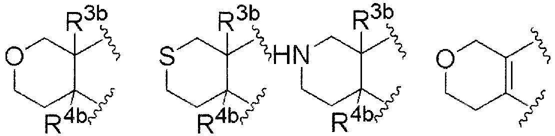

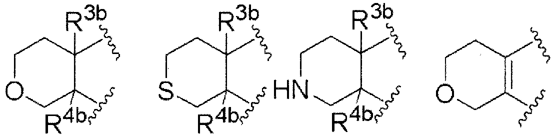

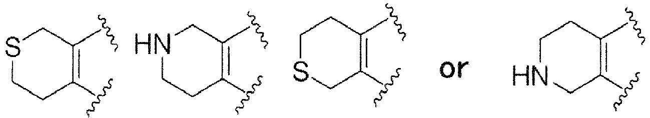

These ring may be substituted at any available position with one or more substituents selected from the substituent group α, unsubstituted alkyl and alkyl substituted with one or more substituents selected from the substituent group α.

When "R^{3a} and R^{3b} together with the carbon atom to which they are attached may form a substituted or unsubstituted carbocycle or a substituted or unsubstituted heterocycle" or "R^{4a} and R^{4b} together with the carbon atom to which they are attached may form a substituted or unsubstituted carbocycle or a substituted or unsubstituted heterocycle", 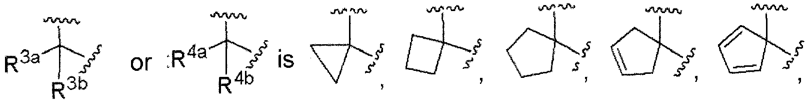 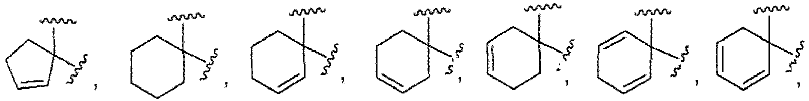 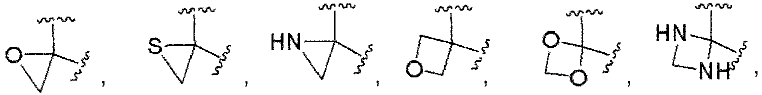 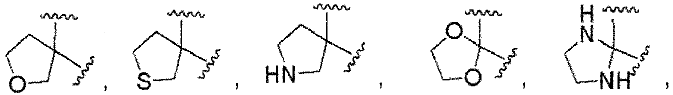 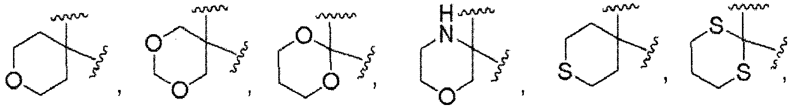 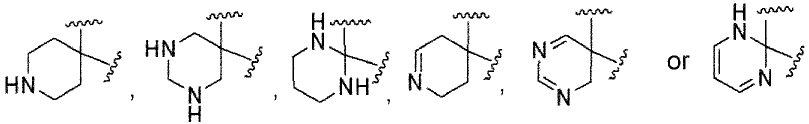 These ring may be substituted at any available position with one or more substituents selected from the substituent group α, unsubstituted alkyl and alkyl substituted with one or more substituents selected from the substituent group α.

In the specification, the term "solvate" includes, for example, solvates with organic solvents and hydrates. It can be prepared in accordance with the known methods. Examples of solvate include a solvate with acetone, 2-butanol, 2-propanol, ethanol, ethyl acetate, tetrahydrofuran or diethylether. For example, it includes a non-toxic and water-soluble hydrate or solvate such as a solvate with ethanol. In the case that a hydrate or solvate is formed, the compound or salt may be coordinated with any number of solvate molecules or water molecules.

The compound of formulae(I) and (Ia) to (Ij) includes a pharmaceutically acceptable salt. Examples include salts with alkali metals such as lithium, sodium or potassium; alkaline earth metals such as calcium; magnesium; transition metals such as zinc or iron; ammonium; organic bases; and amino acids; or salts with inorganic acids such as hydrochloric acid, sulfuric acid, nitric acid, hydrobromic acid, phosphoric acid or hydroiodic acid; and organic acids such as acetic acid, trifluoroacetic acid, citric acid, lactic acid, tartaric acid, oxalic acid, maleic acid, fumaric acid, mandelic acid, glutaric acid, malic acid, benzoic acid, phthalic acid, benzenesulfonic acid, p-toluenesulfonic acid, methanesulfonic acid or ethane sulfonic acid. Particularly, hydrochloric acid, phosphoric acid, tartaric acid or methanesulfonic acid is preferable. These salts can be formed by ordinary methods.

In addition, the compound of formula (I) is not limited to a specific isomer, but includes all possible isomers, such as keto-enol isomers, imine-enamine isomers, diastereoisomers, optical isomers and rotation isomers; and racemate. For example, the compound of formula (I) in which R^{2a} is hydrogen includes the following tautomers.

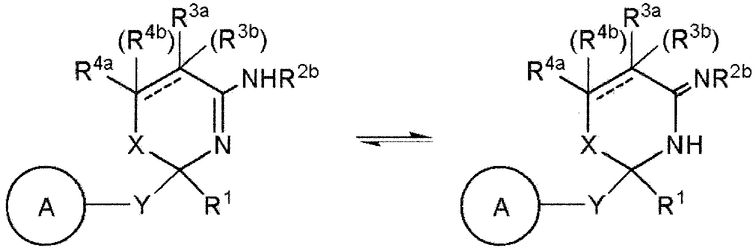

Compounds of formulas(Ia) to (Ij) include the similar tautomers.

The compound of formula (I) has an asymmetric carbon atom and includes any optical isomers described below.

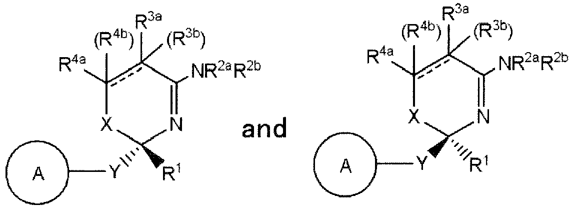

Preferable is

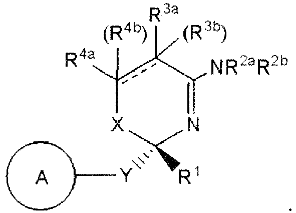

In addition, one or more hydrogen, carbon or other atoms of the compound of formula (I) and (Ia) to (Ij) can be replaced by an isotope of the hydrogen, carbon or other atoms. Compounds of formula (I) include all radiolabeled forms of compounds of formula (I). The "radiolabeled," "radiolabeled form" and the like of the compound of formula (I) are encompassed by the present invention and useful as a research and/or diagnostic tool in metabolism pharmacokinetic studies and in binding assays. It is also useful for a medicament.

Examples of isotopes that can be incorporated into the compound of formula (I) and (Ia) to (Ij) of the invention include isotopes of hydrogen, carbon, nitrogen, oxygen, phosphorous, sulfur, fluorine, iodine and chlorine, such as ²H, ³H, ¹¹C, ¹³C, ¹⁴C, ¹⁵N, ¹⁸O, ¹⁷O, ³¹P, ³²P, ³⁵S, ¹⁸F, ¹²³I and ³⁶Cl, respectively. Radiolabeled compounds of the invention can be prepared by methods known in the art. For example, tritiated compounds of formula (I) and (Ia) to (Ij) can be prepared by introducing tritium into the particular compound of formula(I) and (Ia) to (Ij), for example, by catalytic dehalogenation with tritium. This method may include reacting a suitably halogen-substituted precursor of a compound of formula (I) and (Ia) to (Ij) with tritium gas in the presence of a suitable catalyst such as Pd/C, in the presence or absence of a base. Other suitable methods for preparing tritiated compounds can be found in the document, "The Preparation and Characterization of Tritiated Neurochemicals," Chapter 6, pp. 155-192 in Isotopes in the Physical and Biamedical Sciences, Vol. 1, Labeled Compounds (Part A) (1987). ¹⁴C-labeled compounds can be prepared by employing starting materials having a ¹⁴C carbon.

### (General Preparation)

The compounds of the present invention of formulae (I) and (Ia) to (Ij) can be prepared the method shown below. In the following all steps, when a substituent which interferes with the reaction, e.g. hydroxy, mercapto, amino, formyl, carbonyl, carboxy, is possessed, the substituent is protected by the method such as those described in Protective Groups in organic Synthesis, and Theodora W Greene (John Wiley & Sons) in advance, and the protective group may be removed at a desirable step.

### A. Preparation of a compound of formula (Ia)

The compound of formula (Ia) can be prepared, for example, according to a method of synthesis shown below.

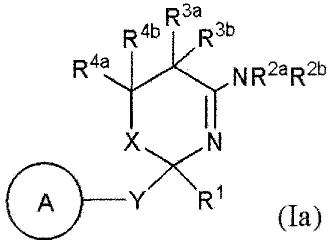

### Synthesis of compound (Ia)

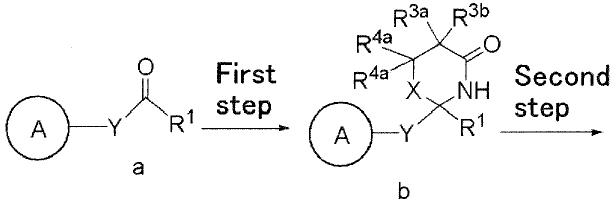

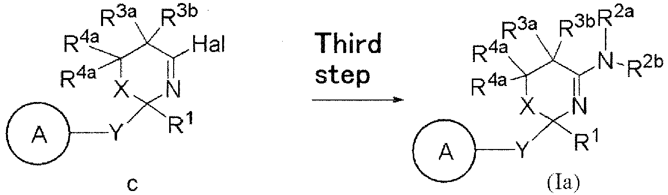

wherein Hal is halogen and the other symbols are as defined above.

### First step

Compound b can be prepared by adding 3-mercapto propane amide derivative, 3-hydroxy propane amide derivative or 3-amino propane amide derivative, which is commercially available or prepared by the known methods, to Compound a, preferably in the presence of an acid such as p-toluene sulfonic acid, hydrochloric acid, hydrobromic acid, sulfuric acid and trifluoroacetic acid, or an amine such as pyrrolidine, piperidine, piperazine and morpholine, and reacting at 0°C to 150°C, preferably 0°C to solvent reflux temperature, for 0.5 to 48 hours, preferably 1 to 24 hours in a solvent such as toluene, dichloromethane, tetrahydrofuran, or a mixed solvent thereof.

### Second step

Compound c can be prepared by adding a halogenating agent such as phosphorus oxychloride, thionyl chloride and oxalyl chloride to Compound b, and reacting at 0°C to 100°C, preferably 10°C to 50°C for 0.5 to 72 hours, preferably 0.5 to 6 hours in a solvent such as dioxane, dichloromethane, tetrahydrofuran, toluene and acetone, or a mixed solvent thereof.

### Third step

Compound (Ia) can be prepared by adding a primary amine derivative or secondary amine derivative, which is commercially available or prepared by the known methods, to Compound c, and reacting at 0°C to 150°C, preferably 0°C to solvent reflux temperature, for 0.5 to 48 hours, preferably 1 to 24 hours in a solvent such as dioxane, dichloromethane, tetrahydrofuran, toluene and acetone, or a mixed solvent thereof.

Compound wherein -X- is -S- which is obtained by the above method can be converted to a compound wherein -X- is -SO- or -SO₂- by oxidizing using the known oxidizing agent such as m-chloro perbenzoic acid, hydrogen peroxide and potassium permanganate.

### B. Preparation of a compound of formula (Ib)

The compound of formula (Ib) can be prepared, for example, according to a method of synthesis shown below.

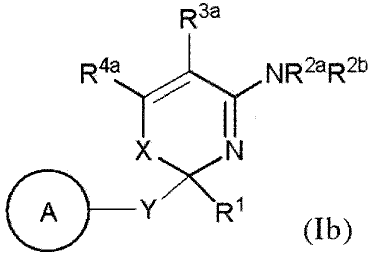

### Synthesis of compound (Ib)

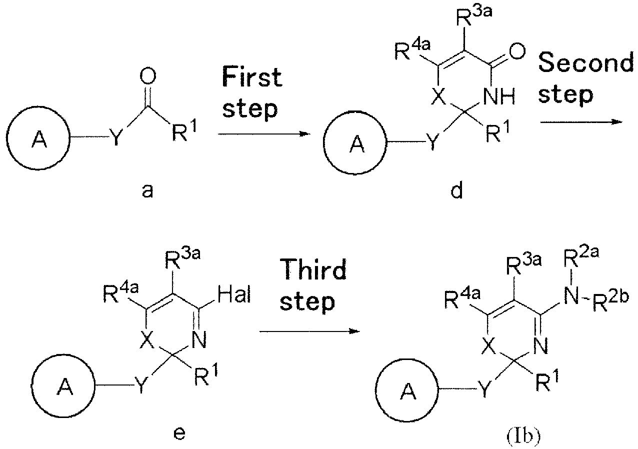

wherein each symbol is as defined above.

### First step

Compound d can be prepared by adding 2-mercaptobenzamide derivative, 2-hydroxybenzamide derivative, 2-aminobenzamide derivative or 2-mercaptopyridine amide derivative, which is commercially available or prepared by the known methods, to Compound a, preferably in the presence of an acid such as p-toluene sulfonic acid, hydrochloric acid, hydrobromic acid, sulfuric acid and trifluoroacetic acid, or an amine such as pyrrolidine, piperidine, piperazine and morpholine, and reacting at 0°C to 150°C, preferably room temperature to solvent reflux temperature, for 0 to 48 hours, preferably 1 to 24 hours in a solvent such as toluene, dichloromethane, and tetrahydrofuran, or a mixed solvent thereof.

### Second step

Compound e can be prepared by adding a halogenating agent such as phosphorus oxychloride, thionyl chloride and oxalyl chloride to Compound d, and reacting at 0°C to 100°C, preferably 10°C to 50°C, for 0.5 to 72 hours, preferably 0.5 to 18 hours in a solvent such as dioxane, dichloromethane, tetrahydrofuran, toluene and acetone, or a mixed solvent thereof.

### Third step

Compound (Ib) can be prepared by adding primary amine derivative or secondary amine derivative, which is commercially available or prepared by the known methods, to Compound e, and reacting at 0°C to 150°C, preferably 0°C to solvent reflux temperature, for 0.5 to 48 hours, preferably 1 to 24 hours in a solvent such as dioxane, dichloromethane, tetrahydrofuran, toluene and acetone, or a mixed solvent thereof.

### D. Synthesis of Compound (Ic)

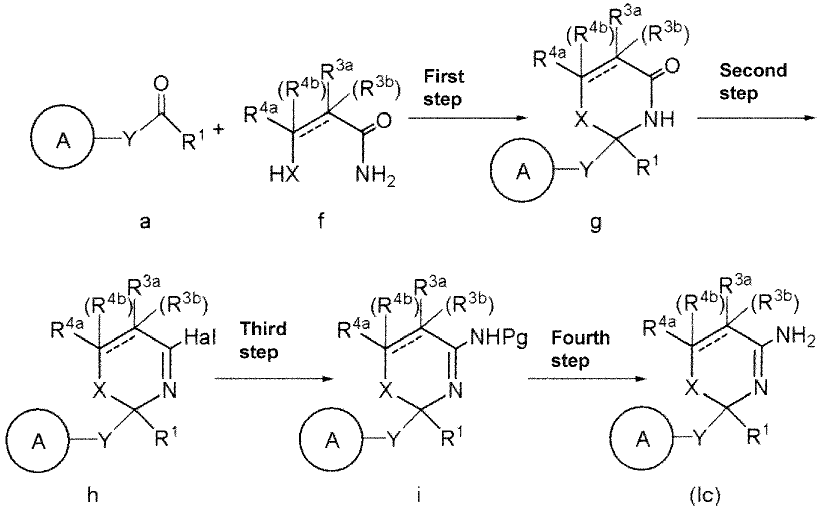

wherein Hal is halogen, Pg is an amino protective group, e.g. a tert-butoxycarbonyl group, a benzyl group, a benzyloxycarbonyl group and the other symbols are as defined above.

### First step

Compound g can be prepared by adding compound f or its derivative, which is commercially available or prepared by the known methods, to Compound a, preferably in the presence of an acid such as p-toluene sulfonic acid, hydrochloric acid, hydrobromic acid, sulfuric acid and trifluoroacetic acid, or an amine such as pyrrolidine, piperidine, piperazine and morpholine, and reacting at 0°C to 150°C, preferably 0°C to solvent reflux temperature, for 0.5 to 48 hours, preferably 1 to 24 hours in a solvent such as toluene, dichloromethane and tetrahydrofuran, or a mixed solvent thereof.

### Second step

Compound h can be prepared by adding a halogenating agent such as phosphorus oxychloride, thionyl chloride and oxalyl chloride to Compound g, and reacting at 0°C to 100°C, preferably 10°C to 50°C, for 0.5 to 72 hours, preferably 0.5 to 18 hours in a solvent such as dioxane, dichloromethane, tetrahydrofuran, toluene and acetone, or a mixed solvent thereof.

### Third step

Compound i can be prepared by adding an amine having a protective group Pg (shown as PgNH₂), which is commercially available or is prepared by the known methods, to Compound h, in the presence of a base, e.g. calcium carbonate, palladium catalyst e.g. palladium chloride (PdClz) or phosphine ligand, e.g. triphenylphosphine (PPh₃), reacting at 0°C to 150°C, preferably 0°C to solvent reflux temperature, for 0.5 to 48 hours, preferably 1 to 24 hours in a solvent such as dioxane, dichloromethane, tetrahydrofuran, toluene and acetone, or a mixed solvent thereof.

### Fourth step

Compound (Ic) can be prepared by deprotecting a Pg-group of Compound i.

Deprotections are performed by the method such as those described in Protective Groups in Organic Synthesis, Theodora W Green (John Wiley & Sons). For example, if a protective group is a benzyl group or a p-methoxybenzyl group, deprotection can be performed by catalytic reduction in the presence of hydrogen.

### E. D. Synthesis of Compound (Id)

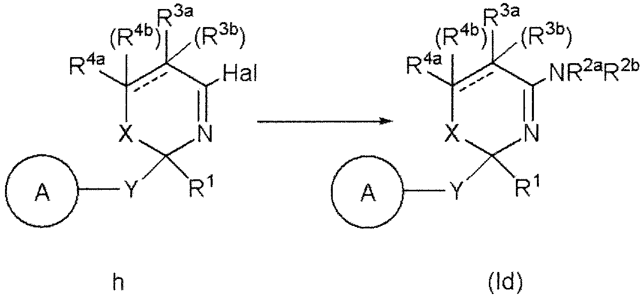

wherein each symbol is as defined above.

Compound (Id) can be prepared by adding amine (shown as R²aR^{2b}NH), which is commercially available or prepared by the known methods, to Compound h, preferably in the presence of a base, e.g. calcium carbonate, palladium catalyst such as palladium chloride (PdCl₂) or phosphine ligand, e.g. triphenylphosphine (PPh₃), and reacting at 0°C to 150°C, preferably 0°C to solvent reflux temperature, for 0.5 to 48 hours, preferably 1 to 24 hours in a solvent such as dioxane, dichloromethane, tetrahydrofuran, toluene and acetone, or a mixed solvent thereof.

### F. Synthesis of Compound (If)

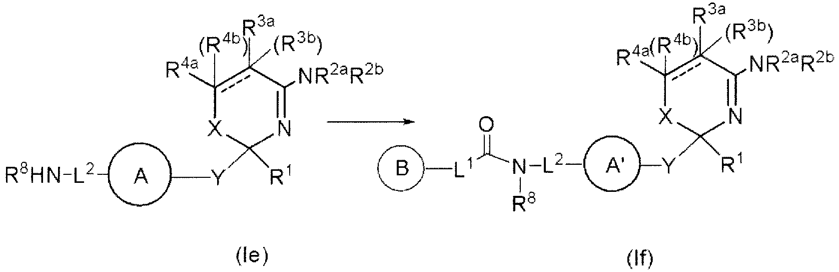

wherein each symbol is as defined above.

Compound (If) can be prepared by reacting a reacting agent such as acid chloride, acid anhydride, chlorocarbonic ester and isocyanate, each of which have substituent corresponding to that of the objective compound, e.g. benzoyl chloride, 2-furoylchloride, acetic anhydride, chlorocarbonic benzyl, 2- carbonic-di-tert-butyl or phenyl isocyanate, with Compound (Ie) having substituted or unsubstituted amino in the ring A, in the absence or presence of a base such as pyridine and triethylamine at -80°C to 100°C, preferably -20°C to 40°C, for 0.1 to 24 hours, preferably 1 to 12 hours in the absence or presence of a solvent such as tetrahydrofuran and dichloromethane.
Compound (If) can also be prepared by reacting carboxylic acid which have a substituent corresponding to that of the objective compound, e.g. benzoic acid, 2-pyridinecarboxylic acid, with the above Compound (Ie), in the presence of a dehydration condensation agent such as dicyclohexylcarbodiimide, carbonyldiimidazole and dicyclohexylcarbo-di-imide-N-hydroxy benzotriazole at -80°C to 100°C, preferably -20°C to 40°C, for 0.1 to 24 hours, preferably 1 to 12 hours in a solvent such as dimethylformamide, tetrahydrofuran and dichloromethane.

### G. Synthesis of Compound (Ih)

Compound (Ih) can be prepared by the following method A or method B

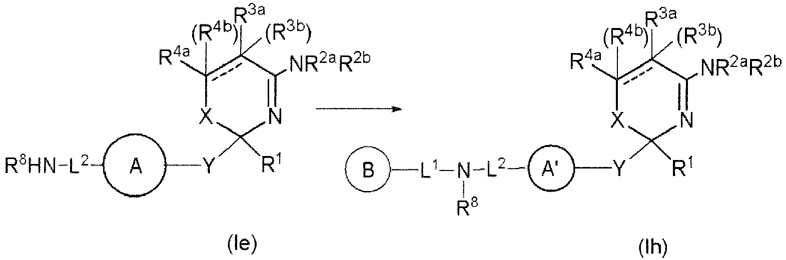

wherein each symbol is as defined above.

### Method A: Condensation under acidic condition

Compound (Ih) can be prepared by adding an acid such as hydrogen chloride, sulfuric acid, trifluoroacetic acid, methanesulfonic acid, trifluoromethanesulfonic acid, and perchloric acid to aryl halide or heteroaryl halide, which is commercially available or prepared by the known methods (Tetrahedron, 2009, Vol. 65, 757-764) and compound (Ie), and reacting at 0°C to solvent reflux temperature, preferably 20°C to 140°C, for 0.1 to 120 hours, preferably 0.5 to 72 hours in a solvent such as methanol, ethanol, isopropyl alcohol, butanol, isobutanol, sec-butanol, acetic acid and water, or a mixed solvent thereof.

### Method B: Condensation under basic condition

Compound (Ih) can be prepared by reacting aryl halide or heteroaryl halide, which is commercially available or prepared by the known methods (Tetrahedron, 2009, Vol. 65, 757-764), and compound (Ie), in the presence of a base such as triethylamine, sodium carbonate, potassium carbonate, cesium carbonate, sodium methoxide, potassium tert-butoxide, n-butyllithium, lithium hexamethyldisilazide, sodium hexamethyldisilazide and potassium hexamethyldisilazide, at 0°C to solvent reflux temperature, preferably 20°C to 140°C, for 0.5 to 100 hours, preferably 0.5 to 72 hours in a solvent such as toluene, tetrahydrofuran, dimethylformamide, 1,2-dimethoxyethane, 1,4-dioxane and methanol.

The reaction can be performed in the presence of tris(dibenzylideneacetone) dipalladium, palladium acetate or palladium (0) prepared in situ or the like and a phosphine ligand such as triphenylphosphine, tri tert-butylphosphine, dicyclohexyl biphenylphosphine, 9,9-dimethyl-4,5-(diphenylphosphino)xanthene (Xantphos), 2-dicyclohexylphosphino-2',4',6'-triisopropylbiphenyl (X-Phos), 2-dicyclohexylphosphino-2',6'-diisopropoxy-1,1'-biphenyl (Ruphos). In the above method, the compounds of the formula (Ih) can be prepared by the reaction at 0°C to 150°C, preferably 10°C to 100°C for 0.5 hour to 72 hours, preferably 1 hour to 24 hours with or without microwave irradiation.

### H. Synthesis of Compound (Ij)

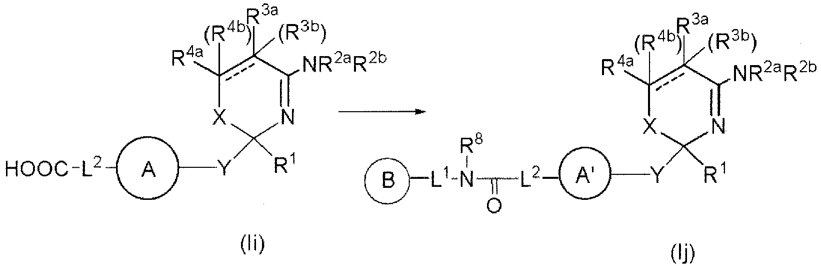

wherein each symbol is as defined above.

Compound (Ij) can be prepared by reacting Compound (Ii) which have a carboxy group in ring A, with primary amine or secondary amine which have a substituent corresponding to that of the objective compound, e.g. aniline, 2-aminopyridine and dimethylamine, in the presence of a dehydration condensation agent such as dicyclohexylcarbodiimide, carbonyldiimidazole and dicyclohexylcarbo-di-imide-N-hydroxy benzotriazole, at -80°C to 100°C, preferably -20°C to 40°C, for 0.1 to 24 hours, preferably 1 to 12 hours in a solvent such as dimethylformamide, tetrahydrofuran and dichloromethane.

In the all above steps, an order of steps to be implemented may be appropriately changed, and each intermediate may be isolated, and used in a next step.

### I. Conversion of substituent

The synthesis of a compound (Ia) to (Ij) and a compound of which the ring A is substituted with various substituent, e.g. substituent of ring B-Z-, can be prepared by the above method or the known methods, e.g. the method such as those described in Patent Literatures 2 to 4.

Moreover, the optically active isomer of the compound (Ia) to (Ij) can be prepared by performing an asymmetric synthesis in the suitable step to prepare an optically active intermediate using a reagent for asymmetric synthesis, or optical resolution of the racemate of the intermediate or the objective compound in the appropriate step. The method of optical resolution include the separation of optical isomer using an optically active column, the kinetics optical resolution using enzyme reaction or the like, the crystallization and separation of diastereomer by the salt formation using chiral acid or chiral base, the preferential crystallization or the like.

Specific embodiments of the present invention are illustrated below. Each symbol is as defined above.

In the formula (I), the followings are exemplified.

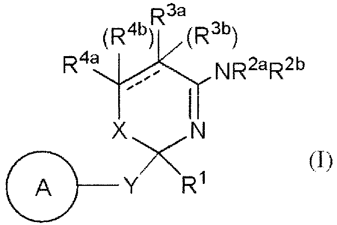

-X- includes -O-, -S-, -SO-, -SO₂- or-N(R^{x})-.

Examples of -X- include -O- or -S-.

R^{x} includes hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl.

Examples of R^{x} include hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclyloxycarbonyl, substituted or unsubstituted heterocyclylsulfinyl or substituted or unsubstituted heterocyclylsulfonyl.

Ring A includes a substituted or unsubstituted carbocycle or a substituted or unsubstituted heterocycle.

Examples of ring A include a substituted or unsubstituted carbocycle.

Examples of ring A include substituted or unsubstituted benzene.

Examples of ring A include the following formula;

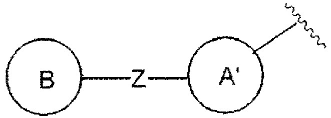

Ring A' and ring B are each independently a substituted or unsubstituted carbocycle or a substituted or unsubstituted heterocycle.

Examples of ring A' include substituted or unsubstituted benzene.

Examples of ring B include substituted or unsubstituted pyridine, substituted or unsubstituted pyrimidine or substituted or unsubstituted pyrazine.

-Z- includes -L¹-C(=O)N(R⁸)-L²-, -L¹-N(R⁸)C(=O)-L²- or -L¹-N(R⁸)-L²-.

Examples of -Z- include -L¹-C(=O)N(R⁸)-L²- wherein L¹ and L² are bond.

L¹ and L² include each independently a bond, substituted or unsubstituted alkylene, substituted or unsubstituted alkenylene or substituted or unsubstituted alkynylene, R⁸ includes hydrogen, substituted or unsubstituted alkyl, substituted or unsubstituted alkenyl, substituted or unsubstituted alkynyl or substituted or unsubstituted acyl.

-Y- includes substituted or unsubstituted alkenylene, substituted or unsubstituted alkynylene, -(CR⁵R⁶)ₙ-, -(CR⁵R⁶)ₙO(CR⁵R⁶)ₘ-, -(CR⁵R⁶)ₙS(CR⁵R⁶)ₘ-, -(CR⁵R⁶)ₙSO₂(CR⁵R⁶)ₘ-, - (CR⁵R⁶)ₙSO(CR⁵R⁶)ₘ-, -(CR⁵R⁶)ₙC(=O)(CR⁵R⁶)ₘ-, -(CR⁵R⁶)ₙN(R⁷)(CR⁵R⁶)ₘ-, - (CR⁵R⁶)ₙC(=O)N(R⁷)(CR⁵R⁶)ₘ-, or -(CR⁵R⁶)ₙN(R⁷)C(=O)(CR⁵R⁶)ₘ-.

Examples of -Y- include substituted or unsubstituted alkenylene, substituted or unsubstituted alkynylene, or -(CR⁵R⁶)ₙ-.

Examples of -Y- include -(CR⁵R⁶)ₙ-.

Examples of -Y- include -(CR⁵R⁶)ₙ- wherein n is 0.

R⁵ and R⁶ include each independently hydrogen, halogen, hydroxy, nitro, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted carbamoyloxy, substituted or unsubstituted thiocarbamoyl, substituted or unsubstituted sulfamoyl, substituted or unsubstituted aminosulfinyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl.

Examples of R⁵ and R⁶ include each independently hydrogen, halogen, hydroxy, nitro, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted carbamoyloxy, substituted or unsubstituted thiocarbamoyl, substituted or unsubstituted sulfamoyl, substituted or unsubstituted aminosulfinyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclyloxycarbonyl, substituted or unsubstituted carbocyclylthio, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted carbocyclyloxycarbonyl, substituted or unsubstituted heterocyclylthio, substituted or unsubstituted heterocyclylsulfinyl or substituted or unsubstituted heterocyclylsulfonyl.

Examples of R⁵ and R⁶ include each independently hydrogen, halogen, hydroxy, nitro, substituted or unsubstituted alkyl, substituted or unsubstituted alkenyl, substituted or unsubstituted alkynyl, substituted or unsubstituted alkoxy, substituted or unsubstituted alkylthio, substituted or unsubstituted acyl, substituted or unsubstituted acyloxy, cyano, carboxy, substituted or unsubstituted alkoxycarbonyl, substituted or unsubstituted amino, substituted or unsubstituted carbamoyl, substituted or unsubstituted thiocarbamoyl, substituted or unsubstituted sulfamoyl, a substituted or unsubstituted carbocyclic group, substituted or unsubstituted carbocyclyloxycarbonyl, a substituted or unsubstituted heterocyclic group, or substituted or unsubstituted carbocyclyloxycarbonyl.

Examples of R⁵ and R⁶ include each independently hydrogen, halogen, hydroxy, nitro, unsubstituted alkyl, unsubstituted alkenyl, unsubstituted alkoxy, unsubstituted alkylthio, unsubstituted acyl, cyano, carboxy, unsubstituted alkoxycarbonyl, unsubstituted amino, unsubstituted carbamoyl, an unsubstituted carbocyclic group, unsubstituted carbocyclyloxycarbonyl, an unsubstituted heterocyclic group or unsubstituted carbocyclyloxycarbonyl.

Examples of R⁵ and R⁶ include each independently hydrogen or unsubstituted alkyl.

R⁷ includes hydrogen, substituted or unsubstituted alkyl, substituted or unsubstituted alkenyl, substituted or unsubstituted alkynyl or substituted or unsubstituted acyl.

Examples of R⁷ include hydrogen or substituted or unsubstituted alkyl.

n and m include each independently an integer of 0 to 3.

Examples of n and m include 0 simultaneously.

R¹ includes substituted or unsubstituted alkyl, substituted or unsubstituted alkenyl, substituted or unsubstituted alkynyl, substituted or unsubstituted acyl, cyano, carboxy, substituted or unsubstituted alkoxycarbonyl, substituted or unsubstituted alkenyloxycarbonyl, substituted or unsubstituted alkynyloxycarbonyl, substituted or unsubstituted carbamoyl, substituted or unsubstituted thiocarbamoyl, a substituted or unsubstituted carbocyclic group or a substituted or unsubstituted heterocyclic group.

Examples of R¹ include unsubstituted alkyl of a carbon number of 1 to 3.

R^{2a} and R^{2b} include each independently hydrogen, substituted or unsubstituted alkyl, substituted or unsubstituted acyl, substituted or unsubstituted alkoxycarbonyl or substituted or unsubstituted carbamoyl.

Examples of R^{2a} and R^{2a} are both hydrogen.

R^{3a}, R^{3b}, R^{4a} and R^{4b} include each independently
hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or
unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl.

Examples of R^{3a}, R^{3b}, R^{4a} and R^{4b} include each independently
hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclyloxycarbonyl, substituted or unsubstituted heterocyclylsulfinyl or substituted or unsubstituted heterocyclylsulfonyl.

Examples of R^{3a}, R^{3b}, R^{4a} and R^{4b} are all hydrogen.

R^{3a} and R^{3b} together with the carbon atom to which they are attached may form a substituted or unsubstituted carbocycle or a substituted or unsubstituted heterocycle.

R^{4a} and R^{4b} together with the carbon atom to which they are attached may form a substituted or unsubstituted carbocycle or a substituted or unsubstituted heterocycle.

R^{3a} and R^{4a} together with the carbon atom to which they are attached may form a substituted or unsubstituted carbocycle or a substituted or unsubstituted heterocycle.

For example, R^{3a} and R^{4a} together with the carbon atom to which they are attached form a substituted or unsubstituted benzene ring.

Dashed line means the presence or absence of a bond.

When dashed line means the presence of a bond, then R^{3b} and R^{4b} are absent.

When -X- is -O- or -N(R^{x})-, and R^{3a} and R^{4a} together with the carbon atom to which they are attached form a substituted or unsubstituted benzene ring, then ring A is not (i) phenyl substituted only with halogen or (ii) phenyl substituted with substituted or unsubstituted phenyl at m-position.

Specific embodiments of the present invention are the compound of formula:

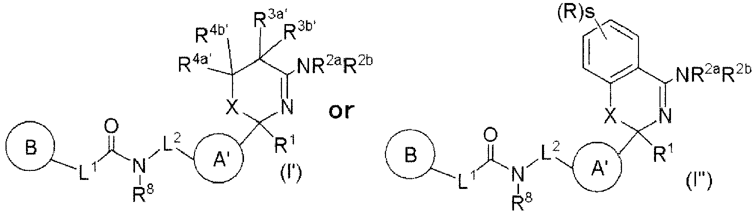

wherein R^{3a'}, R^{3b'}, R^{4a'} and R^{4b'} are each independently
hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclyloxycarbonyl, substituted or unsubstituted heterocyclylsulfinyl or substituted or unsubstituted heterocyclylsulfonyl,
R is a substituent selected from the substituent group α, alkyl substituted with the substituent group α, unsubstituted alkyl, alkenyl substituted with the substituent group α, unsubstituted alkenyl, alkynyl substituted with the substituent group α or unsubstituted alkynyl,
s is an integer of 0 to 3, and
other symbols are as defined above,
its salt, or a solvate thereof.

Specific embodiments are the compound of the above formula (I') or (I") wherein
-X- is -O- or -S-,
R¹is unsubstituted alkyl,
R^{2a} and R^{2b} are hydroxy,
R^{3a'}, R^{3b'}, R^{4a'} and R^{4b'} are each independently hydrogen, halogen, hydroxy, alkyl, alkenyl, alkynyl, alkoxy, alkenyloxy, alkynyloxy, alkylthio, alkenylthio, alkynylthio, acyl, acyloxy, cyano, nitro, carboxy, alkoxycarbonyl, amino, carbamoyl or thiocarbamoyl, sulfamoyl,
Ring A' is substituted or unsubstituted benzene,
herein, the substituent is preferably halogen, hydroxy, alkyl, alkenyl, alkynyl, alkoxy, alkenyloxy, alkynyloxy, acyl, carboxy, alkoxycarbonyl, amino or cyano,
ring B is substituted or unsubstituted pyridine, substituted or unsubstituted pyrimidine or substituted or unsubstituted pyrazine,
herein, the substituent is preferably halogen, hydroxy, alkyl, alkenyl, alkynyl, alkoxy, alkenyloxy, alkynyloxy, acyl, carboxy, alkoxycarbonyl, amino or cyano,
L¹ and L² are a bond,
R⁸ is hydrogen, alkyl or acyl,
R is halogen, hydroxy, alkyl, alkenyl, alkynyl, alkoxy, acyl, carboxy, alkoxycarbonyl, amino, cyano, a carbocyclic group or a heterocyclic group,
herein the carbocyclic group and the heterocyclic group may be substituted with one or more substituents selected form a group consisting halogen, hydroxy, alkyl and alkoxy, and
s is an integer of 0 to 2,
its salt, or a solvate thereof.

In another aspect, specific embodiments are the compound of formula (I) wherein 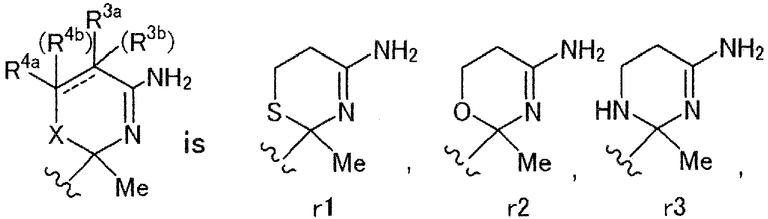 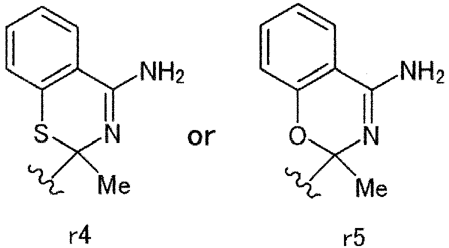 wherein Me is methyl.

The compound of formula (I) wherein Y is a bond. Hereinafter referred to as "Y is y1".
The compound of formula (I) wherein Y is -CH₂-. Hereinafter referred to as "Y is y2".

The compound of formula (I) wherein ring A is

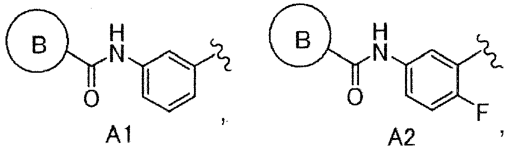

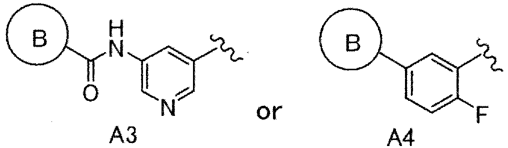

The compound of formula (I) wherein ring B is 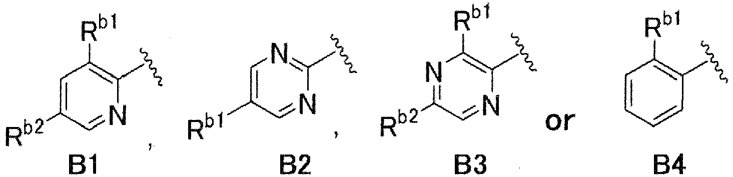 wherein R^{b1} and R^{b2} are each independently hydrogen, chloro, fluoro, methoxy, butynyloxy, cyano, amino, or carbamoyl.

The compound of formula (I) wherein the combination of Ring B, R^{b1} and R^{b2} (B, R^{b1}, R^{b2}) is as follows:
(B1, hydrogen, hydrogen) Hereinafter referred to as "Ring B is b1".
(B1, hydrogen, chloro) Hereinafter referred to as "Ring B isb2".
(B1, hydrogen, fluoro) Hereinafter referred to as "Ring B is b3".
(B1, hydrogen, methoxy) Hereinafter referred to as "Ring B is b4".
(B1, hydrogen, butynyloxy) Hereinafter referred to as "Ring B is b5".
(B1, hydrogen, cyano) Hereinafter referred to as "Ring B is b6".
(B1, hydrogen, amino) Hereinafter referred to as "Ring B is b7".
(B1, hydrogen, carbamoyl) Hereinafter referred to as "Ring B is b8".
(B1, chloro, hydrogen) Hereinafter referred to as "Ring B is b9".
(B1, chloro, chloro) Hereinafter referred to as "Ring B is b10".
(B1, chloro, fluoro) Hereinafter referred to as "Ring B is b11".
(B1, chloro, methoxy) Hereinafter referred to as "Ring B is b12".
(B1, chloro, butynyloxy) Hereinafter referred to as "Ring B is b13".
(B1, chloro, cyano) Hereinafter referred to as "Ring B is b14".
(B1, chloro, amino) Hereinafter referred to as "Ring B is b15".
(B1, chloro, carbamoyl) Hereinafter referred to as "Ring B is b16".
(B1, fluoro, hydrogen) Hereinafter referred to as "Ring B is b17".
(B1, fluoro, chloro) Hereinafter referred to as "Ring B is b18".
(B1, fluoro, fluoro) Hereinafter referred to as "Ring B is b19".
(B1, fluoro, methoxy) Hereinafter referred to as "Ring B is b20".
(B1, fluoro, butynyloxy) Hereinafter referred to as "Ring B is b21".
(B1, fluoro, cyano) Hereinafter referred to as "Ring B is b22".
(B1, fluoro, amino) Hereinafter referred to as "Ring B is b23".
(B1, fluoro, carbamoyl) Hereinafter referred to as "Ring B is b24".
(B I, methoxy, hydrogen) Hereinafter referred to as "Ring B is b25".
(B1, methoxy, chloro) Hereinafter referred to as "Ring B is b26".
(B1, methoxy, fluoro) Hereinafter referred to as "Ring B is b27".
(B1, methoxy, methoxy) Hereinafter referred to as "Ring B is b28".
(B1, methoxy, butynyloxy) Hereinafter referred to as "Ring B is b29".
(B1, methoxy, cyano) Hereinafter referred to as "Ring B is b30".
(B1, methoxy, amino) Hereinafter referred to as "Ring B is b31".
(B1, methoxy, carbamoyl) Hereinafter referred to as "Ring B is b32".
(B1, butynyloxy, hydrogen) Hereinafter referred to as "Ring B is b33".
(B1, butynyloxy, chloro) Hereinafter referred to as "Ring B is b34".
(B1, butynyloxy, fluoro) Hereinafter referred to as "Ring B is b35".
(B1, butynyloxy, methoxy) Hereinafter referred to as "Ring B is b36".
(B1, butynyloxy, cyano) Hereinafter referred to as "Ring B is b37".
(B1, butynyloxy, amino) Hereinafter referred to as "Ring B is b38".,
(B1, butynyloxy, carbamoyl) Hereinafter referred to as "Ring B is b39".
(B1, cyano, hydrogen) Hereinafter referred to as "Ring B is b40".
(B1, cyano, chloro) Hereinafter referred to as "Ring B is b41".
(B1, cyano, fluoro) Hereinafter referred to as "Ring B is b42".
(B1, cyano, methoxy) Hereinafter referred to as "Ring B is b43".
(B1, cyano, butynyloxy) Hereinafter referred to as "Ring B is b44".
(B1, cyano, cyano) Hereinafter referred to as "Ring B is b45".
(B1, cyano, amino) Hereinafter referred to as "Ring B is b46".
(B1, cyano, carbamoyl) Hereinafter referred to as "Ring B is b47".
(B1, amino, hydrogen) Hereinafter referred to as "Ring B is b48".
(B1, amino, chloro) Hereinafter referred to as "Ring B is b49".
(B1, amino, fluoro) Hereinafter referred to as "Ring B is b50".
(B1, amino, methoxy) Hereinafter referred to as "Ring B is b51".
(B1, amino, butynyloxy) Hereinafter referred to as "Ring B is b52".
(B1, amino, cyano) Hereinafter referred to as "Ring B is b53".
(B1, carbamoyl, hydrogen) Hereinafter referred to as "Ring B is b54".
(B1, carbamoyl, chloro) Hereinafter referred to as "Ring B is b55".
(B1, carbamoyl, fluoro) Hereinafter referred to as "Ring B is b56".
(B1, carbamoyl, methoxy) Hereinafter referred to as "Ring B is b57".
(B1, carbamoyl, butynyloxy) Hereinafter referred to as "Ring B is b58".
(B1, carbamoyl, cyano) Hereinafter referred to as "Ring B is b59".

(B3, hydrogen, hydrogen) Hereinafter referred to as "Ring B is b60".
(B3, hydrogen, chloro) Hereinafter referred to as "Ring B is b61".
(B3, hydrogen, fluoro) Hereinafter referred to as "Ring B is b62".
(B3, hydrogen, methoxy) Hereinafter referred to as "Ring B is b63".
(B3, hydrogen, butynyloxy) Hereinafter referred to as "Ring B is b64".
(B3, hydrogen, cyano) Hereinafter referred to as "Ring B is b65".
(B3, hydrogen, amino) Hereinafter referred to as "Ring B is b66".
(B3, hydrogen, carbamoyl) Hereinafter referred to as "Ring B is b67".
(B3, chloro, hydrogen) Hereinafter referred to as "Ring B is b68".
(B3, chloro, chloro) Hereinafter referred to as "Ring B is b69".
(B3, chloro, fluoro) Hereinafter referred to as "Ring B is b70".
(B3, chloro, methoxy) Hereinafter referred to as "Ring B is b71".
(B3, chloro, butynyloxy) Hereinafter referred to as "Ring B is b72".
(B3, chloro, cyano) Hereinafter referred to as "Ring B is b73".
(B3, chloro, amino) Hereinafter referred to as "Ring B is b74".
(B3, chloro, carbamoyl) Hereinafter referred to as "Ring B is b75".
(B3, fluoro, hydrogen) Hereinafter referred to as "Ring B is b76".
(B3, fluoro, chloro) Hereinafter referred to as "Ring B is b77".
(B3, fluoro, fluoro) Hereinafter referred to as "Ring B is b78".
(B3, fluoro, methoxy) Hereinafter referred to as "Ring B is b79".
(B3, fluoro, butynyloxy) Hereinafter referred to as "Ring B is b80".
(B3, fluoro, cyano) Hereinafter referred to as "Ring B is b81".
(B3, fluoro, amino) Hereinafter referred to as "Ring B is b82".
(B3, fluoro, carbamoyl) Hereinafter referred to as "Ring B is b83".
(B3, methoxy, hydrogen) Hereinafter referred to as "Ring B is b84".
(B3, methoxy, chloro) Hereinafter referred to as "Ring B is b85".
(B3, methoxy, fluoro) Hereinafter referred to as "Ring B is b86".
(B3, methoxy, methoxy) Hereinafter referred to as "Ring B is b87".
(B3, methoxy, butynyloxy) Hereinafter referred to as "Ring B is b88".
(B3, methoxy, cyano) Hereinafter referred to as "Ring B is b89".
(B3, methoxy, amino) Hereinafter referred to as "Ring B is b90".
(B3, methoxy, carbamoyl) Hereinafter referred to as "Ring B is b91".
(B3, butynyloxy, hydrogen) Hereinafter referred to as "Ring B is b92".
(B3, butynyloxy, chloro) Hereinafter referred to as "Ring B is b93".
(B3, butynyloxy, fluoro) Hereinafter referred to as "Ring B is b94".
(B3, butynyloxy, methoxy) Hereinafter referred to as "Ring B is b95".
(B3, butynyloxy, cyano) Hereinafter referred to as "Ring B is b96".
(B3, butynyloxy, amino) Hereinafter referred to as "Ring B is b97".
(B3, butynyloxy, carbamoyl) Hereinafter referred to as "Ring B is b98".
(B3, cyano, hydrogen) Hereinafter referred to as "Ring B is b99".
(B3, cyano, chloro) Hereinafter referred to as "Ring B is b100".
(B3, cyano, fluoro) Hereinafter referred to as "Ring B is b101".
(B3, cyano, methoxy) Hereinafter referred to as "Ring B is b102".
(B3, cyano, butynyloxy) Hereinafter referred to as "Ring B is b 103".
(B3, cyano, cyano) Hereinafter referred to as "Ring B is b 104".
(B3, cyano, amino) Hereinafter referred to as "Ring B is b105".
(B3, cyano, carbamoyl) Hereinafter referred to as "Ring B is b 106".
(B3, amino, hydrogen) Hereinafter referred to as "Ring B is b107".
(B3, amino, chloro) Hereinafter referred to as "Ring B is b108".
(B3, amino, fluoro) Hereinafter referred to as "Ring B is b109".
(B3, amino, methoxy) Hereinafter referred to as "Ring B is b110".
(B3, amino, butynyloxy) Hereinafter referred to as "Ring B is b111".
(B3, amino, cyano) Hereinafter referred to as "Ring B is b 112".
(B3, carbamoyl, hydrogen) Hereinafter referred to as "Ring B is b113".
(B3, carbamoyl, chloro) Hereinafter referred to as "Ring B is b114".
(B3, carbamoyl, fluoro) Hereinafter referred to as "Ring B is b115".
(B3, carbamoyl, methoxy) Hereinafter referred to as "Ring B is b116".
(B3, carbamoyl, butynyloxy) Hereinafter referred to as "Ring B is b117".
(B3, carbamoyl, cyano) Hereinafter referred to as "Ring B is b118".

The compound of formula (I) wherein the combination of Ring B and R^{1b}, (B, R^{1b}) is as follows:
(B2, hydrogen) Hereinafter referred to as "Ring B is b119".
(B2, chloro) Hereinafter referred to as "Ring B is b120".
(B2, fluoro) Hereinafter referred to as "Ring B is b121".
(B2, methoxy) Hereinafter referred to as "Ring B is b 122".
(B2, butynyloxy) Hereinafter referred to as "Ring B is b123".
(B2, cyano) Hereinafter referred to as "Ring B is b124".
(B2, amino) Hereinafter referred to as "Ring B is b125".
(B2, carbamoyl) Hereinafter referred to as "Ring B is b126".
(B4, hydrogen) Hereinafter referred to as "Ring B is b127".
(B4, chloro) Hereinafter referred to as "Ring B is b128".
(B4, fluoro) Hereinafter referred to as "Ring B is b 129".
(B4, methoxy) Hereinafter referred to as "Ring B is b130".
(B4, butynyloxy) Hereinafter referred to as "Ring B is b131".
(B4, cyano) Hereinafter referred to as "Ring B is b132".
(B4, amino) Hereinafter referred to as "Ring B is b133".
(B4, carbamoyl) Hereinafter referred to as "Ring B is b134".

The compound of formula (I) wherein the combination of 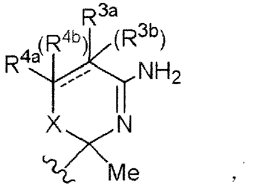 Y, Ring A and Ring B (r, y, A, b) is as follows:
(r1,y1,A1,b1),(r1,y1,A1,b2),(r1,y1,A1,b3),(r1,y1,A1,b4),(r1,y1,A1,b5),(r1,y1,A1,b6),(r1,y1,A1,b7),( r1,y1,A1,b8),(r1,y1,A1,b9),(r1,y1,A1,b10),(r1,y1,A1,b11),(r1,y1,A1,b12),(r1,y1,A1,b13),(r1,y1,A1,b 14),(r1,y1,A1,b15),(r1,y1,A1,b16),(r1,y1,A1,b17),(r1,y1,A1,b18),(r1,y1,A1,b19),(r1,y1,A1,b20),(r1, y1,A1,b21),(r1,y1,A1,b22),(r1,y1,A1,b23),(r1,y1,A1,b24),(r1,y1,A1,b25),(r1,y1,A1,b26),(r1,y1,A1,b 27),(r1,y1,A1,b28),(r1,y1,A1,b29),(r1,y1,A1,b30),(r1,y1,A1,b31),(r1,y1,A1,b32),(r1,y1,A1,b33),(r1, y1,A1,b34),(r1,y1,A1,b35),(r1,y1,A1,b36),(r1,y1,A1,b37),(r1,y1,A1,b38),(r1,y1,A1,b39),(r1,y1,A1,b 40),(r1,y1,A1,b41),(r1,y1,A1,b42),(r1,y1,A1,b43),(r1,y1,A1,b44),(r1,y1,A1,b45),(r1,y1,A1,b46),(r1, y1,A1,b47),(r1,y1,A1,b48),(r1,y1,A1,b49),(r1,y1,A1,b50),(r1,y1,A1,b51),(r1,y1,A1,b52),(r1,y1,A1,b 53),(r1,y1,A1,b54),(r1,y1,A1,b55),(r1,y1,A1,b56),(r1,y1,A1,b57),(r1,y1,A1,b58),(r1,y1,A1,b59),(r1, y1,A1,b60),(r1,y1,A1,b61),(r1,y1,A1,b62),(r1,y1,A1,b63),(r1,y1,A1,b64),(r1,y1,A1,b65),(r1,y1,A1,b 66),(r1,y1,A1,b67),(r1,y1,A1,b68),(r1,y1,A1,b69),(r1,y1,A1,b70),(r1,y1,A1,b71),(r1,y1,A1,b72),(r1, y1,A1,b73),(r1,y1,A1,b74),(r1,y1,A1,b75),(r1,y1,A1,b76),(r1,y1,A1,b77),(r1,y1,A1,b78),(r1,y1,A1,b 79),(r1,y1,A1,b80),(r1,y1,A1,b81),(r1,y1,A1,b82),(r1,y1,A1,b83),(r1,y1,A1,b84),(r1,y1,A1,b85),(r1, y1,A1,b86),(r1,y1,A1,b87),(r1,y1,A1,b88),(r1,y1,A1,b89),(r1,y1,A1,b90),(r1,y1,A1,b91),(r1,y1,A1,b 92),(r1,y1,A1,b93),(r1,y1,A1,b94),(r1,y1,A1,b95),(r1,y1,A1,b96),(r1,y1,A1,b97),(r1,y1,A1,b98),(r1, y1,A1,b99),(r1,y1,A1,b100),(r1,y1,A1,b101),(r1,y1,A1,b102),(r1,y1,A1,b103),(r1,y1,A1,b104),(r1,y 1,A1,b105),(r1,y1,A1,b106),(r1,y1,A1,b107),(r1,y1,A1,b108),(r1,y1,A1,b109),(r1,y1,A1,b110),(r1,y 1,A1,b111),(r1,y1,A1,b112),(r1,y1,A1,b113),(r1,y1,A1,b114),(r1,y1,A1,b115),(r1,y1,A1,b116),(r1,y 1,A1,b117),(r1,y1,A1,b118),(r1,y1,A1,b119),(r1,y1,A1,b120),(r1,y1,A1,b121),(r1,y1,A1,b122),(r1,y 1,A1,b123),(r1,y1,A1,b124),(r1,y1,A1,b125),(r1,y1,A1,b126),(r1,y1,A1,b127),(r1,y1,A1,b128),(r1,y 1,A1,b129),(r1,y1,A1,b130),(r1,y1,A1,b131),(r1,y1,A1,b132),(r1,y1,A1,b133),(r1,y1,A1,b134),(r1,y 1,A2,b1),(r1,y1,A2,b2),(r1,y1,A2,b3),(r1,y1,A2,b4),(r1,y1,A2,b5),(r1,y1,A2,b6),(r1,y1,A2,b7),(r1,y1 ,A2,b8),(r1,y1,A2,b9),(r1,y1,A2,b10),(r1,y1,A2,b11),(r1,y1,A2,b12),(r1,yl,A2,b13),(r1,y1,A2,b14),( r1,y1,A2,b15),(r1,y1,A2,b16),(r1,y1,A2,b17),(r1,y1,A2,b18),(r1,y1,A2,b19),(r1,y1,A2,b20),(r1,y1,A 2,b21),(r1,y1,A2,b22),(r1,y1,A2,b23),(r1,y1,A2,b24),(r1,y1,A2,b25),(r1,y1,A2,b26),(r1,y1,A2,b27),( r1,y1,A2,b28),(r1,y1,A2,b29),(r1,y1,A2,b30),(r1,y1,A2,b31),(r1,y1,A2,b32),(r1,y1,A2,b33),(r1,y1,A 2,b34),(r1,y1,A2,b35),(r1,y1,A2,b36),(r1,y1,A2,b37),(r1,y1,A2,b38),(r1,y1,A2,b39),(r1,y1,A2,b40),( r1,y1,A2,b41),(r1,y1,A2,b42),(r1,y1,A2,b43),(r1,y1,A2,b44),(r1,y1,A2,b45),(r1,y1,A2,b46),(r1,y1,A 2,b47),(r1,y1,A2,b48),(r1,y1,A2,b49),(r1,y1,A2,b50),(r1,y1,A2,b51),(r1,y1,A2,b52),(r1,y1,A2,b53),( r1,y1,A2,b54),(r1,y1,A2,b55),(r1,y1,A2,b56),(r1,y1,A2,b57),(r1,y1,A2,b58),(r1,y1,A2,b59),(r1,y1,A 2,b60),(r1,y1,A2,b61),(r1,y1,A2,b62),(r1,y1,A2,b63),(r1,y1,A2,b64),(r1,y1,A2,b65),(r1,y1,A2,b66),( r1,y1,A2,b67),(r1,y1,A2,b68),(r1,y1,A2,b69),(r1,y1,A2,b70),(r1,y1,A2,b71),(r1,y1,A2,b72),(r1,y1,A 2,b73),(r1,y1,A2,b74),(r1,y1,A2,b75),(r1,y1,A2,b76),(r1,y1,A2,b77),(r1,y1,A2,b78),(r1,y1,A2,b79),( r1,y1,A2,b80),(r1,y1,A2,b81),(r1,y1,A2,b82),(r1,y1,A2,b83),(r1,y1,A2,b84),(r1,y1,A2,b85),(r1,y1,A 2,b86),(r1,y1,A2,b87),(r1,y1,A2,b88),(r1,y1,A2,b89),(r1,y1,A2,b90),(r1,y1,A2,b91),(r1,y1,A2,b92),( r1,y1,A2,b93),(r1,y1,A2,b94),(r1,y1,A2,b95),(r1,y1,A2,b96),(r1,y1,A2,b97),(r1,y1,A2,b98),(r1,y1,A 2,b99),(r1,y1,A2,b100),(r1,y1,A2,b101),(r1,y1,A2,b102),(r1,y1,A2,b103),(r1,y1,A2,b104),(r1,y1,A2, b105),(r1,y1,A2,b106),(r1,y1,A2,b107),(r1,y1,A2,b108),(r1,y1,A2,b109),(r1,y1,A2,b110),(r1,y1,A2, b111),(r1,y1,A2,b112),(r1,y1,A2,b113),(r1,y1,A2,b114),(r1,y1,A2,b115),(r1,y1,A2,b116),(r1,y1,A2, b117),(r1,y1,A2,b118),(r1,y1,A2,b119),(r1,y1,A2,b120),(r1,y1,A2,b121),(r1,y1,A1,b122),(r1,y1,A2, b123),(r1,y1,A2,b124),(r1,y1,A2,b125),(r1,y1,A2,b126),(r1,y1,A2,b127),(r1,y1,A2,b128),(r1,y1,A2, b129),(r1,y1,A2,b130),(r1,y1,A2,b131),(r1,y1,A2,b132),(r1,y1,A2,b133),(r1,y1,A2,b134),(r1,y1,A3, b1),(r1,y1,A3,b2),(r1,y1,A3,b3),(r1,y1,A3,b4),(r1,y1,A3,b5),(r1,y1,A3,b6),(r1,y1,A3,b7),(r1,y1,A3,b 8),(r1,y1,A3,b9),(r1,y1,A3,b10),(r1,y1,A3,b11),(r1,y1,A3,b12),(r1,y1,A3,b13),(r1,y1,A3,b14),(r1,y1, A3,b15),(r1,y1,A3,b16),(r1,y1,A3,b17),(r1,y1,A3,b18),(r1,y1,A3,b19),(r1,y1,A3,b20),(r1,y1,A3,b21) ,(r1,y1,A3,b22),(r1,y1,A3,b23),(r1,y1,A3,b24),(r1,y1,A3,b25),(r1,y1,A3,b26),(r1,y1,A3,b27),(r1,y1, A3,b28),(r1,y1,A3,b29),(r1,y1,A3,b30),(r1,y1,A3,b31),(r1,y1,A3,b32),(r1,y1,A3,b33),(r1,y1,A3,b34) ,(r1,y1,A3,b35),(r1,y1,A3,b36),(r1,y1,A3,b37),(r1,y1,A3,b38),(r1,y1,A3,b39),(r1,y1,A3,b40),(r1,y1, A3,b41),(r1,y1,A3,b42),(r1,y1,A3,b43),(r1,y1,A3,b44),(r1,y1,A3,b45),(r1,y1,A3,b46),(r1,y1,A3,b47) ,(r1,y1,A3,b48),(r1,y1,A3,b49),(r1,y1,A3,b50),(r1,y1,A3,b51),(r1,y1,A3,b52),(r1,y1,A3,b53),(r1,y1, A3,b54),(r1,y1,A3,b55),(r1,y1,A3,b56),(r1,y1,A3,b57),(r1,y1,A3,b58),(r1,y1,A3,b59),(r1,y1,A3,b60) ,(r1,y1,A3,b61),(r1,y1,A3,b62),(r1,y1,A3,b63),(r1,y1,A3,b64),(r1,y1,A3,b65),(r1,y1,A3,b66),(r1,y1, A3,b67),(r1,y1,A3,b68),(r1,y1,A3,b69),(r1,y1,A3,b70),(r1,y1,A3,b71),(r1,y1,A3,b72),(r1,y1,A3,b73) ,(r1,y1,A3,b74),(r1,y1,A3,b75),(r1,y1,A3,b76),(r1,y1,A3,b77),(r1,y1,A3,b78),(r1,y1,A3,b79),(r1,y1, A3,b80),(r1,y1,A3,b81),(r1,y1,A3,b82),(r1,y1,A3,b83),(r1,y1,A3,b84),(r1,y1,A3,b85),(r1,y1,A3,b86) ,(r1,y1,A3,b87),(r1,y1,A3,b88),(r1,y1,A3,b89),(r1,y1,A3,b90),(r1,y1,A3,b91),(r1,y1,A3,b92),(r1,y1, A3,b93),(r1,y1,A3,b94),(r1,y1,A3,b95),(r1,y1,A3,b96),(r1,y1,A3,b97),(r1,y1,A3,b98),(r1,y1,A3,b99) ,(r1,y1,A3,b100),(r1,y1,A3,b101),(r1,y1,A3,b102),(r1,y1,A3,b103),(r1,y1,A3,b104),(r1,y1,A3,b105), (r1,y1,A3,b106),(r1,y1,A3,b107),(r1,y1,A3,b108),(r1,y1,A3,b109),(r1,y1,A3,b110),(r1,y1,A3,b111),( r1,y1,A3,b112),(r1,y1,A3,b113),(r1,y1,A3,b114),(r1,y1,A3,b115),(r1,y1,A3,b116),(r1,y1,A3,b117),( r1,y1,A3,b118),(r1,y1,A3,b119),(r1,y1,A3,b120),(r1,y1,A3,b121),(r1,y1,A3,b122),(r1,y1,A3,b123),( r1,y1,A3,b124),(r1,y1,A3,b125),(r1,y1,A3,b126),(r1,y1,A3,b127),(r1,y1,A3,b128),(r1,y1,A3,b129),( r1,y1,A3,b130),(r1,y1,A3,b131),(r1,y1,A3,b132),(r1,y1,A3,b133),(r1,y1,A3,b134),(r1,y1,A4,b1),(r1, y1,A4,b2),(r1,y1,A4,b3),(r1,y1,A4,b4),(r1,y1,A4,b5),(r1,y1,A4,b6),(r1,y1,A4,b7),(r1,y1,A4,b8),(r1,y 1,A4,b9),(r1,y1,A4,b10),(r1,y1,A4,b11),(r1,y1,A4,b12),(r1,y1,A4,b13),(r1,y1,A4,b14),(r1,y1,A4,b15 ),(r1,y1,A4,b16),(r1,y1,A4,b17),(r1,y1,A4,b18),(r1,y1,A4,b19),(r1,y1,A4,b20),(r1,y1,A4,b21),(r1,y1, A4,b22),(r1,y1,A4,b23),(r1,y1,A4,b24),(r1,y1,A4,b25),(r1,y1,A4,b26),(r1,y1,A4,b27),(r1,y1,A4,b28) ,(r1,y1,A4,b29),(r1,y1,A4,b30),(r1,y1,A4,b31),(r1,y1,A4,b32),(r1,y1,A4,b33),(r1,y1,A4,b34),(r1,y1, A4,b35),(r1,y1,A4,b36),(r1,y1,A4,b37),(r1,y1,A4,b38),(r1,y1,A4,b39),(r1,y1,A4,b40),(r1,y1,A4,b41) ,(r1,y1,A4,b42),(r1,y1,A4,b43),(r1,y1,A4,b44),(r1,y1,A4,b45),(r1,y1,A4,b46),(r1,y1,A4,b47),(r1,y1, A4,b48),(r1,y1,A4,b49),(r1,y1,A4,b50),(r1,y1,A4,b51),(r1,y1,A4,b52),(r1,y1,A4,b53),(r1,y1,A4,b54) ,(r1,y1,A4,b55),(r1,y1,A4,b56),(r1,y1,A4,b57),(r1,y1,A4,b58),(r1,y1,A4,b59),(r1,y1,A4,b60),(r1,y1, A4,b61),(r1,y1,A4,b62),(r1,y1,A4,b63),(r1,y1,A4,b64),(r1,y1,A4,b65),(r1,y1,A4,b66),(r1,y1,A4,b67) ,(r1,y1,A4,b68),(r1,y1,A4,b69),(r1,y1,A4,b70),(r1,y1,A4,b71),(r1,y1,A4,b72),(r1,y1,A4,b73),(r1,y1, A4,b74),(r1,y1,A4,b75),(r1,y1,A4,b76),(r1,y1,A4,b77),(r1,y1,A4,b78),(r1,y1,A4,b79),(r1,y1,A4,b80) ,(r1,y1,A4,b81),(r1,y1,A4,b82),(r1,y1,A4,b83),(r1,y1,A4,b84),(r1,y1,A4,b85),(r1,y1,A4,b86),(r1,y1, A4,b87),(r1,y1,A4,b88),(r1,y1,A4,b89),(r1,y1,A4,b90),(r1,y1,A4,b91),(r1,y1,A4,b92),(r1,y1,A4,b93) ,(r1,y1,A4,b94),(r1,y1,A4,b95),(r1,y1,A4,b96),(r1,y1,A4,b97),(r1,y1,A4,b98),(r1,y1,A4,b99),(r1,y1, A4,b100),(r1,y1,A4,b101),(r1,y1,A4,b102),(r1,y1,A4,b103),(r1,y1,A4,b104),(r1,y1,A4,b105),(r1,y1, A4,b106),(r1,y1,A4,b107),(r1,y1,A4,b108),(r1,y1,A4,b109),(r1,y1,A4,b110),(r1,y1,A4,b111),(r1,y1, A4,b112),(r1,y1,A4,b113),(r1,y1,A4,b114),(r1,y1,A4,b115),(r1,y1,A4,b116),(r1,y1,A4,b117),(r1,y1,
A4,b118),(r1,y1,A4,b119),(r1,y1,A4,b120),(r1,y1,A4,b121),(r1,y1,A4,b122),(r1y1,A4,b123),(r1,y1, A4,b124),(r1,y1,A4,b125),(r1,y1,A4,b126),(r1,y1,A4,b127),(r1,y1,A4,b128),(r1,y1,A4,b129),(r1,y1, A4,b130),(r1,y1,A4,b131),(r1,y1,A4,b132),(r1,y1,A4,b133),(r1,y1,A4,b134),

(r1,y2,A1,b1),(r1,y2,A1,b2),(r1,y2,A1,b3),(r1,y2,A1,b4),(r1,y2,A1,b5),(r1,y2,A1,b6),(r1,y2,A1,b7),( r1,y2,A1,b8),(r1,y2,A1,b9),(r1,y2,A1,b10),(r1,y2,A1,b11),(r1,y2,A1,b12),(r1,y2,A1,b13),(r1,y2,A1,b 14),(r1,y2,A1,b15),(r1,y2,A1,b16),(r1,y2,A1,b17),(r1,y2,A1,b18),(r1,y2,A1,b19),(r1,y2,A1,b20),(r1, y2,A1,b21),(r1,y2,A1,b22),(r1,y2,A1,b23),(r1,y2,A1,b24),(r1,y2,A1,b25),(r1,y2,A1,b26),(r1,y2,A1,b 27),(r1,y2,A1,b28),(r1,y2,A1,b29),(r1,y2,A1,b30),(r1,y2,A1,b31),(r1,y2,A1,b32),(r1,y2,A1,b33),(r1, y2,A1,b34),(r1,y2,A1,b35),(r1,y2,A1,b36),(r1,y2,A1,b37),(r1,y2,A1,b38),(r1,y2,A1,b39),(r1,y2,A1,b 40),(r1,y2,A1,b41),(r1,y2,A1,b42),(r1,y2,A1,b43),(r1,y2,A1,b44),(r1,y2,A1,b45),(r1,y2,A1,b46),(r1, y2,A1,b47),(r1,y2,A1,b48),(r1,y2,A1,b49),(r1,y2,A1,b50),(r1,y2,A1,b51),(r1,y2,A1,b52),(r1,y2,A1,b 53),(r1,y2,A1,b54),(r1,y2,A1,b55),(r1,y2,A1,b56),(r1,y2,A1,b57),(r1,y2,A1,b58),(r1,y2,A1,b59),(r1, y2,A1,b60),(r1,y2,A1,b61),(r1,y2,A1,b62),(r1,y2,A1,b63),(r1,y2,A1,b64),(r1,y2,A1,b65),(r1,y2,A1,b 66),(r1,y2,A1,b67),(r1,y2,A1,b68),(r1,y2,A1,b69),(r1,y2,A1,b70),(r1,y2,A1,b71),(r1,y2,A1,b72),(r1, y2,A1,b73),(r1,y2,A1,b74),(r1,y2,A1,b75),(r1,y2,A1,b76),(r1,y2,A1,b77),(r1,y2,A1,b78),(r1,y2,A1,b 79),(r1,y2,A1,b80),(r1,y2,A1,b81),(r1,y2,A1,b82),(r1,y2,A1,b83),(r1,y2,A1,b84),(r1,y2,A1,b85),(r1, y2,A1,b86),(r1,y2,A1,b87),(r1,y2,A1,b88),(r1,y2,A1,b89),(r1,y2,A1,b90),(r1,y2,A1,b91),(r1,y2,A1,b 92),(r1,y2,A1,b93),(r1,y2,A1,b94),(r1,y2,A1,b95),(r1,y2,A1,b96),(r1,y2,A1,b97),(r1,y2,A1,b98),(r1, y2,A1,b99),(r1,y2,A1,b100),(r1,y2,A1,b101),(r1,y2,A1,b102),(r1,y2,A1,b103),(r1,y2,A1,b104),(r1,y 2,A1,b105),(r1,y2,A1,b106),(r1,y2,A1,b107),(r1,y2,A1,b108),(r1,y2,A1,b109),(r1,y2,A1,b110),(r1,y 2,A1,b111),(r1,y2,A1,b112),(r1,y2,A1,b113),(r1,y2,A1,b114),(r1,y2,A1,b115),(r1,y2,A1,b116),(r1,y 2,A1,b117),(r1,y2,A1,b118),(r1,y2,A1,b119),(r1,y2,A1,b120),(r1,y2,A1,b121),(r1,y2,A1,b122),(r1,y 2,A1,b123),(r1,y2,A1,b124),(r1,y2,A1,b125),(r1,y2,A1,b126),(r1,y2,A1,b127),(r1,y2,A1,b128),(r1,y 2,A1,b129),(r1,y2,A1,b130),(r1,y2,A1,b131),(r1,y2,A1,b132),(r1,y2,A1,b133),(r1,y2,A1,b114),(r1,y 2,A2,b1),(r1,y2,A2,b2),(r1,y2,A2,b3),(r1,y2,A2,b4),(r1,y2,A2,b5),(r1,y2,A2,b6),(r1,y2,A2,b7),(r1,y2 ,A2,b8),(r1,y2,A2,b9),(r1,y2,A2,b10),(r1,y2,A2,b11),(r1,y2,A2,b12),(r1,y2,A2,b13),(r1,y2,A2,b14),( r1,y2,A2,b15),(r1,y2,A2,b16),(r1,y2,A2,b17),(r1,y2,A2,b18),(r1,y2,A2,b19),(r1,y2,A2,b20),(r1,y2,A 2,b21),(r1,y2,A2,b22),(r1,y2,A2,b23),(r1,y2,A2,b24),(r1,y2,A2,b25),(r1,y2,A2,b26),(r1,y2,A2,b27),( r1,y2,A2,b28),(r1,y2,A2,b29),(r1,y2,A2,b30),(r1,y2,A2,b31),(r1,y2,A2,b32),(r1,y2,A2,b33),(r1,y2,A 2,b34),(r1,y2,A2,b35),(r1,y2,A2,b36),(r1,y2,A2,b37),(r1,y2,A2,b38),(r1,y2,A2,b39),(r1,y2,A2,b40),( r1,y2,A2,b41),(r1,y2,A2,b42),(r1,y2,A2,b43),(r1,y2,A2,b44),(r1,y2,A2,b45),(r1,y2,A2,b46),(r1,y2,A 2,b47),(r1,y2,A2,b48),(r1,y2,A2,b49),(r1,y2,A2,b50),(r1,y2,A2,b51),(r1,y2,A2,b52),(r1,y2,A2,b53),( r1,y2,A2,b54),(r1,y2,A2,b55),(r1,y2,A2,b56),(r1,y2,A2,b57),(r1,y2,A2,b58),(r1,y2,A2,b59),(r1,y2,A 2,b60),(r1,y2,A2,b61),(r1,y2,A2,b62),(r1,y2,A2,b63),(r1,y2,A2,b64),(r1,y2,A2,b65),(r1,y2,A2,b66),( r1,y2,A2,b67),(r1,y2,A2,b68),(r1,y2,A2,b69),(r1,y2,A2,b70),(r1,y2,A2,b71),(r1,y2,A2,b72),(r1,y2,A 2,b73),(r1,y2,A2,b74),(r1,y2,A2,b75),(r1,A2,A2,b76),(r1,y2,A2,b77),(r1,y2,A2,b78),(r1,y2,A2,b79),( r1,y2,A2,b80),(r1,y2,A2,b81),(r1,y2,A2,b82),(r1,y2,A2,b83),(r1,y2,A2,b84),(r1,y2,A2,b85),(r1,y2,A 2,b86),(r1,y2,A2,b87),(r1,y2,A2,b88),(r1,y2,A2,b89),(r1,y2,A2,b90),(r1,y2,A2,b91),(r1,y2,A2,b92),( r1,y2,A2,b93),(r1,y2,A2,b94),(r1,y2,A2,b95),(r1,y2,A2,b96),(r1,y2,A2,b97),(r1,y2,A2,b98),(r1,y2,A 2,b99),(r1,y2,A2,b100),(r1,y2,A2,b101),(r1,y2,A2,b102),(r1,y2,A2,b103),(r1,y2,A2,b104),(r1,y2,A2, b105),(r1,y2,A2,b106),(r1,y2,A2,b107),(r1,y2,A2,b108),(r1,y2,A2,b109),(r1,y2,A2,b110),(r1,y2,A2, b111),(r1,y2,A2,b112),(r1,y2,A2,b113),(r1,y2,A2,b114),(r1,y2,A2,b115),(r1,y2,A2,b116),(r1,y2,A2, b117),(r1,y2,A2,b118),(r1,y2,A2,b119),(r1,y2,A2,b120),(r1,y2,A2,b121),(r1,y2,A2,b122),(r1,y2,A2, b123),(r1,y2,A2,b124),(r1,y2,A2,b125),(r1,y2,A2,b126),(r1,y2,A2,b127),(r1,y2,A2,b128),(r1,y2,A2, b129),(r1,y2,A2,b130),(r1,y2,A2,b131),(r1,y2,A2,b132),(r1,y2,A2,b133),(r1,y2,A2,b134),(r1,y2,A3, b1),(r1,y2,A3,b2),(r1,y2,A3,b3),(r1,y2,A3,b4),(r1,y2,A3,b5),(r1,y2,A3,b6),(r1,y2,A3,b7),(r1,y2,A3,b 8),(r1,y2,A3,b9),(r1,y2,A3,b10),(r1,y2,A3,b11),(r1,y2,A3,b12),(r1,y2,A3,b13),(r1,y2,A3,b14),(r1,y2, A3,b15),(r1,y2,A3,b16),(r1,y2,A3,b17),(r1,y2,A3,b18),(r1,y2,A3,b19),(r1,y2,A3,b20),(r1,y2,A3,b21) ,(r1,y2,A3,b22),(r1,y2,A3,b23),(r1,y2,A3,b24),(r1,y2,A3,b25),(r1,y2,A3,b26),(r1,y2,A3,b27),(r1,y2, A3,b28),(r1,y2,A3,b29),(r1,y2,A3,b30),(r1,y2,A3,b31),(r1,y2,A3,b32),(r1,y2,A3,b33),(r1,y2,A3,b34) ,(r1,y2,A3,b35),(r1,y2,A3,b36),(r1,y2,A3,b37),(r1,y2,A3,b38),(r1,y2,A3,b39),(r1,y2,A3,b40),(r1,y2, A3,b41),(r1,y2,A3,b42),(r1,y2,A3,b43),(r1,y2,A3,b44),(r1,y2,A3,b45),(r1,y2,A3,b46),(r1,y2,A3,b47) ,(r1,y2,A3,b48),(r1,y2,A3,b49),(r1,y2,A3,b50),(r1,y2,A3,b51),(r1,y2,A3,b52),(r1,y2,A3,b53),(r1,y2, A3,b54),(r1,y2,A3,b55),(r1,y2,A3,b56),(r1,y2,A3,b57),(r1,y2,A3,b58),(r1,y2,A3,b59),(r1,y2,A3,b60) ,(r1,y2,A3,b61),(r1,y2,A3,b62),(r1,y2,A3,b63),(r1,y2,A3,b64),(r1,y2,A3,b65),(r1,y2,A3,b66),(r1,y2, A3,b67),(r1,y2,A3,b68),(r1,y2,A3,b69),(r1,y2,A3,b70),(r1,y2,A3,b71),(r1,y2,A3,b72),(r1,y2,A3,b73) ,(r1,y2,A3,b74),(r1,y2,A3,b75),(r1,y2,A3,b76),(r1,y2,A3,b77),(r1,y2,A3,b78),(r1,y2,A3,b79),(r1,y2, A3,b80),(r1,y2,A3,b81),(r1,y2,A3,b82),(r1,y2,A3,b83),(r1,y2,A3,b84),(r1,y2,A3,b85),(r1,y2,A3,b86) ,(r1,y2,A3,b87),(r1,y2,A3,b88),(r1,y2,A3,b89),(r1,y2,A3,b90),(r1,y2,A3,b91),(r1,y2,A3,b92),(r1,y2, A3,b93),(r1,y2,A3,b94),(r1,y2,A3,b95),(r1,y2,A3,b96),(r1,y2,A3,b97),(r1,y2,A3,b98),(r1,y2,A3,b99) ,(r1,y2,A3,b100),(r1,y2,A3,b101),(r1,y2,A13,b102),(r1,y2,A3,b103),(r1,y2,A3,b104),(r1,y2,A3,b105), (r1,y2,A3,b106),(r1,y2,A3,b107),(r1,y2,A3,b108),(r1,y2,A3,b109),(r1,y2,A3,b110),(r1,y2,A3,b111),( r1,y2,A3,b112),(r1,y2,A3,b113),(r1,y2,A3,b114),(r1,y2,A3,b115),(r1,y2,A3,b116),(r1,y2,A3,b117),( r1,y2,A3,b118),(r1,y2,A3,b119),(r1,y2,A3,b120),(r1,y2,A3,b121),(r1,y2,A3,b122),(r1,y2,A3,b123),( r1,y2,A3,b124),(r1,y2,A3,b125),(r1,y2,A3,b126),(r1,y2,A3,b127),(r1,y2,A3,b128),(r1,y2,A3,b129),( r1,y2,A3,b130),(r1,y2,A3,b131),(r1,y2,A3,b132),(r1,y2,A3,b133),(r1,y2,A3,b134),(r1,y2,A4,b1),(r1, y2,A4,b2),(r1,y2,A4,b3),(r1,y2,A4,b4),(r1,y2,A4,b5),(r1,y2,A4,b6),(r1,y2,A4,b7),(r1,y2,A4,b8),(r1,y 2,A4,b9),(r1,y2,A4,b10),(r1,y2,A4,b11),(r1,y2,A4,b12),(r1,y2,A4,b13),(r1,y2,A4,b14),(r1,y2,A4,b15 ),(r1,y2,A4,b16),(r1,y2,A4,b17),(r1,y2,A4,b18),(r1,y2,A4,b19),(r1,y2,A4,b20),(r1,y2,A4,b21),(r1,y1, A4,b22),(r1,y2,A4,b23),(r1,y2,A4,b24),(r1,y2,A4,b25),(r1,y2,A4,b26),(r1,y2,A4,b27),(r1,y2,A4,b28) ,(r1,y2,A4,b29),(r1,y2,A4,b30),(r1,y2,A4,b31),(r1,y2,A4,b32),(r1,y2,A4,b33),(r1,y2,A4,b34),(r1,y2, A4,b35),(r1,y1,2,A4,b36),(r1,y2,A4,b37),(r1,y1,2,A4,b38),(r1,y2,A4,b39),(r1,y2,A4,b40),(r1,y2,44,b41) ,(r1,y2,A4,b42),(r1,y2,A4,b43),(r1,y2,A4,b44),(r1,y2,A4,b45),(r1,y2,A4,b46),(r1,y2,A4,b47),(r1,y2, A4,b48),(r1,y2,A4,b49),(r1,y2,A4,b50),(r1,y2,A4,b51),(r1,y2,A4,b52),(r1,y2,A4,b53),(r1,y2,A4,b54) ,(r1,y2,A4,b55),(r1,y2,A4,b56),(r1,y2,A4,b57),(r1,y2,A4,b58),(r1,y2,A4,b59),(r1,y2,A4,b60),(r1,y2, A4,b61),(r1,y2,A4,b62),(r1,y2,A4,b63),(r1,y2,A4,b64),(r1,y2,A4,b65),(r1,y2,A4,b66),(r1,y2,A4,b67) ,(r1,y2,A4,b68),(r1,y2,A4,b69),(r1,y2,A4,b70),(r1,y2,A4,b71),(r1,y2,A4,b72),(r1,y2,A4,b73),(r1,y2, A4,b74),(r1,y2,A4,b75),(r1,y2,A4,b76),(r1,y2,A4,b77),(r1,y2,A4,b78),(r1,y2,A4,b79),(r1,y2,A4,b80) ,(r1,y2,A4,b81),(r1,y2,A4,b82),(r1,y2,A4,b83),(r1,y2,A4,b84),(r1,y2,A4,b85),(r1,y2,A4,b86),(r1,y2, A4,b87),(r1,y2,A4,b88),(r1,y2,A4,b89),(r1,y2,A4,b90),(r1,y2,A4,b91),(r1,y2,A4,b92),(r1,y2,A4,b93) ,(r1,y2,A4,b94),(r1,y2,A4,b95),(r1,y2,A4,b96),(r1,y2,A4,b97),(r1,y2,A4,b98),(r1,y2,A4,b99),(r1,y2, A4,b100),(r1,y2,A4,b101),(r1,y2,A4,b102),(r1,y2,A4,b103),(r1,y2,A4,b104),(r1,y2,A4,b105),(r1,y2, A4,b106),(r1,y2,A4,b107),(r1,y2,A4,b108),(r1,y2,A4,b109),(r1,y2,A4,b110),(r1,y2,A4,b111),(r1,y2, A4,b112),(r1,y2,A4,b113),(r1,y2,A4,b114),(r1,y2,A4,b115),(r1,y2,A4,b116),(r1,y2,A4,b117),(r1,y2, A4,b118),(r1,y2,A4,b119),(r1,y2,A4,b120),(r1,y2,A4,b121),(r1,y2,A4,b122),(r1,y2,A4,b123),(r1,y2, A4,b124),(r1,y2,A4,b125),(r1,y2,A4,b126),(r1,y2,A4,b127),(r1,y2,A4,b128),(r1,y2,A4,b129),(r1,y2, A4,b130),(r1,y2,A4,b131),(r1,y2,A4,b132),(r1,y2,A4,b133),(r1,y2,A4,b134),

(r2,y1,A1,b1),(r2,y1,A1,b2),(r2,y1,A1,b3),(r2,y1,A1,b4),(r2,y1,A1,b5),(r2,y1,A1,b6),(r2,y1,A1,b7),( r2,y1,A1,b8),(r2,y1,A1,b9),(r2,y1,A1,b10),(r2,y1,A1,b11),(r2,y1,A1,b12),(r2,y1,A1,b13),(r2,y1,A1,b 14),(r2,y1,A1,b15),(r2,y1,A1,b16),(r2,y1,A1,b17),(r2,y1,A1,b18),(r2,y1,A1,b19),(r2,y1,A1,b20),(r2, y1,A1,b21),(r2,y1,A1,b22),(r2,y1,A1,b23),(r2,y1,A1,b24),(r2,y1,A1,b25),(r2,y1,A1,b26),(r2,y1,A1,b 27),(r2,y1,A1,b28),(r2,y1,A1,b29),(r2,y1,A1,b30),(r2,y1,A1,b31),(r2,y1,A1,b32),(r2,y1,A1,b33),(r2, y1,A1,b34),(r2,y1,A1,b35),(r2,y1,A1,b36),(r2,y1,A1,b37),(r2,y1,A1,b38),(r2,y1,A1,b39),(r2,y1,A1,b 40),(r2,y1,A1,b41),(r2,y1,A1,b42),(r2,y1,A1,b43),(r2,y1,A1,b44),(r2,y1,A1,b45),(r2,y1,A1,b46),(r2, y1,A1,b47),(r2,y1,A1,b48),(r2,y1,A1,b49),(r2,y1,A1,b50),(r2,y1,A1,b51),(r2,y1,A1,b52),(r2,y1,A1,b 53),(r2,y1,A1,b54),(r2,y1,A1,b55),(r2,y1,A1,b56),(r2,y1,A1,b57),(r2,y1,A1,b58),(r2,y1,A1,b59),(r2, y1,A1,b60),(r2,y1,A1,b61),(r2,y1,A1,b62),(r2,y1,A1,b63),(r2,y1,A1,b64),(r2,y1,A1,b65),(r2,y1,A1,b 66),(r2,y1,A1,b67),(r2,y1,A1,b68),(r2,y1,A1,b69),(r2,y1,A1,b70),(r2,y1,A1,b71),(r2,y1,A1,b72),(r2, y1,A1,b73),(r2,y1,A1,b74),(r2,y1,A1,b75),(r2,y1,A1,b76),(r2,y1,A1,b77),(r2,y1,A1,b78),(r2,y1,A1,b 79),(r2,y1,A1,b80),(r2,y1,A1,b81),(r2,y1,A1,b82),(r2,y1,A1,b83),(r2,y1,A1,b84),(r2,y1,A1,b85),(r2, y1,A1,b86),(r2,y1,A1,b87),(r2,y1,A1,b88),(r2,y1,A1,b89),(r2,y1,A1,b90),(r2,y1,A1,b91),(r2,y1,A1,b 92),(r2,y1,A1,b93),(r2,y1,A1,b94),(r2,y1,A1,b95),(r2,y1,A1,b96),(r2,y1,A1,b97),(r2,y1,A1,b98),(r2, y1,A1,b99),(r2,y1,A1,b100),(r2,y1,A1,b101),(r2,y1,A1,b102),(r2,y1,A1,b103),(r2,y1,A1,b104),(r2,y 1,A1,b105),(r2,y1,A1,b106),(r2,y1,A1,b107),(r2,y1,A1,b108),(r2,y1,A1,b109),(r2,y1,A1,b110),(r2,y 1,A1,b111),(r2,y1,A1,b112),(r2,y1,A1,b113),(r2,y1,A1,b114),(r2,y1,A1,b115),(r2,y1,A1,b116),(r2,y 1,A1,b117),(r2,y1,A1,b118),(r2,y1,A1,b119),(r2,y1,A1,b120),(r2,y1,A1,b121),(r2,y1,A1,b122),(r2,y 1,A1,b123),(r2,y1,A1,b124),(r2,y1,A1,b125),(r2,y1,A1,b126),(r2,y1,A1,b127),(r2,y1,A1,b128),(r2,y 1,A1,b129),(r2,y1,A1,b130),(r2,y1,A1,b131),(r2,y1,A1,b132),(r2,y1,A1,b133),(r2,y1,A1,b134),(r2,y 1,A2,b1),(r2,y1,A2,b2),(r2,y1,A2,b3),(r2,y1,A2,b4),(r2,y1,A2,b5),(r2,y1,A2,b6),(r2,y1,A2,b7),(r2,y1 ,A2,b8),(r2,y1,A2,b9),(r2,y1,A2,b10),(r2,y1,A2,b11),(r2,y1,A2,b12),(r2,y1,A2,b13),(r2,y1,A2,b14),( r2,y1,A2,b15),(r2,y1,A2,b16),(r2,y,A2,b17),(r2,y1,A2,b18),(r2,y1,A2,b19),(r2,y1,A2,b20),(r2,y1,A 2,b21),(r2,y1,A2,b22),(r2,y1,A2,b23),(r2,y1,A2,b24),(r2,y1,A2,b25),(r2,y1,A2,b26),(r2,y1,A2,b27),( r2,y1,A2,b28),(r2,y1,A2,b29),(r2,y1,A2,b30),(r2,y1,A2,b31),(r2,y1,A2,b32),(r2,y1,A2,b33),(r2,y1,A 2,b34),(r2,y1,A2,b35),(r2,y1,A2,b36),(r2,y1,A2,b37),(r2,y1,A2,b38),(r2,y1,A2,b39),(r2,y1,A2,b40),( r2,y1,A2,b41),(r2,y1,A2,b42),(r2,y1,A2,b43),(r2,y1,A2,b44),(r2,y1,A2,b45),(r2,y1,A2,b46),(r2,y1,A 2,b47),(r2,y1,A2,b48),(r2,y1,A2,b49),(r2,y1,A2,b50),(r2,y1,A2,b51),(r2,y1,A2,b52),(r2,y1,A2,b53),( r2,y1,A2,b54),(r2,y1,A2,b55),(r2,y1,A2,b56),(r2,y1,A2,b57),(r2,y1,A2,b58),(r2,y1,A2,b59),(r2,y1,A 2,b60),(r2,y1,A2,b61),(r2,y1,A2,b62),(r2,y1,A2,b63),(r2,y1,A2,b64),(r2,y1,A2,b65),(r2,y1,A2,b66),( r2,y1,A2,b67),(r2,y1,A2,b68),(r2,y1,A2,b69),(r2,y1,A2,b70),(r2,y1,A2,b71),(r2,y1,A2,b72),(r2,y1,A 2,b73),(r2,y1,A2,b74),(r2,y1,A2,b75),(r2,y1,A2,b76),(r2,y1,A2,b77),(r2,y1,A2,b78),(r2,y1,A2,b79),( r2,y1,A2,b80),(r2,y1,A2,b81),(r2,y1,A2,b82),(r2,y1,A2,b83),(r2,y1,A2,b84),(r2,y1,A2,b85),(r2,y1,A 2,b86),(r2,y1,A2,b87),(r2,y1,A2,b88),(r2,y1,A2,b89),(r2,y1,A2,b90),(r2,y1,A2,b91),(r2,y1,A2,b92),( r2,y1,A2,b93),(r2,y1,A2,b94),(r2,y1,A2,b95),(r2,y1,A2,b96),(r2,y1,A2,b97),(r2,y1,A2,b98),(r2,y1,A 2,b99),(r2,y1,A2,b100),(r2,y1,A2,b101),(r2,y1,A2,b102),(r2,y1,A2,b103),(r2,y1,A2,b104),(r2,y1,A2, b105),(r2,y1,A2,b106),(r2,y1,A2,b107),(r2,y1,A2,b108),(r2,y1,A2,b109),(r2,y1,A2,b110),(r2,y1,A2, b111),(r2,y1,A2,b112),(r2,y1,A2,b113),(r2,y1,A2,b114),(r2,y1,A2,b115),(r2,y1,A2,b116),(r2,y1,A2, b117),(r2,y1,A2,b118),(r2,y1,A2,b119),(r2,y1,A2,b120),(r2,y1,A2,b121),(r2,y1,A2,b122),(r2,y1,A2, b123),(r2,y1,A2,b124),(r2,y1,A2,b125),(r2,y1,A2,b126),(r2,y1,A2,b127),(r2,y1,A2,b128),(r2,y1,A2, b129),(r2,y1,A2,b130),(r2,y1,A2,b131),(r2,y1,A2,b132),(r2,y1,A2,b133),(r2,y1,A2,b134),(r2,y1,A3, b1),(r2,y1,A3,b2),(r2,y1,A3,b3),(r2,y1,A3,b4),(r2,y1,A3,b5),(r2,y1,A3,b6),(r2,y1,A3,b7),(r2,y1,A3,b 8),(r2,y1,A3,b9),(r2,y1,A3,b10),(r2,y1,A3,b11),(r2,y1,A3,b12),(r2,y1,A3,b13),(r2,y1,A3,b14),(r2,y1, A3,b15),(r2,y1,A3,b16),(r2,y1,A3,b17),(r2,y1,A3,b18),(r2,y1,A3,b19),(r2,y1,A3,b20),(r2,y1,A3,b21) ,(r2,y1,A3,b22),(r2,y1,A3,b23),(r2,y1,A3,b24),(r2,y1,A3,b25),(r2,y1,A3,b26),(r2,y1,A3,b27),(r2,y1, A3,b28),(r2,y1,A3,b29),(r2,y1,A3,b30),(r2,y1,A3,b31),(r2,y1,A3,b32),(r2,y1,A3,b33),(r2,y1,A3,b34) ,(r2,y1,A3,b35),(r2,y1,A3,b36),(r2,y1,A3,b37),(r2,y1,A3,b38),(r2,y1,A3,b39),(r2,y1,A3,b40),(r2,y1, A3,b41),(r2,y1,A3,b42),(r2,y1,A3,b43),(r2,y1,A3,b44),(r2,y1,A3,b45),(r2,y1,A3,b46),(r2,y1,A3,b47) ,(r2,y1,A3,b48),(r2,y1,A3,b49),(r2,y1,A3,b50),(r2,y1,A3,b51),(r2,y1,A3,b52),(r2,y1,A3,b53),(r2,y1, A3,b54),(r2,y1,A3,b55),(r2,y1,A3,b56),(r2,y1,A3,b57),(r2,y1,A3,b58),(r2,y1,A3,b59),(r2,y1,A3,b60) ,(r2,y1,A3,b61),(r2,y1,A3,b62),(r2,y1,A3,b63),(r2,y1,A3,b64),(r2,y1,A3,b65),(r2,y1,A3,b66),(r2,y1, A3,b67),(r2,y1,A3,b68),(r2,y1,A3,b69),(r2,y1,A3,b70),(r2,y1,A3,b71),(r2,y1,A3,b72),(r2,y1,A3,b73) ,(r2,y1,A3,b74),(r2,y1,A3,b75),(r2,y1,A3,b76),(r2,y1,A3,b77),(r2,y1,A3,b78),(r2,y1,A3,b79),(r2,y1, A3,b80),(r2,y1,A3,b81),(r2,y1,A3,b82),(r2,y1,A3,b83),(r2,y1,A3,b84),(r2,y1,A3,b85),(r2,y1,A3,b86) ,(r2,y1,A3,b87),(r2,y1,A3,b88),(r2,y1,A3,b89),(r2,y1,A3,b90),(r2,y1,A3,b91),(r2,y1,A3,b92),(r2,y1, A3,b93),(r2,y1,A3,b94),(r2,y1,A3,b95),(r2,y1,A3,b96),(r2,y1,A3,b97),(r2,y1,A3,b98),(r2,y1,A3,b99) ,(r2,y1,A3,b100),(r2,y1,A3,b101),(r2,y1,A3,b102),(r2,y1,A3,b103),(r2,y1,A3,b104),(r2,y1,A3,b105), (r2,y1,A3,b106),(r2,y1,A3,b107),(r2,y1,A3,b108),(r2,y1,A3,b109),(r2,y1,A3,b110),(r2,y1,A3,b111),( r2,y1,A3,b112),(r2,y1,A3,b113),(r2,y1,A3,b114),(r2,y1,A3,b115),(r2,y1,A3,b116),(r2,y1,A3,b117),( r2,y1,A3,b118),(r2,y1,A3,b119),(r2,y1,A3,b120),(r2,y1,A3,b121),(r2,y1,A3,b122),(r2,y1,A3,b123),( r2,y1,A3,b124),(r2,y1,A3,b125),(r2,y1,A3,b126),(r2,y1,A3,b127),(r2,y1,A3,b128),(r2,y1,A3,b129),( r2,y1,A3,b130),(r2,y1,A3,b131),(r2,y1,A3,b132),(r2,y1,A3,b133),(r2,y1,A3,b134),(r2,y1,A4,b1),(r2, y1,A4,b2),(r2,y1,A4,b3),(r2,y1,A4,b4),(r2,y1,A4,b5),(r2,y1,A4,b6),(r2,y1,A4,b7),(r2,y1,A4,b8),(r2,y 1,A4,b9),(r2,y1,A4,b10),(r2,y1,A4,b11),(r2,y1,A4,b12),(r2,y1,A4,b13),(r2,y1,A4,b14),(r2,y1,A4,b15 ),(r2,y1,A4,b16),(r2,y1,A4,b17),(r2,y1,A4,b18),(r2,y1,A4,b19),(r2,y1,A4,b20),(r2,y1,A4,b21),(r2,y1, A4,b22),(r2,y1,A4,b23),(r2,y1,A4,b24),(r2,y1,A4,b25),(r2,y1,A4,b26),(r2,y1,A4,b27),(r2,y1,A4,b28) ,(r2,y1,A4,b29),(r2,y1,A4,b30),(r2,y1,A4,b31),(r2,y1,A4,b32),(r2,y1,A4,b33),(r2,y1,A4,b34),(r2,y1, A4,b35),(r2,y1,A4,b36),(r2,y1,A4,b37),(r2,y1,A4,b38),(r2,y1,A4,b39),(r2,y1,A4,b40),(r2,y1,A4,b41) ,(r2,y1,A4,b42),(r2,y1,A4,b43),(r2,y1,A4,b44),(r2,y1,A4,b45),(r2,y1,A4,b46),(r2,y1,A4,b47),(r2,y1, A4,b48),(r2,y1,A4,b49),(r2,y1,A4,b50),(r2,y1,A4,b51),(r2,y1,A4,b52),(r2,y1,A4,b53),(r2,y1,A4,b54) ,(r2,y1,A4,b55),(r2,y1,A4,b56),(r2,y1,A4,b57),(r2,y1,A4,b58),(r2,y1,A4,b59),(r2,y1,A4,b60),(r2,y1, A4,b61),(r2,y1,A4,b62),(r2,y1,A4,b63),(r2,y1,A4,b64),(r2,y1,A4,b65),(r2,y1,A4,b66),(r2,y1,A4,b67) ,(r2,y1,A4,b68),(r2,y1,A4,b69),(r2,y1,A4,b70),(r2,y1,A4,b71),(r2,y1,A4,b72),(r2,y1,A4,b73),(r2,y1, A4,b74),(r2,y1,A4,b75),(r2,y1,A4,b76),(r2,y1,A4,b77),(r2,y1,A4,b78),(r2,y1,A4,b79),(r2,y1,A4,b80) ,(r2,y1,A4,b81),(r2,y1,A4,b82),(r2,y1,A4,b83),(r2,y1,A4,b84),(r2,y1,A4,b85),(r2,y1,A4,b86),(r2,y1, A4,b87),(r2,y1,A4,b88),(r2,y1,A4,b89),(r2,y1,A4,b90),(r2,y1,A4,b91),(r2,y1,A4,b92),(r2,y1,A4,b93) ,(r2,y1,A4,b94),(r2,y1,A4,b95),(r2,y1,A4,b96),(r2,y1,A4,b97),(r2,y1,A4,b98),(r2,y1,A4,b99),(r2,y1, A4,b100),(r2,y1,A4,b101),(r2,y1,A4,b102),(r2,y1,A4,b103),(r2,y1,A4,b104),(r2,y1,A4,b105),(r2,y1, A4,b106),(r2,y1,A4,b107),(r2,y1,A4,b108),(r2,y1,A4,b109),(r2,y1,A4,b110),(r2,y1,A4,b111),(r2,y1, A4,b112),(r2,y1,A4,b113),(r2,y1,A4,b114),(r2,y1,A4,b115),(r2,y1,A4,b116),(r2,y1,A4,b117),(r2,y1, A4,b118),(r2,y1,A4,b119),(r2,y1,A4,b120),(r2,y1,A4,b121),(r2,y1,A4,b122),(r2,y1,A4,b123),(r2,y1, A4,b124),(r2,y1,A4,b125),(r2,y1,A4,b126),(r2,y1,A4,b127),(r2,y1,A4,b128),(r2,y1,A4,b129),(r2,y1, A4,b130),(r2,y1,A4,b131),(r2,y1,A4,b132),(r2,y1,A4,b133),(r2,y1,A4,b134),

(r2,y2,A1,b1),(r2,y2,A1,b2),(r2,y2,A1,b3),(r2,y2,A1,b4),(r2,y2,A1,b5),(r2,y2,A1,b6),(r2,y2,A1,b7),( r2,y2,A1,b8),(r2,y2,A1,b9),(r2,y2,A1,b10),(r2,y2,A1,b11),(r2,y2,A1,b12),(r2,y2,A1,b13),(r2,y2,A1,b 14),(r2,y2,A1,b15),(r2,y2,A1,b16),(r2,y2,A1,b17),(r2,y2,A1,b18),(r2,y2,A1,b19),(r2,y2,A1,b20),(r2, y2,A1,b21),(r2,y2,A1,b22),(r2,y2,A1,b23),(r2,y2,A1,b24),(r2,y2,A1,b25),(r2,y2,A1,b26),(r2,y2,A1,b 27),(r2,y2,A1,b28),(r2,y2,A1,b29),(r2,y2,A1,b30),(r2,y2,A1,b31),(r2,y2,A1,b32),(r2,y2,A1,b33),(r2, y2,A1,b34),(r2,y2,A1,b35),(r2,y2,A1,b36),(r2,y2,A1,b37),(r2,y2,A1,b38),(r2,y2,A1,b39),(r2,y2,A1,b 40),(r2,y2,A1,b41),(r2,y2,A1,b42),(r2,y2,A1,b43),(r2,y2,A1,b44),(r2,y2,A1,b45),(r2,y2,A1,b46),(r2, y2,A1,b47),(r2,y2,A1,b48),(r2,y2,A1,b49),(r2,y2,A1,b50),(r2,y2,A1,b51),(r2,y2,A1,b52),(r2,y2,A1,b 53),(r2,y2,A1,b54),(r2,y2,A1,b55),(r2,y2,A1,b56),(r2,y2,A1,b57),(r2,y2,A1,b58),(r2,y2,A1,b59),(r2, y2,A1,b60),(r2,y2,A1,b61),(r2,y2,A1,b62),(r2,y2,A1,b63),(r2,y2,A1,b64),(r2,y2,A1,b65),(r2,y2,A1,b 66),(r2,y2,A1,b67),(r2,y2,A1,b68),(r2,y2,A1,b69),(r2,y2,A1,b70),(r2,y2,A1,b71),(r2,y2,A1,b72),(r2, y2,A1,b73),(r2,y2,A1,b74),(r2,y2,A1,b75),(r2,y2,A1,b76),(r2,y2,A1,b77),(r2,y2,A1,b78),(r2,y2,Al,b 79),(r2,y2,A1,b80),(r2,y2,A1,b81),(r2,y2,A1,b82),(r2,y2,A1,b83),(r2,y2,A1,b84),(r2,y2,A1,b85),(r2, y2,A1,b86),(r2,y2,A1,b87),(r2,y2,A1,b88),(r2,y2,A1,b89),(r2,y2,A1,b90),(r2,y2,A1,b91),(r2,y2,A1,b 92),(r2,y2,A1,b93),(r2,y2,A1,b94),(r2,y2,A1,b95),(r2,y2,A1,b96),(r2,y2,A1,b97),(r2,y2,A1,b98),(r2, y2,A1,b99),(r2,y2,A1,b100),(r2,y2,A1,b101),(r2,y2,A1,b102),(r2,y2,A1,b103),(r2,y2,A1,b104),(r2,y 2,A1,b105),(r2,y2,A1,b106),(r2,y2,A1,b107),(r2,y2,A1,b108),(r2,y2,A1,b109),(r2,y2,A1,b110),(r2,y 2,A1,b111),(r2,y2,A1,b112),(r2,y2,A1,b113),(r2,y2,A1,b114),(r2,y2,A1,b115),(r2,y2,A1,b116),(r2,y 2,A1,b117),(r2,y2,A1,b118),(r2,y2,A1,b119),(r2,y2,A1,b120),(r2,y2,A1,b121),(r2,y2,A1,b122),(r2,y 2,A1,b123),(r2,y2,A1,b124),(r2,y2,A1,b125),(r2,y2,A1,b126),(r2,y2,A1,b127),(r2,y2,A1,b128),(r2,y 2,A1,b129),(r2,y2,A1,b130),(r2,y2,A1,b131),(r2,y2,A1,b132),(r2,y2,A1,b133),(r2,y2,A1,b134),(r2,y 2,A2,b1),(r2,y2,A2,b2),(r2,y2,A2,b3),(r2,y2,A2,b4),(r2,y2,A2,b5),(r2,y2,A2,b6),(r2,y2,A2,b7),(r2,y2 ,A2,b8),(r2,y2,A2,b9),(r2,y2,A2,b10),(r2,y2,A2,b11),(r2,y2,A2,b12),(r2,y2,A2,b13),(r2,y2,A2,b14),( r2,y2,A2,b15),(r2,y2,A2,b16),(r2,y2,A2,b17),(r2,y2,A2,b18),(r2,y2,A2,b19),(r2,y2,A2,b20),(r2,y2,A 2,b21),(r2,y2,A2,b22),(r2,y2,A2,b23),(r2,y2,A2,b24),(r2,y2,A2,b25),(r2,y2,A2,b26),(r2,y2,A2,b27),( r2,y2,A2,b28),(r2,y2,A2,b29),(r2,y2,A2,b30),(r2,y2,A2,b31),(r2,y2,A2,b32),(r2,y2,A2,b33),(r2,y2,A 2,b34),(r2,y2,A2,b35),(r2,y2,A2,b36),(r2,y2,A2,b37),(r2,y2,A2,b38),(r2,y2,A2,b39),(r2,y2,A2,b40),( r2,y2,A2,b41),(r2,y2,A2,b42),(r2,y2,A2,b43),(r2,y2,A2,b44),(r2,y2,A2,b45),(r2,y2,A2,b46),(r2,y2,A 2,b47),(r2,y2,A2,b48),(r2,y2,A2,b49),(r2,y2,A2,b50),(r2,y2,A2,b51),(r2,y2,A2,b52),(r2,y2,A2,b53),( r2,y2,A2,b54),(r2,y2,A2,b55),(r2,y2,A2,b56),(r2,y2,A2,b57),(r2,y2,A2,b58),(r2,y2,A2,b59),(r2,y2,A 2,b60),(r2,y2,A2,b61),(r2,y2,A2,b62),(r2,y2,A2,b63),(r2,y2,A2,b64),(r2,y2,A2,b65),(r2,y2,A2,b66),( r2,y2,A2,b67),(r2,y2,A2,b68),(r2,y2,A2,b69),(r2,y2,A2,b70),(r2,y2,A2,b71),(r2,y2,A2,b72),(r2,y2,A 2,b73),(r2,y2,A2,b74),(r2,y2,A2,b75),(r2,y2,A2,b76),(r2,y2,A2,b77),(r2,y2,A2,b78),(r2,y2,A2,b79),( r2,y2,A2,b80),(r2,y2,A2,b81),(r2,y2,A2,b82),(r2,y2,A2,b83),(r2,y2,A2,b84),(r2,y2,A2,b85),(r2,y2,A 2,b86),(r2,y2,A2,b87),(r2,y2,A2,b88),(r2,y2,A2,b89),(r2,y2,A2,b90),(r2,y2,A2,b91),(r2,y2,A2,b92),( r2,y2,A2,b93),(r2,y2,A2,b94),(r2,y2,A2,b95),(r2,y2,A2,b96),(r2,y2,A2,b97),(r2,y2,A2,b98),(r2,y2,A 2,b99),(r2,y2,A2,b100),(r2,y2,A2,b101),(r2,y2,A2,b102),(r2,y2,A2,b103),(r2,y2,A2,b104),(r2,y2,A2, b105),(r2,y2,A2,b106),(r2,y2,A2,b107),(r2,y2,A2,b108),(r2,y2,A2,b109),(r2,y2,A2,b110),(r2,y2,A2, b111),(r2,y2,A2,b112),(r2,y2,A2,b113),(r2,y2,A2,b114),(r2,y2,A2,b115),(r2,y2,A2,b116),(r2,y2,A2, b117),(r2,y2,A2,b118),(r2,y2,A2,b119),(r2,y2,A2,b120),(r2,y2,A2,b121),(r2,y2,A2,b122),(r2,y2,A2, b123),(r2,y2,A2,b124),(r2,y2,A2,b125),(r2,y2,A2,b126),(r2,y2,A2,b127),(r2,y2,A2,b128),(r2,y2,A2, b129),(r2,y2,A2,b130),(r2,y2,A2,b131),(r2,y2,A2,b132),(r2,y2,A2,b133),(r2,y2,A2,b134),(r2,y2,A3, b1),(r2,y2,A3,b2),(r2,y2,A3,b3),(r2,y2,A3,b4),(r2,y2,A3,b5),(r2,y2,A3,b6),(r2,y2,A3,b7),(r2,y2,A3,b 8),(r2,y2,A3,b9),(r2,y2,A3,b10),(r2,y2,A3,b11),(r2,y2,A3,b12),(r2,y2,A3,b13),(r2,y2,A3,b14),(r2,y2, A3,b15),(r2,y2,A3,b16),(r2,y2,A3,b17),(r2,y2,A3,b18),(r2,y2,A3,b19),(r2,y2,A3,b20),(r2,y2,A3,b21) ,(r2,y2,A3,b22),(r2,y2,A3,b23),(r2,y2,A3,b24),(r2,y2,A3,b25),(r2,y2,A3,b26),(r2,y2,A3,b27),(r2,y2, A3,b28),(r2,y2,A3,b29),(r2,y2,A3,b30),(r2,y2,A3,b31),(r2,y2,A3,b32),(r2,y2,A3,b33),(r2,y2,A3,b34) ,(r2,y2,A3,b35),(r2,y2,A3,b36),(r2,y2,A3,b37),(r2,y2,A3,b38),(r2,y2,A3,b39),(r2,y2,A3,b40),(r2,y2, A3,b41),(r2,y2,A3,b42),(r2,y2,A3,b43),(r2,y2,A3,b44),(r2,y2,A3,b45),(r2,y2,A3,b46),(r2,y2,A3,b47) ,(r2,y2,A3,b48),(r2,y2,A3,b49),(r2,y2,A3,b50),(r2,y2,A3,b51),(r2,y2,A3,b52),(r2,y2,A3,b53),(r2,y2, A3,b54),(r2,y2,A3,b55),(r2,y2,A3,b56),(r2,y2,A3,b57),(r2,y2,A3,b58),(r2,y2,A3,b59),(r2,y2,A3,b60) ,(r2,y2,A3,b61),(r2,y2,A3,b62),(r2,y2,A3,b63),(r2,y2,A3,b64),(r2,y2,A3,b65),(r2,y2,A3,b66),(r2,y2, A3,b67),(r2,y2,A3,b68),(r2,y2,A3,b69),(r2,y2,A3,b70),(r2,y2,A3,b71),(r2,y2,A3,b72),(r2,y2,A3,b73) ,(r2,y2,A3,b74),(r2,y2,A3,b75),(r2,y2,A3,b76),(r2,y2,A3,b77),(r2,y2,A3,b78),(r2,y2,A3,b79),(r2,y2, A3,b80),(r2,y2,A3,b81),(r2,y2,A3,b82),(r2,y2,A3,b83),(r2,y2,A3,b84),(r2,y2,A3,b85),(r2,y2,A3,b86) ,(r2,y2,A3,b87),(r2,y2,A3,b88),(r2,y2,A3,b89),(r2,y2,A3,b90),(r2,y2,A3,b91),(r2,y2,A3,b92),(r2,y2, A3,b93),(r2,y2,A3,b94),(r2,y2,A3,b95),(r2,y2,A3,b96),(r2,y2,A3,b97),(r2,y2,A3,b98),(r2,y2,A3,b99) ,(r2,y2,A3,b100),(r2,y2,A3,b101),(r2,y2,A3,b102),(r2,y2,A3,b103),(r2,y2,A3,b104),(r2,y2,A3,b105), (r2,y2,A3,b106),(r2,y2,A3,b107),(r2,y2,A3,b108),(r2,y2,A3,b109),(r2,y2,A3,b110),(r2,y2,A3,b111),( r2,y2,A3,b112),(r2,y2,A3,b113),(r2,y2,A3,b114),(r2,y2,A3,b115),(r2,y2,A3,b116),(r2,y2,A3,b117),( r2,y2,A3,b118),(r2,y2,A3,b119),(r2,y2,A3,b120),(r2,y2,A3,b121),(r2,y2,A3,b122),(r2,y2,A3,b123),( r2,y2,A3,b124),(r2,y2,A3,b125),(r2,y2,A3,b126),(r2,y2,A3,b127),(r2,y2,A3,b128),(r2,y2,A3,b129),( r2,y2,A3,b130),(r2,y2,A3,b131),(r2,y2,A3,b132),(r2,y2,A3,b133),(r2,y2,A3,b134),(r2,y2,A4,b1),(r2, y2,A4,b2),(r2,y2,A4,b3),(r2,y2,A4,b4),(r2,y2,A4,b5),(r2,y2,A4,b6),(r2,y2,A4,b7),(r2,y2,A4,b8),(r2,y 2,A4,b9),(r2,y2,A4,b10),(r2,y2,A4,b11),(r2,y2,A4,b12),(r2,y2,A4,b13),(r2,y2,A4,b14),(r2,y2,A4,b15 ),(r2,y2,A4,b16),(r2,y2,A4,b17),(r2,y2,A4,b18),(r2,y2,A4,b19),(r2,y2,A4,b20),(r2,y2,A4,b21),(r2,y2, A4,b22),(r2,y2,A4,b23),(r2,y2,A4,b24),(r2,y2,A4,b25),(r2,y2,A4,b26),(r2,y2,A4,b27),(r2,y2,A4,b28) ,(r2,y2,A4,b29),(r2,y2,A4,b30),(r2,y2,A4,b31),(r2,y2,A4,b32),(r2,y2,A4,b33),(r2,y2,A4,b34),(r2,y2, A4,b35),(r2,y2,A4,b36),(r2,y2,A4,b37),(r2,y2,A4,b38),(r2,y2,A4,b39),(r2,y2,A4,b40),(r2,y2,A4,b41) ,(r2,y2,A4,b42),(r2,y 2,A4,b43),(r2,y2,A4,b44),(r2,y2,A4,b45),(r2,y2,A4,b46),(r2,y2,A4,b47),(r2,y2, A4,b48),(r2,y2,A4,b49),(r2,y2,A4,b50),(r2,y2,A4,b51),(r2,y2,A4,b52),(r2,y2,A4,b53),(r2,y2,A4,b54) ,(r2,y2,A4,b55),(r2,y2,A4,b56),(r2,y2,A4,b57),(r2,y2,A4,b58),(r2,y2,A4,b59),(r2,y2,A4,b60),(r2,y2, A4,b61),(r2,y2,A4,b62),(r2,y2,A4,b63),(r2,y2,A4,b64),(r2,y2,A4,b65),(r2,y2,A4,b66),(r2,y2,A4,b67) ,(r2,y2,A4,b68),(r2,y2,A4,b69),(r2,y2,A4,b70),(r2,y2,A4,b71),(r2,y2,A4,b72),(r2,y2,A4,b73),(r2,y2, A4,b74),(r2,y2,A4,b75),(r2,y2,A4,b76),(r2,y2,A4,b77),(r2,y2,A4,b78),(r2,y2,A4,b79),(r2,y2,A4,b80) ,(r2,y2,A4,b81),(r2,y2,A4,b82),(r2,y2,A4,b83),(r2,y2,A4,b84),(r2,y2,A4,b85),(r2,y2,A4,b86),(r2,y2, A4,b87),(r2,y2,A4,b88),(r2,y2,A4,b89),(r2,y2,A4,b90),(r2,y2,A4,b91),(r2,y2,A4,b92),(r2,y2,A4,b93) ,(r2,y2,A4,b94),(r2,y2,A4,b95),(r2,y2,A4,b96),(r2,y2,A4,b97),(r2,y2,A4,b98),(r2,y2,A4,b99),(r2,y2, A4,b 100),(r2,y2,A4,b101),(r2,y2,A4,b102),(r2,y2,A4,b103),(r2,y2,A4,b104),(r2,y2,A4,b 105),(r2,y2, A4,b106),(r2,y2,A4,b107),(r2,y2,A4,b108),(r2,y2,A4,b109),(r2,y2,A4,b110),(r2,y2,A4,b111),(r2,y2, A4,b 112), (r2,y2,A4,b 113), (r2,y2,A4,b 114),(r2,y2,A4,b115),(r2,y2,A4,b116), (r2,y2,A4,b 117),(r2,y2, A4,b 118), (r2,y2,A4,b 119),(r2,y2,A4,b120),(r2,y2,A4,b121),(r2,y2,A4,b122),(r2,y2,A4,b123),(r2,y2, A4,b 124),(r2,y2,A4,b125),(r2,y2,A4,b126),(r2,y2,A4,b 127),(r2,y2,A4,b128),(r2,y2,A4,b129),(r2,y2, A4,b 130),(r2,y2,A4,b131),(r2,y2,A4,b132),(r2,y 2,A4,b133),(r2,y2,A4,b134),

(r3,y1,A1,b1),(r3,y1,A1,b2),(r3,y1,A1,b3),(r3,y1,A1,b4),(r3,y1,A1,b5),(r3,y1,A1,b6),(r3,y1,A1,b7),( r3,y1,A1,b8),(r3,y1,A1,b9),(r3,y1,A1,b10),(r3,y1,A1,b11),(r3,y1,A1,b12),(r3,y1,A1,b13),(r3,y1,A1,b 14),(r3,y1,A1,b15),(r3,y1,A1,b16),(r3,y1,A1,b17),(r3,y1,A1,b18),(r3,y1,A1,b19),(r3,y1,A1,b20),(r3, y1,A1,b21),(r3,y1,A1,b22),(r3,y1,A1,b23),(r1,y1,A1,b24),(r3,y1,A1,b25),(r3,y1,A1,b26),(r3,y1,A1,b 27),(r3,y1,A1,b28),(r3,y1,A1,b29),(r3,y1,A1,b30),(r3,y1,A1,b31),(r3,y1,A1,b32),(r3,y1,A1,b33),(r3, y1,A1,b34),(r3,y1,A1,b35),(r3,y1,A1,b36),(r3,y1,A1,b37),(r3,y1,A1,b38),(r3,y1,A1,b39),(r3,y1,A1,b 40),(r3,y1,A,b41),(r3,y1,A1,b42),(r3,y1,A1,b43),(r3,y1,A1,b44),(r3,y1,A1,b45),(r3,y1,A1,b46),(r3, y1,A1,b47),(r3,y1,A1,b48),(r3,y1,A1,b49),(r3,y1,A1,b50),(r3,y1,A1,b51),(r3,y1,A1,b52),(r3,y1,A1,b 53),(r3,y1,A1,b54),(r3,y1,A1,b55),(r3,y1,A1,b56),(r3,y1,A1,b57),(r3,y1,A1,b58),(r3,y1,A1,b59),(r3, y1,A1,b60),(r3,y1,A1,b61),(r3,y1,A1,b62),(r3,y1,A1,b63),(r3,y1,A1,b64),(r3,y1,A1,b65),(r3,y1,A1,b 66),(r3,y1,A1,b67),(r3,y1,A1,b68),(r3,y1,A1,b69),(r3,y1,A1,b70),(r3,y1,A1,b71),(r3,y1,A1,b72),(r3, y1,A1,b73),(r3,y1,A1,b74),(r3,y1,A1,b75),(r3,y1,A1,b76),(r3,y1,A1,b77),(r3,y1,A1,b78),(r3,y1,A1,b 79),(r3,y1,A1,b80),(r3,y1,A1,b81),(r3,y1,A1,b82),(r3,y1,A1,b83),(r3,y1,A1,b84),(r3,y1,A1,b85),(r3, y1,A1,b86),(r3,y1,A1,b87),(r3,y1,A1,b88),(r3,y1,A1,b89),(r3,y1,A1,b90),(r3,y1,A1,b91),(r3,y1,A1,b 92),(r3,y1,A1,b93),(r3,y1,A1,b94),(r3,y1,A1,b95),(r3,y1,A1,b96),(r3,y1,A1,b97),(r3,y1,A1,b98),(r3, y1,A1,b99),(r3,y1,A1,b100),(r3,y1,A1,b101),(r3,y1,A1,b102),(r3,y1,A1,b103),(r3,y1,A1,b104),(r3,y 1,A1,b105),(r3,y1,A1,b106),(r3,y1,A1,b107),(r3,y1,A1,b108),(r3,y1,A1,b109),(r3,y1,A1,b110),(r3,y 1,A1,b111),(r3,y1,A1,b112),(r3,y1,A1,b113),(r3,y1,A1,b114),(r3,y1,A1,b115),(r3,y1,A1,b116),(r3,y 1,A1,b117),(r3,y1,A1,b118),(r3,y1,A1,b119),(r3,y1,A1,b120),(r3,y1,A1,b121),(r3,y1,A1,b122),(r3,y 1,A1,b123),(r3,y1,A1,b124),(r3,y1,A1,b125),(r3,y1,A1b126),(r3,y1,A1,b127),(r3,y1,A1,b128),(r3,y 1,A1,b129),(r3,y1,A1,b130),(r3,y1,A1,b131),(r3,y1,A1,b132),(r3,y1,A1,b133),(r3,y1,A1,b134),(r3,y 1,A2,b1),(r3,y1,A2,b2),(r3,y1,A2,b3);(r3,y1,A2,b4),(r3,y1,A2,b5),(r3,y1,A2,b6),(r3,y1,A2,b7),(r3,y1 ,A2,b8),(r3,y1,A2,b9),(r3,y1,A2,b10),(r3,y1,A2,b11),(r3,y1A2,b12),(r3,y1A2,b13),(r3,y1A2,b14),( r3,y1,A2,b15),(r3,y1,A2,b16),(r3,y1,A2,b17),(r3,y1,A2,b18),(r3,y1,A2,b19),(r3,y1,A2,b20),(r3,y1,A 2,b21),(r3,y1,A2,b22),(r3,y1,A2,b23),(r3,y1,A2,b24),(r3,y1,A2,b25),(r3,y1,A2,b26),(r3,y1,A2,b27),( r3,y1,A2,b28),(r3,y1,A2,b29),(r3,y1,A2,b30),(r3,y1,A2,b31),(r3,y1,A2,b32),(r3,y1,A2,b33),(r3,y1,A 2,b34),(r3,y1,A2,b35),(r3,y1,A2,b36),(r3,y1,A2,b37),(r3,y1,A2,b38),(r3,y1,A2,b39),(r3,y1,A2,b40),( r3,y1,A2,b41),(r3,y1,A2,b42),(r3,y1,A2,b43),(r3,y1,A2,b44),(r3,y1,A2,b45),(r3,y1,A2,b46),(r3,y1,A 2,b47),(r3,y1,A2,b48),(r3,y1,A2,b49),(r3,y1,A2,b50),(r3,y1,A2,b51),(r3,y1,A2,b52),(r3,y1,A2,b53),( r3,y1,A2,b54),(r3,y1,A2,b55),(r3,y1,A2,b56),(r3,y1,A2,b57),(r3,y1,A2,b58),(r3,y1,A2,b59),(r3,y1,A 2,b60),(r3,y1,A2,b61),(r3,y1,A2,b62),(r3,y1,A2,b63),(r3,y1,A2,b64),(r3,y1,A2,b65),(r3,y1,A2,b66),( r3,y1,A2,b67),(r3,y1,A2,b68),(r3,y1,A2,b69),(r3,y1,A2,b70),(r3,y1,A2,b71),(r3,y1,A2,b72),(r3,y1,A 2,b73),(r3,y1,A2,b74),(r3,y1,A2,b75),(r3,y1,A2,b76),(r3,y1,A2,b77),(r3,y1,A2,b78),(r3,y1,A2,b79),( r3,y1,A2,b80),(r3,y1,A2,b81),(r3,y1,A2,b82),(r3,y1,A2,b83),(r3,y1,A2,b84),(r3,y1,A2,b85),(r3,y1,A 2,b86), (r3,y1,A2,b87),(r3,y1,A2,b88),(r3,y1,A2,b89),(r3,y1,A2,b90),(r3,y1,A2,b91),(r3,y,A2,b92),( r3,y1,A2,b93),(r3,y1,A2,b94),(r3,y1,A2,b95),(r3,y1,A2,b96),(r3,y1,A2,b97),(r3,y1,A2,b98),(r3,y1,A 2,b99),(r3,y1,A2,b100),(r3,y1,A2,b101),(r3,y1,A2,b102),(r3,y1,A2,b103),(r3,y1,A2,b104),(r3,y1,A2, b105),(r3,y1,A2,b106),(r3,y1,A2,b107), (r3,y1,A2,b108),(r3,y1,A2,b109),(r3,y1,A2,b110),(r3,y1,A2, b111),(r3,y1,A2,b112),(r3,y1,A2,b113),(r3,y1,A2,b114),(r3,y1,A2,b115),(r3,y1,A2,b116),(r3,y1,A2, b117),(r3,y1,A2,b118),(r3,y1,A2,b119),(r3,y1,A2,b120),(r3,y1,A2,b121),(r3,y1,A2,b122),(r3,y1,A2, b123),(r3,y1,A2,b124),(r3,y1,A2,b125),(r3,y1,A2,b126),(r3,y1,A2,b127),(r3,y1,A2,b128),(r3,y1,A2, b129),(r3,y1,A2,b130),(r3,y1,A2,b131),(r3,y1,A2,b132),(r3,y1,A2,b133),(r3,y1,A2,b134),(r3,y1,A3, b1),(r3,y1,A3,b2),(r3,y1,A3,b3),(r3,y1,A3,b4),(r3,y1,A3,b5),(r3,y1,A3,b6),(r3,y1,A3,b7),(r3,y1,A3,b 8),(r3,y1,A3,b9),(r3,y1,A3,b10),(r3,y1,A3,b11),(r3,y1,A3,b12),(r3,y1,A3,b13),(r3,y1,A3,b14),(r3,y1, A3,b15),(r3,y1,A3,b16),(r3,y1,A3,b17),(r3,y1,A3,b18),(r3,y1,A3,b19),(r3,y1,A3,b20),(r3,y1,A3,b21) ,(r3,yl,A3,b22),(r3,y1,A3,b23),(r3,y1,A3,b24),(r3,y1,A3,b25),(r3,y1,A3,b26),(r3,y1,A3,b27),(r3,y1, A3,b28),(r3,y1,A3,b29),(r3,y1,A3,b30),(r3,y1,A3,b31),(r3,y1,A3,b32),(r3,y1,A3,b33),(r3,y1,A3,b34) ,(r3,y1,A3,b35),(r3,y1,A3,b36),(r3,y1,A3,b37),(r3,y1,A3,b38),(r3,y1,A3,b39),(r3,y1,A3,b40),(r3,y1, A3,b41),(r3,y1,A3,b42),(r3,y1,A3,b43),(r3,y1,A3,b44),(r3,y1,A3,b45),(r3,y1,A3,b46),(r3,y1,A3,b47) ,(r3,y1,A3,b48),(r3,y1,A3,b49),(r3,y1,A3,b50),(r3,y1,A3,b51),(r3,y1,A3,b52),(r3,y1,A3,b53),(r3,y1, A3,b54),(r3,y1,A3,b55),(r3,y1,A3,b56),(r3,y1,A3,b57),(r3,yl,A3,b58),(r3,y1,A3,b59),(r3,y1,A3,b60) ,(r3,y1,A3,b61),(r3,y1,A3,b62),(r3,y1,A3,b63),(r3,y1,A3,b64),(r3,y1,A3,b65),(r3,y1,A3,b66),(r3,y1, A3,b67),(r3,y1,A3,b68),(r3,y1,A3,b69),(r3,y1,A3,b70),(r3,y1,A3,b71),(r3,y1,A3,b72),(r3,y1,A3,b73) ,(r3,y1,A3,b74),(r3.y1,A3,b75),(r3,y1,A3,b76),(r3,y1,A3,b77),(r3,y1,A3,b78),(r3,y1,A3,b79),(r3,y1, A3,b80),(r3,y1,A3,b81),(r3,y1,A3,b82),(r3,y1,A3,b83),(r3,y1,A3,b84),(r3,y1,A3,b85),(r3,y1,A3,b86) ,(r3,y1,A3,b87),(r3,y1,A3,b88),(r3,y1,A3,b89),(r3,y1,A3,b90),(r3,y1,A3,b91),(r3,y1,A3,b92),(r3,y1, A3,b93),(r3,y1,A3,b94),(r3,y1,A3,b95),(r3,y1,A3,b96),(r3,y1,A3,b97),(r3,y1,A3,b98),(r3,y1,A3,b99) ,(r3,y1,A3,b100),(r3,y1,A3,b101),(r3,y1,A3,b102),(r3,y1,A3,b103),(r3,y1,A3,b104),(r3,y1,A3,b105), (r3,y1,A3,b106),(r3,y1,A3,b107),(r3,y1,A3,b108),(r3,y1,A3,b109),(r3,y1,A3,b110),(r3,y1,A3,b111),( r3,y1,A3,b112),(r3,y1,A3,b113),(r3,y1,A3,b114),(r3,y1,A3,b115),(r3,y1,A3,b116),(r3,y1,A3,b117),( r3,y1,A3,b118),(r3,y1,A3,b119),(r3,y1,A3,b120),(r3,y1,A3,b121),(r3,y1,A3,b122),(r3,y1,A3,b123),( r3,y1,A3,b124),(r3,y1,A3,b125),(r3,y1,A3,b126),(r3,y1,A3,b127),(r3,y1,A3,b128),(r3,y1,A3,b129),( r3,y1,A3,b130),(r3,y1,A3,b131),(r3,y1,A3,b132),(r3,y1,A3,b133),(r3,y1,A3,b134),(r3,y1,A4,b1),(r3, y1,A4,b2),(r3,y1,A4,b3),(r3,y1,A4,b4),(r3,y1,A4,b5), (r3,y1,A4,b6),(r3,y1,A4,b7),(r3,y1,A4,b8),(r3,y 1,A4,b9),(r3,y1,A4,b10),(r3,y1,A4,b11),(r3,y1,A4,b12),(r3,y1,A4,b13),(r3,y1,A4,b14),(r3,y1,A4,b15 ),(r3,y1,A4,b16),(r3,y1,A4,b17),(r3,y1,A4,b18),(r3,y1,A4,b19),(r3,y1,A4,b20),(r3,y1,A4,b21),(r3,y1, A4,b22),(r3,y1,A4,b23),(r3,y1,A4,b24),(r3,y1,A4,b25),(r3,y1,A4,b26),(r3,y ,A4,b27),(r3,y1,A4,b28) ,(r3,y1,A4,b29),(r3,y1,A4,b30),(r3,y1,A4,b31),(r3,y1,A4,b32),(r3,y1,A4,b33),(r3,y1,A4,b34),(r3,y1, A4,b35),(r3,y1,A4,b36),(r3,y1,A4,b37),(r3,y1,A4,b38),(r3,y1,A4,b39),(r3,y1,A4,b40),(r3,y1,A4,b41) ,(r3,y1,A4,b42),(r3,y1,A4,b43),(r3,y1,A4,b44),(r3,y1,A4,b45),(r3,y1,A4,b46),(r3,y1,A4,b47),(r3,y1, A4,b48),(r3,y1,A4,b49),(r3,y1,A4,b50),(r3,y1,A4,b51),(r3,y1,A4,b52),(r3,y1,A4,b53),(r3,y1,A4,b54) ,(r3,y1,A4,b55),(r3,y1,A4,b56),(r3,y1,A4,b57),(r3,y1,A4,b58),(r3,y1,A4,b59),(r3,y1,A4,b60),(r3,y1, A4,b61),(r3,y1,A4,b62),(r3,y1,A4,b63),(r3,y1,A4,b64),(r3,y1,A4,b65),(r3,y1,A4,b66),(r3,y1,A4,b67) ,(r3,y1,A4,b68),(r3,y1,A4,b69),(r3,y1,A4,b70),(r3,y1,A4,b71),(r3,y1,A4,b72),(r3,y1,A4,b73),(r3,yl, A4,b74),(r3,y1,A4,b75),(r3,y1,A4,b76),(r3,y1,A4,b77),(r3,y1,A4,b78),(r3,y1,A4,b79),(r3,y1,A4,b80) ,(r3,y1,A4,b81),(r3,y1,A4,b82),(r3,y1,A4,b83),(r3,y1,A4,b84),(r3,y1,A4,b85),(r3,y1,A4,b86),(r3,y1, A4,b87),(r3,y1,A4,b88),(r3,y1,A4,b89),(r3,y1,A4,b90),(r3,y1,A4,b91),(r3,y1,A4,b92),(r3,y1,A4,b93) ,(r3,y1,A4,b94),(r3,y1,A4,b95),(r3,y1,A4,b96),(r3,y1,A4,b97),(r3,y1,A4,b98),(r3,y1,A4,b99),(r3,y1, A4,b100),(r3,y1,A4,b101),(r3,y1,A4,b102),(r3,y1,A4,b103),(r3,y1,A4,b104),(r3,y1,A4,b105),(r3,y1, A4,b106),(r3,y1,A4,b107),(r3,y1,A4,b108),(r3,y1,A4,b109),(r3,y1,A4,b110),(r3,y1,A4,b111),(r3,y1, A4,b112),(r3,y1,A4,b113),(r3,y1,A4,b114),(r3,y1,A4,b115),(r3,y1,A4,b116),(r3,y1,A4,b117).(r3,yl, A4,b118),(r3,y1,A4,b119),(r3,y1,A4,b120),(r3,y1,A4,b121),(r3,y1,A4,b122),(r3,y1,A4,b123),(r3,y1, A4,b124),(r3,y1,A4,b125),(r3,y1,A4,b126),(r3,y1,A4,b127),(r3,y1,A4,b128),(r3,y1,A4,b129),(r3,y1, A4,b130),(r3,y1,A4,b131),(r3,y1,A4,b132),(r3,y1,A4,b133),(r3,y1,A4,b134),

(r3,y2,A1,b1),(r3,y2,A1,b2),(r3,y2,A1,b3),(r3,y2,A1,b4),(r3,y2,A1,b5),(r3,y2,A1,b6),(r3,y2,A1,b7),( r3,y2,A1,b8),(r3,y2,A1,b9),(r3,y2,A1,b10),(r3,y2,A1,b11),(r3,y2,A1,b12),(r3,y2,A1,b13),(r3,y2,A1,b 14),(r3,y2,A1,b15),(r3,y2,A1,b16),(r3,y2,A1,b17),(r3,y2,A1,b18),(r3,y2,A1,b19),(r3,y2,A1,b20),(r3, y2,A1,b21),(r3,y2,A1,b22),(r3,y2,A1,b23),(r3,y2,A1,b24),(r3,y2,A1,b25),(r3,y2,A1,b26),(r3,y2,A1,b 27),(r3,y2,A1,b28),(r3,y2,A1,b29),(r3,y2,A1,b30),(r3,y2,A1,b31),(r3,y2,A1,b32),(r3,y2,A1,b33),(r3, y2,A1,b34),(r3,y2,A1,b35),(r3,y2,A1,b36),(r3,y2,A1,b37),(r3,y2,A1,b38),(r3,y2,A1,b39),(r3,y2,A1,b 40),(r3,y2,A1,b41),(r3,y2,A1,b42),(r3,y2,A1,b43),(r3,y2,A1,b44),(r3,y2,A1,b45),(r3,y2,A1,b46),(r3, y2,A1,b47),(r3,y2,A1,b48),(r3,y2,A1,b49),(r3,y2,A1,b50),(r3,y2,A1,b51),(r3,y2,A1,b52),(r3,y2.A1,b 53),(r3,y2,A1,b54),(r3,y2,A1,b55),(r3,y2,A1,b56),(r3,y2,A1,b57),(r3,y2,A1,b58),(r3,y2,A1,b59),(r3, y2,A1,b60),(r3,y2,A1,b61),(r3,y2,A1,b62),(r3,y2,A1,b63),(r3,y2,A1,b64),(r3,y2,A1,b65),(r3,y2,A1,b 66),(r3,y2,A1,b67),(r3,y2,A1,b68),(r3,y2,A1,b69),(r3,y2,A1,b70),(r3,y2,A1,b71),(r3,y2,A1,b72),(r3, y2,A1,b73),(r3,y2,A1,b74),(r3,y2,A1,b75),(r3,y2,A1,b76),(r3,y2,A1,b77),(r3,y2,A1,b78),(r3,y2,A1,b 79),(r3,y2,A1,b80),(r3,y2,A1,b81),(r3,y2,A1,b82),(r3,y2,A1,b83),(r3,y2,A1,b84),(r3,y2,A1,b85),(r3, y2,A1,b86),(r3,y2,A1,b87),(r3,y2,A1,b88),(r3,y2,A1,b89),(r3,y2,A1,b90),(r3,y2,A1,b91),(r3,y2,A1,b 92),(r3,y2,A1,b93),(r3,y2,A1,b94),(r3,y2,A1,b95),(r3,y2,A1,b96),(r3,y2,A1,b97),(r3,y2,A1,b98),(r3, y2,A1,b99),(r3,y2,A1,b100),(r3,y2,A1,b101),(r3,y2,A1,b102),(r3,y2,A1,b103),(r3,y2,A1,b104),(r3,y 2,A1,b105),(r3,y2,A1,b106),(r3,y2,A1,b107),(r3,y2,A1,b108),(r3,y2,A1,b109),(r3,y2,A1,b110),(r3,y 2,A1,b111),(r3,y2,A1,b112),(r3,y2,A1,b113),(r3,y2,A1,b114),(r3,y2,A1,b115),(r3,y2,A1,b116),(r3,y 2,A1,b117),(r3,y2,A1,b118),(r3,y2,A1,b119),(r3,y2,A1,b120),(r3,y2,A1,b121),(r3,y2,A1,b122),(r3,y 2,A1,b123),(r3,y2,A1,b124),(r3,y2,A1,b125),(r3,y2,A1,b126),(r3,y2,A1,b127),(r3,y2,A1,b128),(r3,y 2,A1,b129),(r3,y2,A1,b130),(r3,y2,A1,b 131),(r3,y2,A1,b132),(r3,y2,A1,b133),(r3,y2,A1,b134),(r3,y 2,A2,b1),(r3,y2,A2,b2),(r3,y2,A2,b3),(r3,y2,A2,b4),(r3,y2,A2,b5),(r3,y2,A2,b6),(r3,y2,A2,b7),(r3,y2 ,A2,b8),(r3,y2,A2,b9),(r3,y2,A2,b10),(r3,y2,A2,b 11),(r3,y2,A2,b 12),(r3,y2,A2,b 13),(r3,y2,A2,b 14),( r3,y2,A2,b15),(r3,y2,A2,b16),(r3,y2,A2,b17),(r3,y2,A2,b18),(r3,y2,A2,b19),(r3,y2,A2,b20),(r3,y2,A 2,b21),(r3,y2,A2,b22),(r3,y2,A2,b23),(r3,y2,A2,b24),(r3,y2,A2,b25),(r3,y2,A2,b26),(r3,y2,A2,b27),( r3,y2,A2,b28),(r3,y2,A2,b29),(r3,y2,A2,b30),(r3,y2,A2,b31),(r3,y2,A2,b32),(r3,y2,A2,b33),(r3,y2,A 2,b34),(r3,y2,A2,b35),(r3,y2,A2,b36),(r3,y2,A2,b37),(r3,y2,A2,b38),(r3,y2,A2,b39),(r3,y2,A2,b40),( r3,y2,A2,b41),(r3,y2,A2,b42),(r3,y2,A2,b43),(r3,y2,A2,b44),(r3,y2,A2,b45),(r3,y2,A2,b46),(r3,y2,A 2,b47),(r3,y2,A2,b48),(r3,y2,A2,b49),(r3,y2,A2,b50),(r3,y2,A2,b51),(r3,y2,A2,b52),(r3,y2,A2,b53),( r3,y2,A2,b54),(r3,y2,A2,b55),(r3,y2,A2,b56),(r3,y2,A2,b57),(r3,y2,A2,b58),(r3,y2,A2,b59),(r3,y2,A 2,b60),(r3,y2,A2,b61),(r3,y2,A2,b62),(r3,y2,A2,b63),(r3,y2,A2,b64),(r3,y2,A2,b65),(r3,y2,A2,b66),( r3,y2,A2,b67),(r3,y2,A2,b68),(r3,y2,A2,b69),(r3,y2,A2,b70),(r3,y2,A2,b71),(r3,y2,A2,b72),(r3,y2,A 2,b73),(r3,y2,A2,b74),(r3,y2,A2,b75),(r3,y2,A2,b76),(r3,y2,A2,b77),(r3,y2,A2,b78),(r3,y2,A2,b79),( r3,y2,A2,b80),(r3,y2,A2,b81),(r3,y2,A2,b82),(r3,y2,A2,b83),(r3,y2,A2,b84),(r3,y2,A2,b85),(r3,y2,A 2,b86),(r3,y2,A2,b87),(r3,y2,A2,b88),(r3,y2,A2,b89),(r3,y2,A2,b90),(r3,y2,A2,b91),(r3,y2,A2,b92),( r3,y2,A2,b93),(r3,y2,A2,b94),(r3,y2,A2,b95),(r3,y2,A2,b96),(r3,y2,A2,b97),(r3,y2,A2,b98),(r3,y2,A 2,b99),(r3,y2,A2,b 100),(r3,y2,A2,b 101),(r3,y2,A2,b102),(r3,y2,A2,b 103),(r3,y2,A2,b104),(r3,y2,A2, b 105),(r3,y2,A2,b106),(r3,y2,A2,b 107),(r3,y2,A2,b108),(r3,y2,A2,b109),(r3,y2,A2,b 110),(r3,y2,A2, b 111),(r3,y2,A2,b112),(r3,y2,A2,b113),(r3,y2,A2,b114),(r3,y2,A2,b115),(r3,y2,A2,b116),(r3,y2,A2, b117),(r3,y2,A2,b118),(r3,y2,A2,b119),(r3,y2,A2,b120),(r3,y2,A2,b121),(r3,y2,A2,b122),(r3,y2,A2, b123),(r3,y2,A2,b124),(r3,y2,A2,b125),(r3,y2,A2,b126),(r3,y2,A2,b127),(r3,y2,A2,b128),(r3,y2,A2, b129),(r3,y2,A2,b130),(r3,y2,A2,b131),(r3,y2,A2,b132),(r3,y2,A2,b133),(r3,y2,A2,b134),(r3,y2,A3, b1),(r3,y2,A3,b2),(r3,y2,A3,b3),(r3,y2,A3,b4),(r3,y2,A3,b5),(r3,y2,A3,b6),(r3,y2,A3,b7),(r3,y2,A3,b 8),(r3,y2,A3,b9),(r3,y2,A3,b10),(r3,y2,A3,b11),(r3,y2,A3,b12),(r3,y2,A3,b13),(r3,y2,A3,b14),(r3,y2, A3,b15),(r3,y2,A3,b16),(r3,y2,A3,b17),(r3,y2,A3,b18),(r3,y2,A3,b19),(r3,y2,A3,b20),(r3,y2,A3,b21) ,(r3,y2,A3,b22),(r3,y2,A3,b23),(r3,y2,A3,b24),(r3,y2,A3,b25),(r3,y2,A3,b26),(r3,y2,A3,b27),(r3,y2, A3,b28),(r3,y2,A3,b29),(r3,y2,A3,b30),(r3,y2,A3,b31),(r3,y2,A3,b32),(r3,y2,A3,b33),(r3,y2,A3,b34) ,(r3,y2,A3,b35),(r3,y2,A3,b36),(r3,y2,A3,b37),(r3,y2,A3,b38),(r3,y2,A3,b39),(r3,y2,A3,b40),(r3,y2, A3,b41),(r3,y2,A3,b42),(r3,y2,A3,b43),(r3,y2,A3,b44),(r3,y2,A3,b45),(r3,y2,A3,b46),(r3,y2,A3,b47) ,(r3,y2,A3,b48),(r3,y2,A3,b49),(r3,y2,A3,b50),(r3,y2,A3,b5l),(r3,y2,A3,b52),(r3,y2,A3,b53),(r3,y2, A3,b54),(r3,y2,A3,b55),(r3,y2,A3,b56),(r3,y2,A3,b57),(r3,y2,A3,b58),(r3,y2,A3,b59),(r3,y2,A3,b60) ,(r3,y2,A3,b61),(r3,y2,A3,b62),(r3,y2,A3,b63),(r3,y2,A3,b64),(r3,y2,A3,b65),(r3,y2,A3,b66),(r3,y2, A3,b67),(r3,y2,A3,b68),(r3,y2,A3,b69),(r3,y2,A3,b70).(r3,y2,A3,b71),(r3,y2,A3,b72),(r3,y2,A3,b73) ,(r3,y2,A3,b74),(r3,y2,A3,b75),(r3,y2,A3,b76),(r3,y2,A3,b77),(r3,y2,A3,b78),(r3,y2,A3,b79),(r3,y2, A3,b80),(r3,y2,A3,b81),(r3,y2,A3,b82),(r3,y2,A3,b83),(r3,y2,A3,b84),(r3,y2,A3,b85),(r3,y2,A3,b86) ,(r3,y2,A3,b87),(r3,y2,A3,b88),(r3,y2,A3,b89),(r3,y2,A3,b90),(r3,y2,A3,b91),(r3,y2,A3,b92),(r3,y2, A3,b93),(r3,y2,A3,b94),(r3,y2,A3,b95),(r3,y2,A3,b96),(r3,y2,A3,b97),(r3,y2,A3,b98),(r3,y2,A3,b99) ,(r3,y2,A3,b100),(r3,y2,A3,b 101),(r3,y2,A3,b102),(r3,y2,A3,b103),(r3,y2,A3,b104),(r3,y2,A3,b105), (r3,y2,A3,b 106),(r3,y2,A3,b107),(r3,y2,A3,b 108),(r3,y2,A3,b109),(r3,y2,A3,b110),(r3,y2,A3,b 111),( r3,y2,A3,b112),(r3,y2,A3,b113),(r3,y2,A3,b114),(r3,y2,A3,b115),(r3,y2,A3,b116),(r3,y2,A3,b117),( r3,y2,A3,b 118),(r3,y2,A3,b119),(r3,y2,A3,b 120),(r3,y2,A3,b121),(r3,y2,A3,b 122),(r3,y2,A3,b 123),( r3,y2,A3,b124),(r3,y2,A3,b125),(r3,y2,A3,b126),(r3,y2,A3,b127),(r3,y2,A3,b128),(r3,y2,A3,b129),( r3,y2,A3,b130),(r3,y2,A3,b131),(r3,y2,A3,b 132),(r3,y2,A3,b133),(r3,y2,A3,b134),(r3,y2,A4,b1),(r3, y2,A4,b2),(r3,y2,A4,b3),(r3,y2,A4,b4),(r3,y2,A4,b5),(r3,y2,A4,b6),(r3,y2,A4,b7),(r3,y2,A4,b8),(r3,y 2,A4,b9),(r3,y2,A4,b10),(r3,y2,A4,b 11),(r3,y2,A4,b 12),(r3,y2,A4,b 13),(r3,y2,A4,b14),(r3,y2,A4,b15 ),(r3,y2,A4,b 16),(r3,y2,A4,b17),(r3,y2,A4,b18),(r3,y2,A4,b19),(r3,y2,A4,b20),(r3,y2,A4,b21),(r3,y2, A4,b22),(r3,y2,A4,b23),(r3,y2,A4,b24),(r3,y2,A4,b25),(r3,y2,A4,b26),(r3,y2,A4,b27),(r3,y2,A4,b28) ,(r3,y2,A4,b29),(r3,y2,A4,b30),(r3,y2,A4,b31),(r3,y2,A4,b32),(r3,y2,A4,b33),(r3,y2,A4,b34),(r3,y2, A4,b35),(r3,y2,A4,b36),(r3,y2,A4,b37),(r3,y2,A4,b38),(r3,y2,A4,b39),(r3,y2,A4,b40),(r3,y2,A4,b41) ,(r3,y2,A4,b42),(r3,y2,A4,b43),(r3,y2,A4,b44),(r3,y2,A4,b45),(r3,y2,A4,b46),(r3,y2,A4,b47),(r3,y2, A4,b48),(r3,y2,A4,b49),(r3,y2,A4,b50),(r3,y2,A4,b51),(r3,y2,A4,b52),(r3,y2,A4,b53),(r3,y2,A4,b54) ,(r3,y2,A4,b55),(r3,y2,A4,b56),(r3,y2,A4,b57),(r3,y2,A4,b58),(r3,y2,A4,b59),(r3,y2,A4,b60),(r3,y2, A4,b61),(r3,y2,A4,b62),(r3,y2,A4,b63),(r3,y2,A4,b64),(r3,y2,A4,b65),(r3,y2,A4,b66),(r3,y2,A4,b67) ,(r3,y2,A4,b68),(r3,y2,A4,b69),(r3,y2,A4,b70),(r3,y2,A4,b71),(r3,y2,A4,b72),(r3,y2,A4,b73),(r3,y2, A4,b74),(r3,y2,A4,b75),(r3,y2,A4,b76),(r3,y2,A4,b77),(r3,y2,A4,b78),(r3,y2,A4,b79),(r3,y2,A4,b80) ,(r3,y2,A4,b81),(r3,y2,A4,b82),(r3,y2,A4,b83),(r3,y2,A4,b84),(r3,y2,A4,b85),(r3,y2,A4,b86),(r3,y2, A4,b87),(r3,y2,A4,b88),(r3,y2,A4,b89),(r3,y2,A4,b90),(r3,y2,A4,b91),(r3,y2,A4,b92),(r3,y2,A4,b93) ,(r3,y2,A4,b94),(r3,y2,A4,b95),(r3,y2,A4,b96),(r3,y2,A4,b97),(r3,y2,A4,b98),(r3,y2,A4,b99),(r3,y2, A4,b100),(r3,y2,A4,b101),(r3,y2,A4,b102),(r3,y2,A4,b103),(r3,y2,A4,b104),(r3,y2,A4,b105),(r3,y2, A4,b106),(r3,y2,A4,b107),(r3,y2,A4,b108),(r3,y2,A4,b109),(r3,y2,A4,b110),(r3,y2,A4,b111),(r3,y2, A4,b 112),(r3,y2,A4,b113),(r3,y2.A4,b114),(r3,y2,A4,b115),(r3,y2,A4,b116),(r3,y2,A4,b117),(r3,y2, A4,b118),(r3,y2,A4,b119),(r3,y2,A4,b120),(r3,y2,A4,b121),(r3,y2,A4,b122),(r3,y2,A4,b123),(r3,y2, A4,b124),(r3,y2,A4,b125),(r3,y2,A4,b126),(r3,y2,A4,b127),(r3,y2,A4,b128),(r3,y2,A4,b129),(r3,y2, A4,b130),(r3,y2,A4,b131),(r3,y2,A4,b132),(r3,y2,A4,b133),(r3,y2,A4,b134),

(r4,y1,A1,b1),(r4,y1,A1,b2),(r4,y1,A1,b3),(r4,y1,A1,b4),(r4,y1,A1,b5),(r4,y1,A1,b6),(r4,y1,A1,b7),( r4,y1,A1,b8),(r4,y1,A1,b9),(r4,y1*,*A1,b10),(r4,y1,A1,b11),(r4,y1,A1,b12),(r4,y1,A1,b13),(r4,y1,A1,b 14),(r4,y1,A1,b15),(r4,y1,A1,b16),(r4,y1,A1,b17),(r4,y1,A1,b18),(r4,y1,A1,b19),(r4,y1,A1,b20),(r4, y1,A1,b21),(r4,y1,A1,b22),(r4,y1,A1,b23),(r4,y1,A1,b24),(r4,y1,A1,b25),(r4,y1,A1,b26),(r4,y1,A1,b 27),(r4,y1,A1,b28),(r4,y1,A1,b29),(r4,y1,A1,b30),(r4,y1,A1,b31),(r4,y1,A1,b32),(r4,y1,A1,b33),(r4, y1,A1,b34),(r4,y1,A1,b35),(r4,y1,A1,b36),(r4,y1,A1,b37),(r4,y1,A1,b3S),(r4,y1,A1,b39),(r4,y1,A1,b 40),(r4,y1,Al,b41),(r4,y1,A1,b42),(r4,y1,A1,b43),(r4,y1,A1,b44),(r4,y1,A1,b45),(r4,y1,A1,b46),(r4, y1,A1,b47),(r4,y1,A1,b48),(r4,y1,A1,b49),(r4,y1,A1,b50),(r4,y1,A1,b51),(r4,y1,A1,b52),(r4,y1,A1,b 53),(r4,y1,A1,b54),(r4,y1,A1,b55),(r4,y1,A1,b56),(r4,y1,A1,b57),(r4,y1,A1,b58),(r4,y1,A1,b59),(r4, y1,A1,b60),(r4,y1,A1,b61),(r4,y1,A1,b62),(r4,y1,A1,b63),(r4,y1,A1,b64),(r4,y1,A1,b65),(r4,y1,A1,b 66),(r4,y1,A1,b67),(r4,y1,A1,b68),(r4,y1,A1,b69),(r4,y1,A1,b70),(r4,y1,A1,b71),(r4,y1,A1,b72),(r4, y1,A1,b73),(r4,y1,A1,b74),(r4,y1,A1,b75),(r4,y1,A1,b76),(r4,y1,A1,b77),(r4,y1,A1,b78),(r4,y1,A1,b 79),(r4,y1,A1,b80),(r4,y1,A1,b81),(r4,y1,A1,b82),(r4,y1,A1,b83),(r4,y1,A1,b84),(r4,y1,A1,b85),(r4, y1,A1,b86),(r4,y1,A1,b87),(r4,y1,A1,b88),(r4,y1,A1,b89),(r4,y1,A1,b90),(r4,y1,A1,b91),(r4,y1,A1,b 92),(r4,y1,A1,b93),(r4,y1,A1,b94),(r4,y1,A1,b95),(r4,y1,A1,b96),(r4,y1,A1,b97),(r4,y1,A1,b98),(r4, y1,A1,b99),(r4,y1,A1,b100),(r4,y1,A1,b101),(r4,y1,A1,b102),(r4,y1,A1,b103),(r4,y1,A1,b104),(r4,y 1,A1,b105),(r4,y1,A1,b106),(r4,y1,A1,b107),(r4,y1,A1,b108),(r4,y1,A1,b109),(r4,y1,A1,b110),(r4,y 1,A1,b111),(r4,y1,A1,b112),(r4,y1,A1,b113),(r4,y1,A1,b114),(r4,y1,A1,b115),(r4,y1,A1,b116),(r4,y 1,A1,b117),(r4,y1,A1,b118),(r4,y1,A1,b119),(r4,y1,A1,b120),(r4,y1,A1,b121),(r4,y1,A1,b122),(r4,y 1,A1,b123),(r4,y1,A1,b124),(r4,y1,A1,b125),(r4,y1,A1,b126),(r4,y1,A1,b127),(r4,y1,A1,b128),(r4,y 1,A1,b129),(r4,y1,A1,b130),(r4,y1,A1,b131),(r4,y1,A1,b132),(r4,y1,A1,b133),(r4,y1,A1,b134),(r4,y 1,A2,b1),(r4,y1,A2,b2),(r4,y1,A2,b3),(r4,y1,A2,b4),(r4,y1,A2,b5),(r4,y1,A2,b6),(r4,y1,A2,b7),(r4,y1 ,A2,b8),(r4,y1,A2,b9),(r4,y1,A2,b10),(r4,y1,A2,b11),(r4,y1,A2,b12),(r4,y1,A2,b13),(r4,y1,A2,b14),( r4,y1,A2,b15),(r4,y1,A2,b16),(r4,y1,A2,1a17),(r4,y1,A2,b18),(r4,y1,A2,b19),(r4,y1,A2,b20),(r4,y1,A 2,b21),(r4,y1,A2,b22),(r4,y1,A2,b23),(r4,y1,A2,b24),(r4,y1,A2,b25),(r4,y1,A2,b26),(r4,y1,A2,b27),( r4,y1,A2,b28),(r4,y1,A2,b29),(r4,y1,A2,b30),(r4,y1,A2,b31),(r4,y1,A2,b32),(r4,y1,A2,b33),(r4,y1,A 2,b34),(r4,y1,A2,b35),(r4,y1,A2,b36),(r4,y1,A2,b37),(r4,y1,A2,b38),(r4,y1,A2,b39),(r4,y1,A2,b40),( r4,y1,A2,b41),(r4,y1,A2,b42),(r4,y1,A2,b43),(r4,y1,A2,b44),(r4,y1,A2,b45),(r4,y1,A2,b46),(r4,y1,A 2,b47),(r4,y1,A2,b48),(r4,y1,A2,b49),(r4,y1,A2,b50),(r4,y1,A2,b51),(r4,y1,A2,b52),(r4,y1,A2,b53),( r4,y1,A2,b54),(r4,y1,A2,b55),(r4,y1,A2,b56),(r4,y1,A2,b57),(r4,y1,A2,b58),(r4,y1,A2,b59),(r4,y1,A 2,b60),(r4,y1,A2,b61),(r4,y1,A2,b62),(r4,y1,A2,b63),(r4,y1,A2,b64),(r4,y1,A2,b65),(r4,y1,A2,b66),( r4,y1,A2,b67),(r4,y1,A2,b68),(r4,y1,A2,b69),(r4,y1,A2,b70),(r4,y1,A2,b71),(r4,y1,A2,b72),(r4,y1,A 2,b73),(r4,y1,A2,b74),(r4,y1,A2,b75),(r4,y1,A2,b76),(r4,y1,A2,b77),(r4,y1,A2,b78),(r4,y1,A2,b79),( r4,y1,A2,b80),(r4,y1,A2,b81),(r4,y1,A2,b82),(r4,y1,A2,b83),(r4,y1,A2,b84),(r4,y1,A2,b85).(r4,y1_{;}A 2,b86),(r4,y1,A2,b87),(r4,y1,A2,b88),(r4,y1,A2,b89),(r4,y1,A2,b90),(r4,y1,A2,b91),(r4,y1,A2,b92),( r4,y1,A2,b93),(r4,y1,A2,b94),(r4,y1,A2,b95),(r4,y1,A2,b96),(r4,y1,A2,b97),(r4,y1,A2,b98),(r4,y,A 2,b99),(r4,y1,A2,b100),(r4,y1,A2,b101),(r4,y1,A2,b102),(r4,y1,A2,b103),(r4,y1,A2,b104),(r4,y1,A2, b105),(r4,y1,A2,b106),(r4,y1,A2,b107),(r4,y1,A2,b108),(r4,y1,A2,b109),(r4,y1,A2,b110),(r4,y1,A2, b111),(r4,y1,A2,b112),(r4,y1,A2,b113),(r4,y1,A2,b114),(r4,y1,A2,b115),(r4,y1,A2,b116),(r4,y1,A2, b117),(r4,y1,A2,b118),(r4,yl,A2,b119),(r4,y1,A2,b120),(r4,y1,A2,b121),(r4,yi,A2,b122),(r4,y1,A2, b123),(r4,y1,A2,b124),(r4,y1,A2,b125),(r4,y1,A2,b126),(r4,y1,A2,b127),(r4,y1,A2,b128),(r4,y1,A2, b129),(r4,y1,A2,b130),(r4,y1,A2,b131),(r4,y1,A2,b132),(r4,y1,A2,b133),(r4,y1,A2,b134),(r4,y1,A3, b1),(r4,y1,A3,b2),(r4,y1,A3,b3),(r4,y1,A3,b4),(r4,y1,A3,b5),(r4,y1,A3,b6),(r4,y1,A3,b7),(r4,y1,A3,b 8),(r4,y1.,A3,b9),(r4,y1,A3,b10),(r4,y1,A3,b11),(r4,y1,A3,b12),(r4,y1,A3,b13),(r4,y1,A3,b14),(r4,y1, A3,b15),(r4,y1,A3,b16),(r4,y1,A3,b17),(r4,y1,A3,b18),(r4,y1,A3,b19),(r4,y1,A3,b20),(r4,y1,A3,b21) ,(r4,y1,A3,b22),(r4,y1,A3,b23),(r4,y1,A3,b24),(r4,y1,A3,b25),(r4,y1,A3,b26),(r4,y1,A3,b27),(r4,y1, A3,b28),(r4,y1,A3,b29),(r4,y1,A3,b30),(r4,y1,A3,b31),(r4,y1,A3,b32),(r4,y1,A3,b33),(r4,y1,A3,b34) ,(r4,y1,A3,b35),(r4,y1,A3,b36),(r4,y1,A3,b37),(r4,y1,A3,b38),(r4,y1,A3,b39),(r4,y1,A3,b40),(r4,y1, A3,b41),(r4,y1,A3,b42),(r4,y1,A3,b43),(r4,y1,A3,b44),(r4,y1,A3,b45),(r4,y1,A3,b46),(r4,y1,3,b47) ,(r4,y1,A3,b48),(r4,y1,A3,b49),(r4,y1,A3,b50),(r4,y1,A3,b51),(r4,y1,A3,b52),(r4,y1,A3,b53),(r4,y1, A3,b54),(r4,y1,A3,b55),(r4,y1,A3,b56),(r4,y1,A3,b57),(r4,y1,A3,b58),(r4,y1,A3,b59),(r4,y1,A3,b60) ,(r4,y1,A3,b61),(r4,y1,A3,b62),(r4,y1,A3,b63),(r4,y1,A3,b64),(r4,y1,A3,b65),(r4,y1,A3,b66),(r4,y1, A3,b67),(r4,y1,A3,b68),(r4,y1,A3,b69),(r4,y1,A3,b70),(r4,y1,A3,b71),(r4,y1,A3,b72),(r4,y1,A3,b73) ,(r4,y1,A3,b74),(r4,y1,A3,b75),(r4,y1,A3,b76),(r4,y1,A3,b77),(r4,y1,A3,b78),(r4,y1,A3,b79),(r4,y1, A3,b80),(r4,y1,A3,b81),(r4,y1,A3,b82),(r4,y1,A3,b83),(r4,y1,A3,b84),(r4,y1,A3,b85),(r4,y1,A3,b86) ,(r4,y1,A3,b87),(r4,y1,A3,b88),(r4,y1,A3,b89),(r4,y1,A3,b90),(r4,y1,A3,b91),(r4,y1,A3,b92),(r4,y1, A3,b93),(r4,y1,A3,b94),(r4,y1,A3,b95),(r4,y1,A3,b96),(r4,y1,A3,b97),(r4,y1,A3,b98),(r4,y1,A3,b99) ,(r4,y1,A3,b100),(r4,y1,A3,b101),(r4,y1,A3,b102),(r4,y1,A3,b103),(r4,y1,A3,b104),(r4,y1,A3,b105), (r4,y1,A3,b106),(r4,y1,A3,b107),(r4,y1,A3,bl08),(r4,y1,A3,b109),(r4,y1,A3,b110),(r4,y1,A3,b111),( r4,y1,A3,b112),(r4,y1,A3,b113),(r4,y1,A3,b114),(r4,y1,A3,b115),(r4,y1,A3,b116),(r4,y1,A3,b117),( r4,y1.,A3,b118),(r4,y1,A3,b119),(r4,y1,A3,b120),(r4,y1,A3,b121),(r4,y1,A3,b122),(r4,y1,A3,b123),( r4,y1,A3,b124),(r4,y1,A3,b125),(r4,y1,A3,b126),(r4,y1,A3,b127),(r4,y1,A3,b128),(r4,y1,A3,b129),( r4,y1,A3,b130),(r4,y1,A3,b131),(r4,y1,A3,b132),(r4,y1,A3,b133),(r4,y1,A3,b134),(r4,y1,A4,b1),(r4, y1,A4,b2),(r4,y1,A4,b3)>(r4,y1,A4,b4),(r4,y1,A4,b5),(r4,y1,A4,b6),(r4,y1,A4,b7),(r4,y1,A4,b8),(r4,y 1,A4,b9),(r4,y1,A4,b10),(r4,y1,A4,b11),(r4,y1,A4,b12),(r4,y1,A4,b13),(r4,y1,A4,b14),(r4,y1,A4,b15 ),(r4,y1,A4,b16),(r4,y1,A4,b17),(r4,y1,A4,b18),(r4,y1,A4,b19),(r4,y1,A4,b20),(r4,y1,A4,b21),(r4,y1, A4,b22),(r4,y1,A4,b23),(r4,y1,A4,b24),(r4,y1,A4,b25),(r4,y1,A4,b26),(r4,y1,A4,b27),(r4,y1,A4,b28) ,(r4,y1,A4,b29),(r4,y1,A4,b30),(r4,y1,A4,b31),(r4,y1,A4,b32),(r4,y1,A4,b33),(r4,y1,A4,b34)_{;}(r4,y1, A4,b35),(r4,y1,A4,b36),(r4,y1,A4,b37),(r4,y1,A4.b38),(r4,y1,A4,b39),(r4,y1,A4,b40),(r4,y1,A4,b41) ,(r4,y1,A4,b42),(r4,y1,A4,b43),(r4,y1,A4,b44),(r4,y1,A4,b45),(r4,y1,A4,b46),(r4,y1,A4,b47),(r4,y1, A4,b48),(r4,y1,A4,b49),(r4,y1,A4,b50),(r4,y1,A4,b51),(.r4,y1,A4,b52),(r4,y1,A4,b53),(r4,y1,A4,b54) ,(r4,y1,A4,b55),(r4,y1,A4,b56),(r4,y1,A4,b57),(r4,y1,A4,b58),(r4,y1,A4,b59),(r4,y1,A4,b60),(r4,y1, A4,b61),(r4,y1,A4,b62),(r4,y1,A4,b63),(r4,y1,A4,b64),(r4,y1,A4,b65),(r4,y1,A4,b66),(r4,y1,A4,b67) ,(r4,y1,A4,b68),(r4,y1,A4,b69),(r4,y1,A4,b70),(r4,y1,A4,b71),(r4,y1,A4,b72),(r4,y1,A4,b73),(r4,y1, A4,b74),(r4,y1,A4,b75),(r4,y1,A4,b76),(r4,y1,A4,b77),(r4,y1,A4,b78),(r4,y1,A4,b79),(r4,y1,A4,b80) ,(r4,y1,A4,b81.),(r4,y1,A4,b82),(r4,y1,A4,b83),(r4,y1,A4,b84),(r4,y1,A4,b85),(r4,y1,A4,b86),(r4,y1, A4,b87),(r4,y1,A4,b88),(r4,y1,A4,b89),(r4,y1,A4,b90),(r4,y1,A4,b91),(r4,y1,A4,b92),(r4,y1,A4,b93) ,(r4,y1,A4,b94),(r4,y1,A4,b95),(r4,y1,A4,b96),(r4,y1,A4,b97),(r4,y1,A4,b98),(r4,y1,A4,b99),(r4,y1, A4,b100),(r4,y1,A4,b101),(r4,y1,A4,b102),(r4,y1,A4,b103),(r4,y1,A4,b104),(r4,y1,A4,b105),(r4,y1, A4,b106),(r4,y1,A4_{;}b107),(r4,y1,A4,b108),(r4,y1,A4,b109),(r4,y1,A4,b110),(r4,y1,A4,b111),(r4,y1, A4,b112),(r4,y1,A4,b113),(r4,y1,A4,b114),(r4,y1,A4,b115),(r4,y1,A4,b116),(r4,y1,A4,b117),(r4,y1, A4,b118),(r4,y1,A4,b119),(r4,y1,A4,b120),(r4,y1,A4,b121),(r4,y1,A4,b122),(r4,y1,A4,b123),(r4,y1, A4,b124),(r4,y1,A4,b125),(r4,y1,A4,b126),(r4,y1,A4,b127),(r4,y1,A4,b128),(r4,y1,A4,b129),(r4,y1, A4,b130),(r4,y1,A4,b131),(r4,y1,A4,b132),(r4,y1,A4,b133),(r4,y1,A4,b134),

(r4,y2,A1,b1),(r4,y2,A1,b2),(r4,y2,A1,b3),(r4,y2,A1,b4),(r4,y2,A1,bS),(r4,y2,A1,b6),(r4,y2,A1,b7),( r4,y2,A1,bB),(r4,y2,A1,b9),fr4,y2.A1,b10),(r4,y2,A1,b11),(r4,y2,A1,b12),(r4,y2,A1,bl3),(r4,y2,A1,b 14),(r4,y2,A1,b15),(r4,y2,A1,b16),(r4,y2,A1,b17),(r4,y2,A1,b18),(r4,y2,A1,b19),(r4,y2,A1,b20),(r4, y2,A1,b21),(r4,y2,A1,b22),(r4,y2,A1,b23),(r4,y2,A1,b24),(r4,y2,A1,b25),(r4,y2,A1,b26),(r4,v2,A1,b 27),(r4,y2,A1,b28),(r4,y2,A1,b29),(r4,y2,A1,b30),(r4,y2,A1,b31),(r4,y2,A1,b32),(r4,y2,A1,b33),(r4, y2,A1,b34),(r4,y2,A1,b35),(r4,y2,A1,b36),(r4,y2,A1,b37),(r4,y2,A1,b38),(r4,y2,A1,b39),(r4,y2,A1,b 40),(r4,y2,A1,b41),(r4,y2,A1,b42),(r4,y2,A1,b43),(r4,y2,A1,b44),(r4,y2,A1,b45),(r4,y2,A1,b46),(r4, y2,A1,b47),(r4,y2,A1,b48),(r4,y2,A1,b49),(r4,y2,A1,b50),(r4,y2,A1,b51),(r4,y2,A1,b52),(r4,y2,A1,b 53),(r4,y2,A1,b54),(r4,y2,A1,b55),(r4,y2,A1,b56),(r4,y2,A1,b57),(r4,y2,A1,b58),(r4,y2,A1,b59),(r4, y2,A1,b60),(r4,y2,A1,b61),(r4,y2,A1,b62),(r4,y2,A1,b63),(r4,y2,A1,b64),(r4,y2,A1,b65),(r4,y2,A1,b 66),(r4,y2,A1,b67),(r4,y2,A1,b68),(r4,y2,A1,b69),(r4,y2,A1,b70),(r4,y2,A1,b71),(r4,y2,A1,b72),(r4, y2,A1,b73),(r4,y2,A1,b74),(r4,y2,A1,b75),(r4,y2,A1,b76),(r4,y2,A1,b77),(r4,y2,A1,b78),(r4,y2,A1,b 79),(r4,y2,A1,b80),(r4,y2,A1,b81),(r4,y2,A1,b82),(r4,y2,A1,b83),(r4,y2,A1,b84),(r4,y2,A1,b85),(r4, y2,A1,b86),(r4,y2,A1,b87),(r4,y2,A1,b88),(r4,y2,A1,b89),(r4,y2,A1,b90),(r4,y2,A1,b91),(r4,y2,A1,b 92),(r4,y2,A1,b93),(r4,y2,A1,b94),(r4,y2,A1,b95),(r4,y2,A1,b96),(r4,y2,A1,b97),(r4,y2,A1,b98),(r4, y2,A1,b99),(r4,y2,A1,b100),(r4,y2,A1,b101),(r4,y2,A1,b102),(r4,y2,A1,b103),(r4,y2,A1,b104),(r4,y 2,A1,b105),(r4,y2,A1,b106),(r4,y2,A1,b107),(r4,y2,A1,b108),(r4,y2,A1,b109),(r4,y2,A1.,b110),(r4,y 2,A1,b111),(r4,y2,A1,b112),(r4,y2,A1,b113),(r4,y2,A1,b114),(r4,y2,A1,b115),(r4,y2,A1,b116),(r4,y 2,A1,b117),(r4,y2,A1,b118),(r4,y2,A1,b119),(r4,y2,A1,b120),(r4,y2,A1,b121),(r4,y2,A1,b122),(r4,y 2,A1,b123),(r4,y2,A1,b124),(r4,y2,A1,b125),(r4,y2,A1,b126),(r4,y2,A1,b127),(r4,y2,A1,b128),(r4,y 2,A1,b129),(r4,y2,A1,b130),(r4,y2,A1,b131),(r4,y2,A1,b132),(r4,y2,A1,b133),(r4,y2,A1,b134),(r4,y 2,A2,b1),(r4,y2,A2,b2),(r4,y2,A2,b3),(r4,y2,A2,b4),(r4,y2,A2,b5),(r4,y2,A2,b6),(r4,y2,A2,b7),(r4,y2 ,A2,b8),(r4,y2,A2,b9),(r4,y2,A2,b10),(r4,y2,A2,b11),(r4,y2,A2,b12),(r4,y2,A2,b13),(r4,y2,A2,b14),( r4,y2,A2,b15),(r4,y2,A2,b16),(r4,y2,A2,b17),(r4,y2,A2,b18),(r4,y2,A2,b19),(r4,y2,A2,b20),(r4,y2,A 2,b21),(r4,y2,A2,b22),(r4,y2,A2,b23),(r4,y2,A2,b24),(r4,y2,A2,b25),(r4,y2,A2,b26),(r4,y2,A2,b27),( r4,y2,A2,b28),(r4,y2,A2,b29),(r4,y2,A2,b30),(r4,y2,A2,b31),(r4,y2,A2,b32),(r4,y2,A2,b33),(r4,y2,A 2,b34),(r4,y2,A2,b35),(r4,y2,A2,b36),(r4,y2,A2,b37),(r4,y2,A2,b38),(r4,y2,A2,b39),(r4,y2,A2,b40),( r4,y2,A2,b41),(r4,y2,A2,b42),(r4,y2,A2,b43),(r4,y2,A2,b44),(r4,y2,A2,b45),(r4,y2,A2,b46),(r4,y2,A 2,b47),(r4,y2,A2,b48),(r4,y2,A2,b49),(r4,y2,A2,b50),(r4,y2,A2,b51),(r4,y2,A2,b52),(r4,y2,A2,b53),( r4,y2,A2,b54),(r4,y2,A2,b55),(r4,y2,A2,b56),(r4,y2,A2,b57),(r4,y2,A2,b58),(r4,y2,A2,b59),(r4,y2,A 2,b60),(r4,y2,A2,b61),(r4,y2,A2,b62),(r4,y2,A2,b63),(r4,y2,A2,b64),(r4,y2,A2,b65),(r4,y2,A2,b66),( r4,y2,A2,b67),(r4,y2,A2,b68),(r4,y2,A2,b69),(r4,y2,A2,b70),(r4,y2,A2,b71),(r4,y2,A2,b72),(r4,y2,A 2,b73),(x4,y2,A2,b74),(r4,y2,A2.b75),(r4,y2,A2,b76),(r4,y2,A2,b77),(r4,y2,A2,b78),(r4,y2,A2,b79),( r4,y2,A2,b80),(r4,y2,A2,b81),(r4,y2,A2,b82),(r4,y2,A2,b83),(r4,y2,A2,b84),(r4,y2,A2,b85),(r4,y2,A 2,b86),(r4,y2,A2,b87),(r4,y2,A2,b88),(r4,y2,A2,b89),(r4,y2,A2,b90),(r4,y2,A2,b91),(r4,y2,A2,b92),( r4,y2,A2,b93),(r4,y2,A2,b94),(r4,y2,A2,b95),(r4,y2,A2,b96),(r4,y2,A2,b97),(r4,y2,A2,b98),(r4,y2,A 2,b99),(r4.y2,A2,b100),(r4,y2,A2,b101),(r4,y2,A2,b102),(r4,y2,A2,b103),(r4,y2,A2,b104),(r4,y2,A2, b105),(r4,y2,A2,b106),(r4,y2,A2,b107),(r4,y2,A2,b108),(r4,y2,A2,b109),(r4,y2,A2,b110),(r4,y2,A2, b111),(r4,y2,A2,b112),(r4,y2,A2,b113),(r4,y2,A2,b114),(r4,y2,A2,b115),(r4,y2,A2,b116),(r4,y2,A2, b117),(r4,y2,A2,b118),(r4,y2,A2,b119),(r4,y2,A2,b120),(r4,y2,A2,b121),(r4,y2,A2,b122),(r4,y2,A2, b123),(r4,y2,A2,b124),(r4,y2,A2,b125),(r4,y2,A2,b126),(r4,y2,A2,b127),(r4,y2,A2,b128),(r4,y2,A2, b129),(r4,y2,A2,b130),(r4,y2,A2,b131),(r4,y2,A2,b132),(r4,y2,A2,b133),(r4,y2,A2,b134),(r4,y2,A3, b1),(r4,y2,A3,b2),(r4,y2,A3,b3),(r4,y2,A3,b4),(r4,y2,A3,b5),(r4,y2,A3,b6),(r4,y2,A3,b7),(r4,y2,A3,b 8),(r4,y2,A3,b9),(r4,y2,A3,b10),(r4,y2,A3,b11),(r4,y2,A3,b12),(r4,y2,A3,b13),(r4,y2,A3,b14),(r4,y2, A3,b15),(r4,y2,A3,b16),(r4,y2,A3,b17),(r4,y2,A3,b18),(r4,y2,A3,b19),(r4,y2,A3,b20),(r4,y2,A3,b21) ,(r4,y2,A3,b22),(r4,y2,A3,b23),(r4,y2,A3,b24),(r4,y2,A3,b25),(r4,y2,A3,b26),(r4,y2,A3,b27),(r4,y2, A3,b28),(r4,y2,A3,b29),(r4,y2,A3,b30),(r4,y2,A3,b31),(r4,y2,A3,b32),(r4,y2,A3,b33),(r4,y2,A3,b34) ,(r4,y2,A3,b35),(r4,y2,A3,b36),(r4,y2,A3,b37),(r4,y2,A3,b38),(r4,y2,A3,b39),(r4,y2,A3,b40),(r4,y2, A3,b41),(r4,y2,A3,b42),(r4,y2,A3,b43),(r4,y2,A3,b44),(r4,y2,A3,b45),(r4,y2,A3,b46),(r4,y2,A3,b47) ,(r4,y2,A3,b48),(r4,y2,A3,b49),(r4,y2,A3,b50),(r4,y2,A3,b51),(r4,y2,A3,b52),(r4,y2,A3,b53),(r4,y2, A3,b54),(r4,y2,A3,b55),(r4,y2,A3,b56),(r4,y2,A3,b57),(r4,y2,A3,b58),(r4,y2,A3,b59),(r4,y2,A3,b60) ,(r4,y2,A3,b61),(r4,y2,A3,b62),(r4,y2,A3,b63),(r4,y2,A3,b64),(r4,y2,A3,b65),(r4,y2,A3,b66),(r4,y2, A3,b67),(r4,y2,A3,b68),(r4,y2,A3,b69),(r4,y2,A3,b70),(r4,y2,A3,b71),(r4,y2,A3,b72),(r4,y2,A3,b73) ,(r4,y2,A3,b74),(r4,y2,A3,b75),(r4,y2,A3,b76),(r4,y2,A3,b77),(r4,y2,A3,b78),(r4,y2,A3,b79),(r4,y2, A3,b80),(r4,y2,A3,b81),(r4,y2,A3,b82),(r4,y2,A3,b83),(r4,y2,A3,b84),(r4,y2,A3,b85),(r4,y2,A3,b86) ,(r4,y2,A3,b87),(r4,y2,A3,b88),(r4,y2,A3,b89),(r4,y2,A3,b90),(r4,y2,A3,b91),(r4,y2,A3,b92),(r4,y2, A3,b93),(r4,y2,A3,b94),(r4,y2,A3,b95),(r4,y2,A3,b96),(r4,y2,A3,b97),(r4,y2,A3,b98),(r4,y2,A3,b99) ,(r4,y2,A3,b100),(r4,y2,A3,b1.01),(r4,y2,A3,b102),(r4,y2,A3,b103),(r4,y2,A3,b104),(r4,y2,A3,b105), (r4,y2,A3,b106),(r4,y2,A3,b107),(r4,y2,A3,b108),(r4,y2,A3,b109),(r4,y2,A3,b110),(r4,y2,A3,b111),( r4,y2,A3,b112),(r4,y2,A3,b113),(r4,y2,A3,b114),(r4,y2,A3,b115),(r4,y2,A3,b116),(r4,y2,A3,b117),( r4,y2,A3,b118),(r4,y2,A3,b119),(r4,y2,A3,b120),(r4,y2,A3,b121),(r4,y2,A3,b122),(r4,y2,A3,b123),( r4,y2,A3,b124),(r4,y2,A3,b125),(r4,y2,A3,b126),(r4,y2,A3,bl27),(r4,y2,A3,b128),(r4,y2,A3,b129),( r4,y2,A3,b130),(r4,y2,A3,b131),(r4,y2,A3,b132),(r4,y2,A3,b133),(r4,y2,A3,b134),(r4,y2,A4,b1),(r4, y2,A4,b2),(r4,y2,A4,b3),(r4.y2,A4,b4),(r4,y2,A4,b5),(r4,y2,A4,b6),(r4,y2,A4,b7),(r4,y2,A4,b8),(r4,y 2,A4,b9),(r4,y2,A4,b10),(r4,y2,A4,b11),(r4,y2,A4,b12),(r4,y2,A4,b13),(r4,y2,A4,b14),(r4,y2,A4,b15 ),(r4,y2,A4,b16),(r4,y2,A4,b17),(r4,y2,A4,b18),(r4,y2,A4,b19),(r4,y2,A4,b20),(r4,y2,A4,b21),(r4,y2, A4,b22),(r4,y2,A4,b23),(r4,y2,A4,b24),(r4,y2,A4,b25),(r4,y2,A4,b26),(r4,y2,A4,b27),(r4,y2,A4,b28) ,(r4,y2,A4,b29),(r4,y2,A4,b30),(r4,y2,A4,b31),(r4,y2,A4,b32),(r4,y2,A4,b33),(r4,y2,A4,b34),(r4,y2, A4,b35),(r4,y2,A4,b36),(r4,y2,A4,b37),(r4,y2,A4,b38),(r4,y2,A4,b39),(r4,y2,A4,b40),(r4,y2,A4,b41) ,(r4,y2,A4,b42),(r4,y2,A4,b43),(r4,y2,A4,b44),(r4,y2,A4,b45),(r4,y2,A4,b46),(r4,y2,A4,b47),(r4,y2, A4,b48),(r4,y2,A4,b49),(r4,y2,A4,b50),(r4,y2,A4,b51),(r4,y2,A4,b52),(r4,y2,A4,b53),(r4,y2,A4,b54) ,(r4,y2,A4,b55),(r4,y2,A4,b56),(r4,y2,A4,b57),(r4,y2,A4,b58),(r4,y2,A4,b59),(r4,y2,A4,b60),(r4,y2, A4,b61),(r4,y2,A4,b62),(r4,y2,A4,b63),(r4,y2,A4,b64),(r4,y2,A4,b65),(r4,y2,A4,b66),(r4,y2,A4,b67) ,(r4,y2,A4,b68),(r4,y2,A4,b69),(r4,y2,A4,b70),(r4,y2,A4,b71),(r4,y2,A4,b72),(r4,y2,A4,b73),(r4,y2, A4,b74),(r4,y2,A4,b75),(r4,y2,A4,b76),(r4,y2,A4,b77),(r4,y2,A4,b78),(r4,y2,A4,b79),(r4,y2,A4,b80) ,(r4,y2,A4,b81),(r4,y2,A4,b82),(r4,y2,A4,b83),(r4,y2,A4,b84),(r4,y2,A4,b85),(r4,y2,A4,b86),(r4,y2, A4,b87),(r4,y2,A4,b88),(r4,y2,A4,b89),(r4,y2,A4,b90),(r4,y2,A4,b91),(r4,y2,A4,b92),(r4,y2,A4,b93) ,(r4,y2,A4,b94),(r4,y2,A4,b95),(r4,y2,A4,b96),(r4,y2,A4,b97),(r4,y2,A4,b98),(r4,y2,A4,b99),(r4,y2, A4,b100),(r4,y2,A4,b101),(r4,y2,A4,b102),(r4,y2,A4,b103),(r4,y2,A4,b104),(r4,y2,A4,b105),(r4,y2, A4,b106),(r4,y2,A4,b107),(r4,y2,A4,b108),(r4,y2,A4,b109),(r4,y2,A4,b110),(r4,y2,A4,b111),(r4,y2, A4,b112),(r4,y2,A4,b113),(r4,y2,A4,b114),(r4,y2,A4,b115),(r4,y2,A4,b116),(r4,y2,A4,b117),(r4,y2, A4,b118),(r4,y2,A4,b119),(r4,y2,A4,b120),(r4,y2,A4,b121),(r4,y2,A4,b122),(r4,y2,A4,b123),(r4,y2, A4,b124),(r4,y2,A4,b125),(r4,y2,A4,b126),(r4,y2,A4,b127),(r4,y2,A4,b128),(r4,y2,A4,b129),(r4,y2, A4,b130),(r4,y2,A4,b131),(r4,y2,A4,b132),(r4,y2,A4,b133),(r4,y2,A4,b134),

(r5,y1,A1,b1),(r5,y1,A1,b2),(r5,y1,A1,b3),(r5,y1,A1,b4),(r5,y1,A1,b5),(r5,y1,A1,b6),(r5,y1,A1,b7),( r5,y1,A1,b8),(r5,y1,A1,b9),(r5,y1,A1,blO),(r5,y1,A1,b11),(r5,y1,A1,b12),(r5,y1,A1,b13),(r5,y1,A1,b 14),(r5,y1,A1,b15),(r5,y1,A1,b16),(r5,y1,A1,b17),(r5,y1,A1,b18),(r5,y1,A1,b19),(r5,y1,A1,b20),(r5, y1,A1,b21),(r5,y1,A1,b22),(r5,y1,A1,b23),(r5,y1,A1,b24),(r5,y1,A1,b25),(r5,y1,A1,b26),(r5,y1,A1,b 27),(r5,y1,A1,b28),(r5,y1,A1,b29),(r5,y1,A1,b30),(r5,y1,A1,b31),(r5,y1,A1,b32),(r5,y1,A1,b33),(r5, y1,A1,b34),(r5,y1,A1,b35),(r5,y1,A1,b36),(r5,y1,A1,b37),(r5,y1,A1,b38),(r5,y1,A1,b39),(r5,y1,A1,b 40),(r5,y1,A1,b41),(r5,y1,A1,b42),(r5,y1,A1,b43),(r5,y1,A1,b44),(r5,y1,A1,b45),(r5,y1,A1,b46),(r5, yl,Al,b47),(r5,yl,Al,b48),(r5,yl,Al,b49),(r5,yl,Al,b50),(r5,yI,Al,b51),(r5,yl,Al,b52),(r5,yl,Al,b 53),(r5,y1,A1,b54),(r5,y1,A1,b55),(r5,y1,A1,b56),(r5,y1,A1,b57),(r5,y1,A1,b58),(r5,y1,A1,b59),(r5, y1,A1,b60),(r5,y1,A1,b61),(r5,y1,A1,b62),(r5,y1,A1,b63),(r5,y1,A1,b64),(r5,y1,A1,b65),(r5,y1,A1,b 66),(r5,y1,A1,b67),(r5,y1,A1,b68),(r5,y1,A1,b69),(r5,y1,A1,b70),(r5,y1,A1,b71),(r5,y1,A1,b72),(r5, y1,A1,b73),(r5,y1,A1,b74),(r5,y1,A1,b75),(r5,y1,A1,b76),(r5,y1,A1,b77),(r5,y1,A1,b78),(r5,y1,A1,b 79),(r5,y1,A1,b80),(r5,y1,A1,b81),(r5,y1,A1,b82),(r5,y1,A1,b83),(r5,y1,A1,b84),(r5,y1,A1,b85),(r5, y1,A1,b86),(r5,y1,A1,b87),(r5,y1,A1,b88),(r5,y1,A1,b89),(r5,y1,A1,b90),(r5,y1,A1,b91),(r5,y1,A1,b 92),(r5,y1,A1,b93),(r5,y1,A1,b94),(r5,y1,A1,b95),(r5,y1,A1,b96),(r5,y1,A1,b97),(r5,y1,A1,b98),(r5, y1,A1,b99),(r5,y1,A1,b100),(r5,y1,A1,b101),(r5,y1,A1,b102),(r5,y1,A1,b103),(r5,y1,A1,b104),(r5,y 1,A1,b105),(r5,y1,A1,b106),(r5,y1,A1,b107),(r5,y1,A1,b108),(r5,y1,A1,b109),(r5,y1,A1,b110),(r5,y 1,A1,b111),(r5,y1,A1,b112),(r5,y1,A1,b113),(r5,y1,A1,b114),(r5,y1,A1,b115),(r5,y1,A1,b116),(r5,y 1,A1,b117),(r5,y1,A1,b118),(r5,y1,A1,b119),(r5,y1,A1,b120),(r5,y1,A1,b121),(r5,y1,A1,b122),(r5,y 1,A1,b123),(r5,y1,A1,b124),(r5,y1,A1,b125),(r5,y1,A1,b126),(r5,y1,A1,b127),(r5,y1,A1,b128),(r5,y 1,A1,b129),(r5,y1,A1,b130),(r5,y1,A1,b131),(r5,y1,A1,b132),(r5,y1,A1,b33),(r5,y1,A1,b134),(r5,y 1,A2,b1),(r5,y1,A2,b2),(r5,y1,A2,b3),(r5,y1,A2,b4),(r5,y1,A2,b5),(r5,y1,A2,b6),(r5,y1,A2,b7),(r5,y1 ,A2,b8),(r5,y1,A2,b9),(r5,y1,A2,b10),(r5,y1,A2,b11),(r5,y1,A2,b12),(r5,y1,A2,b13),(r5,y1,A2,b14),( r5,y1,A2,b15),(r5,y1,A2,b16),(r5,y1,A2,b17),(r5,y1,A2,b18),(r5,y1,A2,b19),(r5,y1,A2,b20),(r5,y1,A 2,b21),(r5,y1,A2,b22),(r5,y1,A2,b23),(r5,y1,A2,b24),(r5,y1,A2,b25),(r5,y1,A2,b26),(r5,y1,A2,b27)_{,}( r5,y1,A2,b28),(r5,y1,A2,b29),(r5,y1,A2,b30),(r5,y1,A2,b31),(r5,y1,A2,b32),(r5,y1,A2,b33),(r5,y1,A 2,b34),(r5,y1,A2,b35),(r5,y1,A2,b36),(r5,y1,A2,b37),(r5,y1,A2,b38),(r5,y1,A2,b39),(r5,y1,A2,b40),( r5.y1,A2,b41),(r5,y1,A2,b42),(r5,y1,A2,b43),(r5,y1,A2,b44),(r5,y1,A2,b45),(r5,y1,A2,b46),(r5,y1,A 2,b47),(r5,y1,A2,b48),(r5,yl,A2,b49),(r5,y1,A2,b50),(r5,y1,A2,b51),(r5,y1,42,b52),(r5,y1,A2,b53),( r5,y1,A2,b54),(r5,y1,A2,b55),(r5,y1,A2,b56),(r5,y1,A2,b57),(r5,y1,A2,b58),(r5,y1,A2,b59),(r5,y1,A 2,b60),(r5,y1,A2,b61),(r5,y1,A2,b62),(r5,y1,A2,b63),(r5,y1,A2,b64),(r5,y1,A2,b65),(r5,y1,A2,b66),( r5,y1,A2,b67),(r5,y1,A2,b68),(r5,y1,A2,b69),(r5,y1,A2,b70),(r5,y1,A2,b71),(r5,y1,A2,b72),(r5,y1,A 2,b73),fr5,y1,A2,b74),(r5,y1,A2,b75),(r5,y1,A2,b76),(r5,y1,A2,b77).(r5,y1,A2,b78),(r5,y1,A2,b79),( r5,y1,A2,b80),(r5,y1,A2,b81),(r5,y1.,A2,b82),(r5,y1,A2,b83),(r5,y1,A2,b84),(r5,y1,A2,b85),(r5,y1,A 2,b86),(r5,y1,A2,b87),(r5,y1,A2,b88),(r5,y1,A2,b89),(r5,y1,A2,b90),(ri,y1,A2,b91),(r5,y1,A2,b92).( r5,y1,A2,b93),(r5,y1,A2,b94),(r5,y1,A2,b95),(r5,y1,A2,b96),(r5,y1,A2,b97),(r5,y1,A2,b98),(r5,y1,A 2,b99),(r5,y1,r12,b100),(r5,y1,A2,b101),(r5,y1,A2,b102),(r5,y1,A2,b103),(r5,y1,A2,b104),(r5,y1,A2, b105),(r5,y1,A2,b106),(r5,y1,A2,b107),(r5,y1,A2,b108),(r5,y1,A2,b109),(r5,y1,A2,b110),(r5,y1,A2, b111),(r5,y1,A2,b112),(r5,y1,A2,b113),(r5,y1,A2,b114),(r5,y1,A2,b115),(r5,y1,A2,b116),(r5,y1,A2, b117),(r5,y1,A2,b118),(r5,y1,A2,b119),(r5,y1,A2,b120),(r5,y1,A2,b121),(r5,y1,A2,b122),(r5,y1,A2, b123),(r5,y1,A2,b124),(r5,y1,A2,b125),(r5,y1,A2,b126),(r5,y1,A2,b127),(r5,y1,A2,b128),(r5,y1,A2, b129),(r5,y1,A2,b130),(r5,y1,A2,b131),(r5,y1,A2,b132),(r5,y1,A2,b133),(r5,y1,A2,b134),(r5,y1,A3, b1),(r5,y1,A3,b2),(r5,y1,A3,b3),(r5,y1,A3,b4),(r5,y1,A3,b5),(r5,y1,A3,b6),(r5,y1,A3,b7),(r5,y1,A3,b 8),(r5,y1,A3,b9),(r5,y1,A3,b10),(r5,y1,A3,b11),(r5,y1,A3,b12),(r5,y1,A3,b13),(r5,y1,A3,b14),(r5,y1, A3,b15),(r5,y1,A3,b16),(r5,y1,A3,b17),(r5,y1,A3,b18),(r5,y1,A3,b19),(r5,y1,A3,b20),(r5,y1,A3,b21) ,(r5,y1,A3,b22),(r5,y1,A3,b23),(r5,y1,A3,b24),(r5,y1,A3,b25),(r5,y1,A3,b26),(r5,y1,A3,b27),(r5,y1, A3,b28),(r5,y1,A3,b29),(r5,y1,A3,b30),(r5,y1,A3,b31),(r5,y1,A3,b32),(r5,y1,A3,b33),(r5,y1,A3,b34) ,(r5,y1,A3,b35),(r5,y1,A3,b36),(r5,y1,A3,b37),(r5,y1,A3,b38),(r5,y1,A3,b39),(r5,y1,A3,b40),(r5,y1, A3,b41),(r5,y1,A3,b42),(r5,y1,A3,b43),(r5,y1,A3,b44),(r5,y1,A3,b45),(r5,y1,A3,b46),(r5,y1,A3,b47) ,(r5,y1,A3,b48),(r5,y1,A3,b49),(r5,y1,A3,b50),(r5,y1,A3,b51),(r5,y1,A3,b52),(r5,y1,A3,b53),(r5,y1, A3,b54),(r5,y1,A3,b55),(r5,y1,A3,b56),(r5,y1,A3,b57),(r5,y1,A3,b58),(r5,y1,A3,b59),(r5,y1,A3,b60) ,(r5,y1,A3,b61),(r5,y1,A3,b62),(r5,y1,A3,b63),(r5,y1,A3,b64),(r5,y1,A3,b65),(r5,y1,A3,b66),(r5,y1, A3,b67),(r5,y1,A3,b68),(r5,y1,A3,b69),(r5,y1,A3,b70),(r5,y1,A3,b71),(r5,y1,A3,b72),(r5,y1,A3,b73) ,(r5,y1,A3,b74),(r5,y1,A3,b75),(r5,y1,A3,b76),(r5,y1,A3,b77),(r5,y1,A3,b78),(r5,y1,A3,b79),(r5,y1, A3,b80),(r5,y1,A3,b81),(r5,y1,A3,b82),(r5,y1,A3,b83),(r5,y1,A3,b84),(r5,y1,A3,b85),(r5,y1,A3,b86) ,(r5,y1,A3,b87),(r5,y1,A3,b88),(r5,y1,A3,b89),(r5,y1,A3,b90),(r5,y1,A3,b91),(r5,y1,A3,b92),(r5,y1, A3,b93),(r5,y1,A3,b94),(r5,y1,A3,b95),(r5,y1,A3,b96),(r5,y1,A3,b97),(r5,y1,A3,b98),(r5,y1,A3,b99) ,(r5,y1,A3,b100),(r5,y1,A3,b101),(r5,y1,A3,b102),(r5,y1,A3,b103),(r5,y1,A3,b104),(r5,y1,A3,b105), (r5,y1,A3,b106),(r5,y1,A3,b107),(r5,y1,A3,b108),(r5,y1,A3,b109),(r5,y1,A3,b110),(r5,y1,A3,b111),( r5,y1,A3,b112),(r5,y1,A3,b113),(r5,y1,A3,b114),(r5,y1,A3,b115),(r5,y1,A3,b116),(r5,y1,A3,b117),( r5,y1,A3,b118),(r5,y1,A3,b119),(r5,y1,A3,b120),(r5,y1,A3,b121),(r5,y1,A3,b122),(r5,y1,A3,b123),( r5,y1,A3,b124),(r5,y1,A3,b125),(r5,y1,A3,b126),(r5,y1,A3,b127),(r5,y1,A3,b128),(r5,y1,A3,b129),( r5,y1,A3,b130),(r5,y1,A3,b131),(r5,y1,A3,b132),(r5,y1,A3,b133),(r5,y1,A3,b134),(r5,y1,A4,b1),(r5, y1,A4,b2),(r5,y1,A4,b3),(r5,y1,A4,b4),(r5,y1,A4,b5),(r5,y1,A4,b6),(r5,y1,A4,b7),(r5,y1,A4,b8),(r5,y 1,A4,b9),(r5,y1,A4,b20),(r5,y1,A4,b11),(r5,y1,A4,b12),(r5,y1,A4,b13),(r5,y1,A4,b14),(r5,y1,A4,b15 ),(r5,y1,A4,b16),(r5,y1,A4,b17),(r5,y1,A4,b18),(r5,y1,A4,b19),(r5,y1,A4,b20),(r5,y1,A4,b21),(r5,y1, A4,b22),(r5,y1,A4,b23),(r5,y1,A4,b24),(r5,y1,A4,b25),(r5,y1,A4,b26),(r5,y1,A4,b27),(r5,y1,A4,b28) ,(r5,y1,A4,b29),(r5,y1,A4,b30),(r5,y1,A4,b31),(r5,y1,A4,b32),(r5,y1,A4,b33),(r5,y1,A4,b34),(r5,y1, A4,b35),(r5,y1,A4,b36),(r5,y1,A4,b37),(r5,y1,A4,b38),(r5,y1,A4,b39),(r5,y1,A4,b40),(r5,y1,A4,b41) ,(r5,y1,A4,b42),(r5,y1,A4,b43),(r5,y1,A4,b44),(r5,y1,A4,b45),(r5,y1,A4,b46),(r5,y1,A4,b47),(r5,y1 A4,b48),(r5,y1,A4,b49),(r5,y1,A4,b50),(r5,y1,A4,b51),(r5,y1,A4,b52),(r5,y1,A4,b53),(r5,y1,A4,b54) (r5,y1,A4,b55),(r5,y1,A4,b56),(r5,y1,A4,b57),(r5,y1,A4,b58),(r5,y1,A4,b59),(r5,y1,A4,b60),(r5,y1, A4,b61),(r5,y1,A4,b62),(r5,y1,A4,b63),(r5,y1,A4,b64),(r5,y1,A4,b65),(r5,y1,A4,b66),(r5,y1,A4,b67) ,(r5,y1,A4,b68),(r5,y1,A4,b69),(r5,y1,A4,b70),(r,5,y1,A4,b71),(r5,y1,A4,b72),(r5,y1,A4,b73),(r5,y1, A4,b74),(r5,y1,A4,b75),(r5,y1,A4,b76),(r5,y1,A4,b77),(r5,y1,A4,b78),(r5,y1,A4,b79),(r5,y1,A4,b80) ,(r5,y1,A4,b81),(r5,y1,A4,b82),(r5,y1,A4,b83),(r5,y1,A,1,b84),(r5,y1,A4,b85),(r5,y1,A4,b86),(r5,y1, A4,b87),(r5,y1,A4,b88),(r5,y1,A4,b89),(r5,y1,A4,b90),(r5,y1,A4,b91),(r5,y1,A4,b92),(r5,y1,A4,b93) ,(r5,y1,A4,b94),(r5,y1,A4,b95),(r5,y1,A4,b96),(r5,y1,A4,b97),(r5,y1,A4,b98),(r5,y1,A4,b99),(r5,y1, A4,b100),(r5,y1,A4,b101),(r5,y1,A4,b102),(r5,y1,A4,b103),(r5,y1,A4,b104),(r5,y1,A4,b105),(r5,y1, A4,b106),(r5,y1,A4,b107),(r5,y1,A4,b108),(r5,y1,A4,b109),(r5,y1,A4,b110),(r5,y1,A4,b111),(r5,y1, A4,b112),(r5,y1,A4,b113),(r5,y1,A4,b114),(r5,y1,A4,b115),(r5,y1,A4,b116),(r5,y1,A4,b117),(r5,y1, A4,b118),(r5,y1,A4,b119),(r5,y1,A4,b120),(r5,y1,A4,b121),(r5,y1,A4,b122),(r5,y1,A4,b123),(r5,y1, A4,b124),(r5,y1,A4,b125),(r5,y1,A4,b126),(r5,y1,A4,b127),(r5,y1,A4,b128),(r5,y1,A4,b129),(r5,y1, A4,b130),(r5,y1,A4,b131),(r5,y1,A4,b132),(r5,y1,A4,b133),(r5,y1,A4,b134),

(r5,y2,A1,b1),(r5,y2,A1,b2),(r5,y2,A1,b3),(r5,y2,A1,b4),(r5,y2,A1,b5),(r5,y2,A1,b6),(r5,y2,A1,b7),( r5,y2,A1,bB),(r5,y2,A1,b9),(r5,y2,A1,b10),(r5,y2,A1,b11),(r5,y2,A1,b12),(r5,y2,A1,b13),(r5,y2,A1,b 14),(r5,y2,A1,b15),(r5,y2,A1,b16),(r5,y2,A1,b17),(r5,y2,A1,b18),(r5,y2,A1,b19),(r5,y2,A1,b20),(r5, y2,A1,b21),(r5,y2,A1,b22),(r5,y2,A1,b23),(r5,y2,A1,b24),(r5,y2,A1,b25),(r5,y2,A1,b26),(r5,y2,A1,b 27),(r5,y2,A1,b28),(r5,y2,A1,b29),(r5,y2,A1,b30),(r5,y2,A1,b31),(r5,y2,A1,b32),(r5,y2,A1,b33),(r5, y2,A1,b34),(r5,y2,A1,b35),(r5,y2,A1,b36),(r5,y2,A1,b37),(r5,y2,A1,b3S),(r5,y2,A1,b39),(r5,y2,A1,b 40),(r5,y2,A1,b41),(r5,y2,A1,b42),(r5,y2,A1,b43),(r5,y2,A1,b44),(r5,y2,A1,b45),(r5,y2,A1,b46),(r5, y2;A1,b47),(r5,y2,A1,b48),(r5,y2,A1,b49),(r5,y2,A1,b50),(r5,y2,A1,b51),(r5,y2,A1,b52),(r5,y2,A1,b 53),(r5,y2,A1,b54),(r5,y2,A1,b55),(r5,y2,A1,b56),(r5,y2,A1,b57),(r5,y2,A1,b58),(r5,y2,A1,b59),(r5, y2,A1,b60),(r5,y2,A1,b61),(r5,y2,A1,b62),(r5,y2,A1,b63),(r5,y2,A1,b64),(r5,y2,A1,b65),(r5,y2,A1,b 66),(r5,y2,A1,b67),(r5,y2,A1,b68),(r5,y2,A1,b69),(r5,y2,A1,b70),(r5,y2,A1,b71),(r5,y2,A1,b72),(r5, y2,A1,b73),(r5,y2,A1,b74),(r5,y2,A1,b75),(r5,y2,A1,b76),(r5,y2,A1,b77),(r5,y2,A1,b78),(r5,y2,A1,b 79),(r5,y2,A1,b80),(r5,y2,A1,b81),(r5,y2,A1,b82),(r5,y2,A1,b83),(r5,y2,A1,b84),(r5,y2,A1,b85),(r5, y2,A1,b86),(r5,y2,A1,b87),(r5,y2,A1,b88),(r5,y2,A1,b89),(r5,y2,A1,b90),(r5,y2,A1,b91),(r5,y2,A1,b 92),(r5,y2,A1,b93),(r5,y2,A1,b94),(r5,y2_{;}A1,b95),(r5,y2,A1,b96),(r5,y2,A1,b97),(r5,y2,A1,b98),(r5, y2,A1,b99),(r5,y2,A1,b100),(r5,y2,A1,b101),(r5,y2,A1,b102),(r5,y2,A1,b103),(r5,y2,A1,b104),(r5,y 2,A1,b105),(r5,y2,A1,b106),(r5,y2,A1,b107),(r5,y2,A1,b108),(r5,y2,A1,b109),(r5,y2,A1,b110),(r5,y 2,A1,b111),(r5,y2,A1,b112),(r5,y2,A1,b113),(r5,y2,A1,b114),(r5,y2,A1,b115),(r5,y2,A1,b116),(r5,y 2,A1,b117),(r5,y2,A1,b118),(r5,y2,A1,b119),(r5,y2,A1,b120),(r5,y2,A1,b121),(r5,y2,A1,b122),(r5,y 2,A1,b123),(r5,y2,A1,b124),(r5,y2,A1,b125),(r5,y2,A1,b126),(r5,y2,A1,b127),(r5,y2,A1,b128),(r5,y 2,A1,b129),(r5,y2,A1,b130),(r5,y2,A1,b131),(r5,y2,A1,b132),(r5,y2,A1,b133),(r5,y2,A1,b134),(r5,y 2,A2,b1),(r5,y2,A2,b2),(r5,y2,A2,b3),(r5,y2,A2,b4),(r5,y2,A2,b5),(r5,y2,A2,b6),(r5,y2,A2,b7),(r5,y2 ,A2,b8),(r5,y2,A2,b9),(r5,y2,A2,b10),(r5,y2,A2,b11),(r5,y2,A2,b12),(r5,y2,A2,b13),(r5,y2,A2,b14),( r5,y2,A2,b15),(r5,y2,A2,b16),(r5,y2,A2,b17),(r5,y2,A2,b18),(r5,y2,A2,b19),(r5,y2,A2,b20),(r5,y2,A 2,b21),(r5,y2,A2,b22),(r5,y2,A2,b23),(r5,y2,A2,b24),(r5,y2,A2,b25),(r5,y2,A2,b26),(r5,y2,A2,b27),( r5,y2,A2,b28),(r5,y2,A2,b29),(r5,y2,A2,b30),(r5,y2,A2,b3l),(r5,y2,A2,b32),(r5,y2,A2,b33),(r5,y2,A 2,b34),(r5,y2,A2,b35),(r5,y2,A2,b36),(r5,y2,A2,b37),(r5,y2,A2,b38),(r5,y2,A2,b39),(r5,y2,A2,b40),( r5,y2,A2,b41),(r5,y2,A2,b42),(r5,y2,A2,b43),(r5,y2,A2,b44),(r5,y2,A2,b45),(r5,y2,A2,b46),(r5,y2,A 2,b47),(r5,y2,A2,b48),(r5,y2,A2,b49),(r5,y2,A2,b50),(r5,y2,A2,b51),(r5,y2,A2,b52),(r5,y2,A2,b53),( r5,y2,A2,b54),(r5,y2,A2,b55),(r5,y2,A2,b56),(r5,y2,A2,b57),(r5,y2,A2,b58),(r5,y2,A2,b59),(r5,y2,A 2,b60),(r5,y2,A2,b61),(r5,y2,A2,b62),(r5,y2,A2,b63),(r5,y2,A2,b64),(r5,y2,A2,b65),(r5,y2,A2,b66),( r5,y2,A2,b67),(r5,y2,A2,b68),(r5,y2,A2,b69),(r5,y2,A2,b70),(r5,y2,A2,b71),(r5,y2,A2,b72),(r5,y2,A 2,b73),(r5,y2,A2,b74),(r5,y2,A2,b75),(r5,y2,A2,b76),(r5,y2,A2,b77),(r5,y2,A2,b78),(r5,y2,A2,b79),( r5,y2,A2,b80),(r5,y2,A2,b81),(r5,y2,A2,b82),(r5,y2,A2,b83),(r5,y2,A2,b84),(r5,y2,A2,b85),(r5,y2,A 2,b86),(r5,y2,A2,b87),(r5,y2,A2,b88),(r5,y2,A2,b89),(r5,y2,A2,b90),(r5,y2,A2,b91),(r5,y2,A2,b92),( r5,y2,A2,b93),(r5,y2,A2,b94),(r5,y2,A2,b95),(r5,y2,A2,b96),(r5,y2,A2,b97),(r5,y2,A2,b98),(r5,y2,A 2,b99),(r5,y2,A2,b100),(r5,y2,A2,b101),(r5,y2,A2,b102),(r5,y2,A2,b103),(r5,y2,A2,b104),(r5,y2,A2, b105),(r5,y2,A2,b106),(r5,y2,A2,b107),(r5,y2,A2,b108),(r5,y2,A2,b109),(r5,y2,A2,bl10),(r5,y2,A2, b111),(r5,y2,A2,b112),(r5,y2,A2,b113),(r5,y2,A2,b114),(r5,y2,A2,b115),(r5,y2,A2,b116),(r5,y2,A2, b117),(r5,y2,A2,b118),(ri,y2,A2,b119),(r5,y2,A2,b120),(r5,y2,A2,b121),(r5,y2,A2,b122),(r5,y2,A2, b123),(r5,y2,A2,b124),(r5,y2,A2,b125),(r5,y2,A2,b126),(r5,y2,A2,b127),(r5,y2,A2,b128),(r5,y2,A2, b129),(r5,y2,A2,b130),(r5,y2,A2,b131),(r5,y2,A2,b132),(r5,y2,A2,b133),(r5,y2,A2,b134),(r5,y2,A3, b1),(r5,y2,A3,b2),(r5,y2,A3,b3),(r5,y2,A3,b4),(r5,y2,A3,b5),(r5,y2,A3,b6),(r5,y2,A3,b7),(r5,y2,A3,b 8),(r5,y2,A3,b9),(r5,y2,A3,b10),(r5,y2,A3,b11),(r5,y2,A3,b12),(r5,y2,A3,b13),(r5,y2,A3,b14),(r5,y2, A3,b15),(r5,y2,A3,b16),(r5,y2,A3,b17),(r5,y2,A3,b18),(r5,y2,A3,b19),(r5,y2,A3,b20),(r5,y2,A3,b21) ,(r5,y2,A3,b22),(r5,y2,A3,b23),(r5,y2,A3,b24),(r5,y2,A3,b25),(r5,y2,A3,b26),(r5,y2,A3,b27),(r5,y2, A3,b28),(r5,y2,A3,b29),(r5,y2,A3,b30),(r5,y2,A3,b31),(r5,y2,A3,b32),(r5,y2,A3,b33),(r5,y2,A3,b34) ,(r5,y2,A3,b35),(r5,y2,A3,b36),(r5,y2,A3,b37),(r5,y2,A3,b38),(r5,y2,A3_{;}b39),(r5,y2,A3,b40),(r5,y2, A3,b41),(r5,y2,A3,b42),(r5,y2,A3,b43),(r5,y2,A3,b44),(r5,y2,A3,b45),(r5,y2,A3,b46),(r5,y2,A3,b47) ,(r5,y2,A3,b48)_{,}(r5,y2,A3,b49),(r5,y2,A3,b50),(r5,y2,A3,b51),(r5,y2,A3,b52),(r5,y2,A3,b53),(r5,y2, A3,b54),(r5,y2,A3,b55),(r5,y2,A3,b56),(r5,y2,A3,b57),(r5,y2,A3,b58),(r5,y2,A3,b59),(r5,y2,A3,b60) ,(r5,y2,A3,b61),(r5,y2,A3,b62),(r5,y2,A3,b63),(r5,y2,A3,b64),(r5,y2,A3,b65),(r5,y2,A3,b66),(r5,y2, A3,b67),(r5,y2,A3,b68),(r5,y2,A3,b69),(r5,y2,A3,b70),(r5,y2,A3,b71),(r5,y2,A3,b72),(r5,y2,A3,b73) ,(r5,y2,A3,b74),(r5,y2,A3,b75),(r5,y2,A3,b76),(r5,y2,A3,b77),(r5,y2,A3,b78),(r5,y2,A3,b79),(r5,y2, A3,b80),(r5,y2,A3,b81),(r5,y2,A3,b82),(r5,y2,A3,b83),(r5,y2,A3,b84),(r5,y2,A3,b85),(r5,y2,A3,b86) ,(r5,y2,A3,b87),(r5,y2,A3,b88),(r5,y2,A3,b89),(r5,y2,A3,b90),(r5,y2,A3,b91),(r5,y2,A3,b92),(r5,y2, A3,b93),(r5,y2,A3,b94),(r5,y2,A3,b95),(r5,y2,A3,b96),(r5,y2,A3,b97),(r5,y2,A3,b98),(r5,y2,A3,b99) ,(r5,y2,A3,b100),(r5,y2,A3,b101),(r5,y2,A3,b102),(r5,y2,A3,b103),(r5,y2,A3,b104),(r5,y2,A3,b105), (r5,y2,A3,b106),(r5,y2,A3,b107),(r5,y2,A3,b108),(r5,y2,A3,b109),(r5,y2,A3,b110),(r5,y2,A3,b111).( r5,y2,A3,b112),(r5,y2,A3,b113),(r5,y2,A3,b114),(r5,y2,A3,b115),(r5,y2,A3,b116),(r5,y2,A3,b117),( r5,y2,A3,b118),(r5,y2,A3,b119),(r5,y2,A3,b120),(r5,y2,A3,b12l),(r5,y2,A3,b122),(r5,y2,A3,b123),( r5,y2,A3,b124),(r5,y2,A3,b125),(r5,y2,A3,b126),(r5,y2,A3,b127),(r5,y2,A3,b128),(r5,y2,A3,b129),( r5,y2,A3,b130),(r5,y2,A3,b131),(r5,y2,A3,bI32),(r5,y2,A3,b133),(r5,y2,A3,b134),(r5,y2,A4,b1),(r5, y2,A4,b2),(r5,y2,A4,b3),(r5,y2,A4,b4),(r5,y2,A4,b5),(r5,y2,A4,b6),(r5,y2,A4,b7),(r5,y2,A4,b8),(r5,y 2,A4,b9),(r5,y2,A4,b10),(r5,y2,A4,b11),(r5,y2,A4,b12),(r5,y2,A4,b13),(r5,y2,A4,b14),(r5,y2,A4,b15 ),(r5,y2,A4,b16),(r5,y2,A4,b17),(r5,y2,A4,b18),(r5,y2,A4,b19),(r5,y2,A4,b20),(r5,y2,A4,b21),(r5,y2, A4,b22),(r5,y2,A4,b23),(r5,y2,A4,b24),(r5,y2,A4,b25),(r5,y2,A4,b26),(r5,y2,A4,b27),(r5,y2,A4,b28) ,(r5,y2,A4,b29),(r5,y2,A4,b30),(r5,y2,A4,b3l),(r5,y2,A4,b32),(r5,y2,A4,b33),(r5,y2,A4,b34),(r5,y2, A4,b35),(r5,y2,A4,b36),(r5,y2,A4,b37),(r5,y2,A4,b38),(r5,y2,A4,b39),(r5,y2,A4,b40),(r5,y2,A4,b41) ,(r5,y2,A4,b42),(r5,y2,A4,b43),(r5,y2,A4,b44),(r5,y2,A4,b45),(r5,y2,A4,b46),(r5,y2,A4,b47),(r5,y2, A4,b48),(r5,y2,A4,b49),(r5,y2,A4,b50),(r5,y2,A4,b51),(r5,y2,A4,b52),(r5,y2,A4,b53),(r5,y2,A4,b54) ,(r5,y2,A4,b55),(r5,y2,A4,b56),(r5,y2,A4,b57),(r5,y2,A4,b58),(r5,y2,A4,b59),(r5,y2,A4,b60),(r5,y2, A4,b61),(r5,y2,A4,b62),(r5,y2,A4,b63),(r5,y2,A4,b64),(r5,y2,A4,b65),(r5,y2,A4,b66),(r5,y2,A4,b67) ,(r5,y2,A4,b68),(r5,y2,A4,b69),(r5,y2,A4,b70),(r5,y2,A4,b71),(r5,y2,A4,b72),(r5,y2,A4,b73),(r5,y2, A1,b74),(r5,y2,A4,b75),(r5,y2,A4,b76),(r5,y2,A4,b77),(r5,y2,A4,b78),(r5,y2,A4,b79),(r5,y2,A4,b80) ,(r5,y2,A4,b81),(r5,y2,A4,b82),(r5,y2,A4,b83),(r5,y2,A4,b84),(r5,y2,A4,b85),(r5,y2,A4,b86),(r5,y2, A4,b87),(r5,y2,A4,b88),(r5,y2,A4,b89),(r5,y2,A4,b90),(r5,y2,A4,b91),(r5,y2,A4,b92),(r5,y2,A4,b93) ,(r5,y2,A4,b94),(r5,y2,A4,b95),(r5,y2,A4,b96),(r5,y2,A4,b97),(r5,y2,A4,b98),(r5,y2,A4,b99),(r5,y2, A4,b100),(r5,y2,A4,b101),(r5,y2,A4.b102),(r5,y2,A4,b103),(r5,y2,A4,b104),(r5,y2,A4,b105),(r5,y2, A4,b106),(r5,y2,A4,b107),(r5,y2,A4,b108),(r5,y2,A4,b109),(r5,y2,A4,b110),(r5,y2,A4,b111),(r5,y2, A4,b112),(r5,y2,A4,b113),(r5,y2,A4,b114),(r5,y2,A4,b115),(r5,y2,A4,b116),(r5,y2,A4,b117),(r5,y2, A4,b118),(r5,y2,A4,b119),(r5,y2,A4,b120),(r5,y2,A4,b121),(r5,y2,A4,b122),(r5,y2,A4,b123),(r5,y2, A4,b124),(r5,y2,A4,b125),(r5,y2,A4,b126),(r5,y2,A4,b127),(r5,y2,A4,b128),(r5,y2,A4,b129),(r5,y2, A4,b130),(r5,y2,A4,b131),(r5,y2,A4,b132),(r5,y2,A4,b133) or (r5,y2,A4,b134).

The present compounds are useful in disease induced by the production, secretion or deposition of-amyloid β protein, and are effective in treatment and/or prevention, and symptom improvement of such as dementia of the Alzheimer's type (Alzheimer's disease, senile dementia of Alzheimer type), Down's syndrome, memory impairment, prion disease (Creutzfeldt-Jakob disease), mild cognitive impairment (MCI), Dutch type of hereditary cerebral hemorrhage with amyloidosis, cerebral amyloid angiopathy, other type of degenerative dementia, mixed dementia with Alzheimer's and vascular type, dementia with Parkinson's Disease, dementia with progressive supranuclear palsy, dementia with Cortico-basal degeneration, Alzheimer's disease with diffuse Lewy body disease, age-related macular degeneration, Parkinson's Disease, amyloid angiopathy and so on.

Example of "treating Alzheimer's disease" includes prevention of aggravation of mild cognitive impairment (MCI) and prevention of onset of familial Alzheimer's disease. Example of "a pharmaceutical composition for treating Alzheimer's disease" includes pharmaceutical composition for prevention of aggravation of mild cognitive impairment (MCI) and prevention of onset of familial Alzheimer's disease.

Since the present compound has high inhibitory activity on BACE1, and/or has high selectivity on other enzymes, it can be a medicament with reduced side effect. Further, since the compound has high effect of reducing amyloid β production in a cell system, particularly, has high effect of reducing amyloid β production in brain, it can be an excellent medicament. In addition, by converting the compound into an optically active compound having suitable stereochemistry, the compound can be a medicament having a larger safety margin on the side effect. In addition, the present compound also has advantages that metabolism stability is high, solubility is high, oral absorbability is high, good bioavailability is exhibited, clearance is good, brain transference is high, a half life is high, non-protein binding rate is high, hERG channel inhibition is low, CYP inhibition is low, CYP MBI (irreversible inhibition (mechanism-based inhibition)) is low and/or an Ames test is negative.

The present compounds can be administrated in combination with other pharmaceutical agents such as other therapeutic or preventive drugs for Alzheimer's disease, e.g., acetylcholinesterase and the like. The present compounds can be treated with concomitantly with the anti-dementia agents such as Donepezil Hydrochloride, Tacrine, Galantamine, Rivastigmine, Zanapezil, Memantine, and Vinpocetine.

When the present compound is administered to a human, it can be administered orally as powders, granules, tablets, capsules, pills, solutions, or the like, or parenterally as injectables, suppositories, transdermal absorbable agents, inhalations, or the like. In addition, the present compound can be formulated into pharmaceutical preparations by adding pharmaceutical additives such as excipients, binders, wetting agents, disintegrating agents, lubricants and the like, which are suitable for formulations and an effective amount of the present compound.

A dose is different depending on state of disease, an administration route, and an age and a weight of a patient, and is usually 0.1 µg to 1 g/day, preferably 0.01 to 200 mg/day when orally administered to an adult, and is usually 0.1 µg to 10 g/day, preferably 0.1 to 2 g/day when parenterally administered.

### [Examples]

Following examples and test examples illustrate the present invention in more detail, but the present invention is not limited by these examples.

¹H-NMR was measured in deuterium chloroform (CDCl₃) using tetramethylsilane as an internal standard, or measured in dimethylsulfoxide-D6 (DMSO-d₆). δ values were shown as ppm. Binding constants(J) were shown as Hz. In the data, s, d, t, sext, m, br or brs means singlet, doublet, triplet, sextet, multiplet, broad or broad singlet, respectively.

In example, the meaning of each abbreviation is as follows:
Me methyl
Bz benzoyl
Boc tert-butoxycarbonyl
MPM p-methoxybenzyl
THF tetrahydrofuran

LC/MS data of the present compound were measured under any of the following condition, and a retention time and [M+H]⁺ are shown.

### (Method A)

Column: Xbridge (registered trade name) C18 (5 µm, i.d. 4.6 x 50 mm)(Waters)
Flow rate: 3 mL/min.
UV detection wavelength: 254 nm
Mobile phase: [A] 0.1% formic acid-containing aqueous solution; [B] 0.1% formic acid-containing acetonitrile solution
Gradient: performing linear gradient of 10% to 100% solvent [B] for 3 minutes, and keeping 100% solvent [B] for 1 minute

### (Method B)

column: Shim-pack XR-ODS (2.2 µm, i.d. 50 x 3.0 mm) (Shimadzu)
Flow rate: 1.6 mL/min.
UV detection wavelength: 254 nm
Mobile phase: [A] 0.1% formic acid-containing aqueous solution; [B] 0.1% formic acid-containing acetonitrile solution
Gradient: performing linear gradient of 10% to 100% solvent [B] for 3 minutes, and keeping 100% solvent [B] for 1 minute

### Example 1

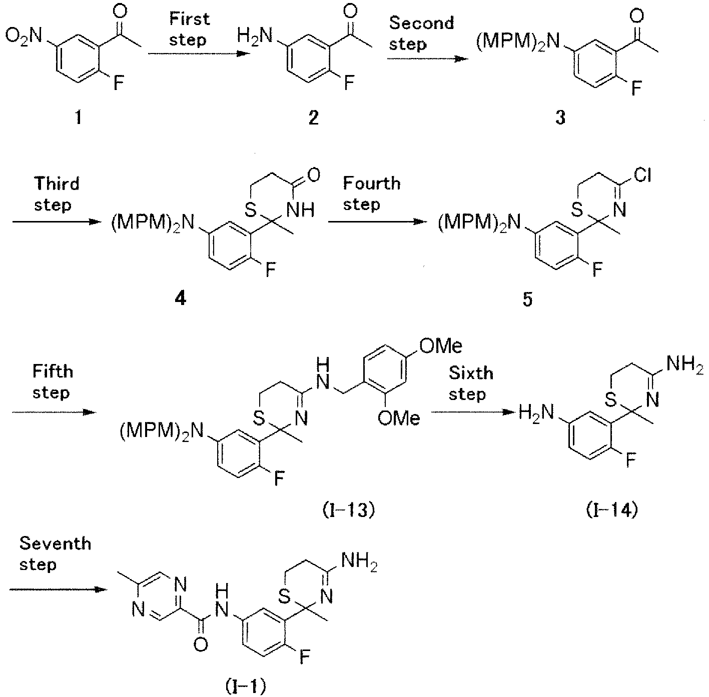

### First step

To a solution of compound 1 (15.00 mg) in ethanol (300 ml) was added 10% Palladium-carbon (3.00 g), and the mixture was reacted under hydrogen atmosphere at room temperature for 8 hours. After removing the insoluble material by filtration, the filtrate was concentrated to give compound 2 (18.75 g) as a crude product.
¹H-NMR (CDCl₃)
δ:2.61(3H,d,J=4.9Hz),6.80(1H,ddd,J=8.8,4.0,3.1Hz),6.93(1H,dd,J=10.6,8.8Hz),7.12(1H,dd,J=5.9, 3.1Hz).

### Second step

To a solution of compound 2 (12.56 g) obtained at First step in N, N-dimethylformamide (130 ml) were added potassium carbonate (45.30 g) and 4-methoxy benzyl bromide (52.80 g), and the mixture was reacted at 50 °C for 7 hours and 30 minutes. After water was added to the mixture, the mixture was extracted with ethyl acetate. The organic layer was dried over anhydrous sodium sulfate. The solvent was evaporated in vacuo. The obtained residue was purified by silica gel column chromatography to give compound 3 (24.90 g).
¹H-NMR (CDCl₃) δ:2.58(3H,d,J=5.0Hz),3.78(6H,s),4.52(4H,s),6.78-6.94(6H,m),7.11(4H,d,J=8.5Hz),7.20-7.25(1H,m).

### Third step

To a solution of compound 3 (24.90 g) in toluene (249 ml) were added p-toluene sulfonic acid (22.49 g) and 3-mercapto propane amide (7.99 g). It was heated under reflux for 14 hours being removed generated water. After water was added to the mixture, the mixture was extracted with ethyl acetate. The organic layer was dried over anhydrous sodium sulfate. The obtained residue was purified by silica gel column chromatography to give compound 4 (1.49 g). ¹H-NMR (CDCl₃) δ:1.88(3H,s),2.13-2.21(2H,m),2.39-2.48(2H,m),3.79(6H,s),4.49-4.63(4H,m),6.19(1H,s),6.60-6.67(2H,m),6.83-6.90(5H,m),7.14(4H,d,J=8.7Hz).

### Fourth step

To compound 4 (2.48 g) was added phosphorus oxychloride (12.4 ml). It was stirred at room temperature for 23 hours. The phosphorus oxychloride was evaporated in vacuo to give the compound 5 as a crude product.

### Fifth step

To a solution of compound 5 obtained at the Fourth step in toluene (12.9 ml) was added 2-4-dimethoxy benzylamine (17.26 g) at room temperature, and the mixture was heated at 100 °C for 5 hours and 30 minutes. Water was added thereto and the mixture was extracted with ethyl acetate and dried over anhydrous sodium sulfate. The solvent was evaporated in vacuo. The obtained residue was purified by silica gel column chromatography to give compound (I-13) (1.82 g).
¹H-NMR(CDCl₃) δ: 1.95(3H,d,J=2.0Hz),2.00-2.11(2H,m),2.31-2.35(2H,m),3.78~3.81(14H,m),4.22-4.52(5H,m),6.41-6.49(4H,m),6.79-6.83(5H,m),7.05(4H,d,J=8.7Hz),7.19-7.26(1H,m).

### Sixth step

To a solution of compound (I-13) was added trifluoroacetic acid (18.2 ml) It was heated under reflux for 22 hours. After water and potassium carbonate were added to the mixture, the mixture was extracted with chloroform. The organic layer was dried over anhydrous sodium sulfate. The solvent was evaporated in vacuo. The obtained residue was purified by silica gel column chromatography to give compound (I-14) (0.63 g).
2.45(2H,m),2.57(1H,ddd,J=13.8,9.1,4.8Hz),2.70(1H,ddd,J=13.8,5.7,4.8Hz),3.50(2H,s),6.50(1H,dt, J=8.5,3.3Hz),6.58(1H,dd,J=7.0,3.3Hz),6.82(1H,dd,J=11.8,8.5Hz).

### Seventh step

5-methyl pyrazine-2-carbonic acid (0.11 g) was dissolved in methanol (1 ml) and 4-(4,6-dimethoxy-1, 3, 5-triazine-2-yl-4-methylmorpholinium chloride (0.26 g) were added thereto. The mixture was stirred at room temperature for 5 minutes, a solution of compound (I-14) (0.15 g) in methanol (1.5 ml) was added thereto.
After being stirred at room temperature for 1 hour, ice water and sodium hydroxide solution were added thereto, and the mixture was extracted with ethyl acetate. The organic layer was dried over anhydrous sodium sulfate. The solvent was evaporated in vacuo. The obtained residue was purified by silica gel column chromatography to give compound (I-1) (0.23 g). ¹H-NMR (CDCl₃) δ=1.97(3H,d,J=1.7Hz),2.32-2.49(2H,m),2.58(1H,ddd,J=13.8,8.4,5.5Hz),2.71-2.75(4H,m),4.44(2H,s),7.06(1H,dd,J=11.4,8.7Hz),7.49(1H,dd,J=8.7,2.7Hz),7.76(1H,ddd,J=8.7,3.9, 2.7Hz),8.42(1H,d,J=0.9Hz),9.35(1H,d,J=0.9Hz),9.57(1H,s).

### Example 2

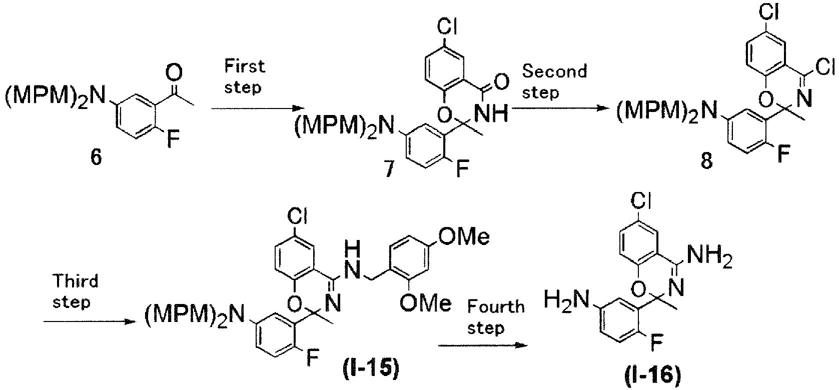

### First step

To a solution of compound 6 (2.00 g) in toluene (30 ml) were added pyrrolidine (0.04 g) and 5-chloro 2-hydroxybenzamide (0.87g). It was heated under reflux for 18 hours being removed generated water. After water were added to the mixture, the mixture was extracted with ethyl acetate. The organic layer was dried over anhydrous sodium sulfate. The solvent was evaporated in vacuo. The obtained residue was purified by silica gel column chromatography to give compound 7 (0.72 g).
¹H-NMR (CDCl₃) δ:1.93(3H,s),3.81(6H,s),4.36(2H,d,J=16.8Hz),4.49(2H,d,J=16.8Hz),6.52-6.61(4H,m),6.78.6.87(5H,m),6.95(4H,d,J=8.1Hz),7.11(1H,dd,J=8.7,2.6Hz),7.74(1H,d,J=2.6Hz).

### Second step

To compound 7 (0.48 g) was added phosphorus oxychloride (1.5 ml). It was stirred at 80 °C for 2.5 hours. The phosphorus oxychloride was evaporated in vacuo to give the compound 8 as a crude product.

### Third step

To a solution of compound 8 obtained in Second step in toluene (5 ml) was added 2-4-dimethoxybenzylamine (1.47 g) with stirring at room temperature. It was heated under reflux for 1.5 hours. After water was added to the mixture, the mixture was extracted with ethyl acetate. The organic layer was dried over anhydrous sodium sulfate. The solvent was evaporated in vacuo. The obtained residue was purified by silica gel column chromatography to give compound (I-15) (0.1 g).
¹H-NMR (CDCl₃) δ:1.97(3H,s),3.78-3.80(12H,m),4.35-4.45(6H,m),6.40-6.45(4H,m),6.73-6.88(7H,m),7.01-7.13(6H,m).

### Fourth step

To compound (I-15) (0.1 g) was added trifluoroacetic acid (1.5 ml), the mixture was heated under reflux for 8 hours. Water and potassium carbonate were added to the mixture and the mixture was extracted with ethyl acetate. The organic layer was dried over anhydrous sodium sulfate. The solvent was evaporated in vacuo. The obtained residue was purified by silica gel column chromatography to give compound (I-16) (0.01 g).
¹H-NMR (CDCl₃) δ: 1.94(3H,s),6.43-6.51(1H,m),6.73-6.79(2H,m),6.89(1H,d,J=8.7Hz),7.21-7.31(2H,m).

### Example 3

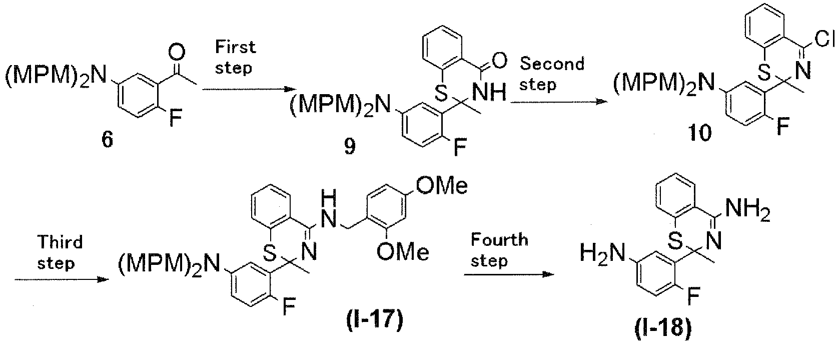

### First step

To a solution of compound 6 (39.61 g) in toluene (594 ml) were added p-toluene sulfonic acid (1.91 g) and 2-mercaptobenzamide (18.51g). It was heated under reflux for 15 hours being removed generated water. After water was added to the mixture, the mixture was extracted with ethyl acetate. The organic layer was dried over anhydrous sodium sulfate. The obtained residue was purified by silica gel column chromatography to give compound 9 (2.9 g). ¹H-NMR (CDCl₃) δ:2.01(3H,s),3.80(6H,s),4.36(4H,s),6.45-6.48(2H,m),6.78-6.80(5H,m),6.90-7.05(6H,m),7.10-7.30(2H,m),7.92(1H,dd,J=7.9,1.5Hz).

### Second step

To compound 9 (0.31 g) was added phosphorus oxychloride (3.1 ml). It was stirred at 80 °C for 15 hours. The phosphorus oxychloride was evaporated in vacuo. After the evaporation, the obtained residue was poured into ice water, and the mixture was extracted with ethyl acetate and dried over anhydrous sodium sulfate. The solvent was evaporated in vacuo to give compound 10 as a crude product.

### Third step

To a solution of compound 10 obtained in Second step in toluene (1.6 ml) was added 2-4-dimethoxybenzylamine (0.49 g) at room temperature. It was heated under reflux for 2.5 hours. Water was added to the mixture and the mixture was extracted with ethyl acetate. The organic layer was dried over anhydrous sodium sulfate. The solvent was evaporated in vacuo. The obtained residue was purified by silica gel column chromatography to give compound (I-17) (0.27 g).
¹H-NMR (CDCl₃) δ:1.88(3H,s),3.72-3.78(12H,m),4.33-4.38(6H,m),6.38-6.48(4H,m).6.78-6.82(5H,m),7.01-7.41(10H,m).

### Fourth step

To compound (I-17) (0.27 g) was added trifluoroacetic acid (1.4 ml), the mixture was heated under reflux for 3 hours. Water and potassium carbonate were added to the mixture , and the mixture was extracted with ethyl acetate. The organic layer was dried over anhydrous sodium sulfate. The solvent was evaporated in vacuo. The obtained residue was purified by silica gel column chromatography to give compound (I-18) (0.04 g).
¹H-NMR (CDCl₃)
δ:1.91(3H,d,J=1.2Hz),6.47(1H,ddd,J=8.5,3.7,3.1Hz),6,81(1H,dd,J=11.7,8.5Hz),6.98(1H,dd,J=6.8, 3.0Hz),7.11-7.17(1H,m),7.28-7.30(2H,m),7.55-7.57(1H,m).

The following compounds are prepared in accordance with the above examples. In the tables, RT means a retention time (minutes).

**[Table 1-1]**

| **Comp. No.** | **Structure** | **NMR (solvent, shift value ascending order)** | **MS [M+1]** | **LC/MS RT** | **LC/MS Method** |
|---|---|---|---|---|---|
| I-1 | 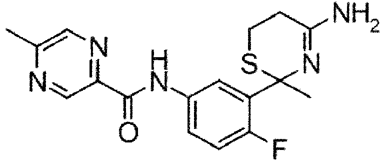 | 1H-NMR (CDCl3) δ : 1.97 (3H, d, J = 1.7 Hz), 2.32-2.49 (2H, m), 2.58 (1H, ddd, J = 13.8, 8.4, 5.5 Hz), 2.71-2.75 (4H. m), 4.44 (2H, s), 7.06 (1 H, dd, J = 11.4, 8.7 Hz), 7.49 (1H, dd, J = 8.7, 2.7 Hz), 7.76 (1H, ddd, J = 8.7, 3.9, 2.7 Hz), 8.42 (1H, d, J = 0.9 Hz), 9.35 (1H, d, J = 0.9 Hz), 9.57 (1H, s). | | | |
| I-2 | 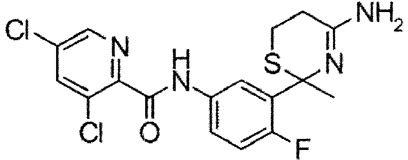 | | 413 | 1.06 | A |
| I-3 | 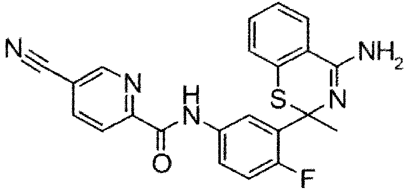 | | 418 | 1.31 | B |
| I-4 | 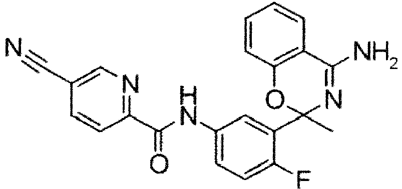 | | 402 | 1.32 | B |

**[Table 1-2]**

| **Comp. No.** | **Structure** | **NMR (solvent, shift value, ascendong order)** | **MS [M+1]** | **LC/MS RT** | **LC/MS Method** |
|---|---|---|---|---|---|
| I-5 | 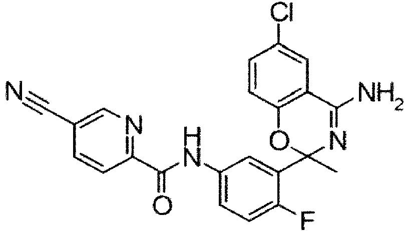 | ¹H-NMR (CDCl₃) δ : 1.94 (3H, s), 6.43-6.51 (1H, m), 6.73-6.79 (2H, m), 6.89 (1H, d, *J* = 8.7 Hz), 7.21-7.31 (2H, m). | 436 | 1.24 | A |
| I-6 | 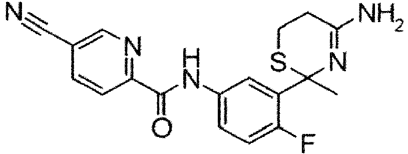 | | 370 | 1.09 | A |
| I-7 | 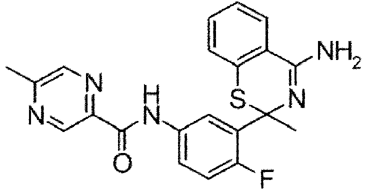 | | 408 | 1.25 | B |
| I-8 | 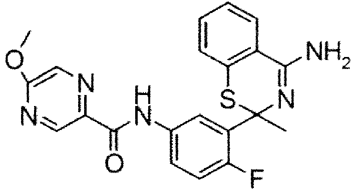 | | 424 | 1.36 | B |
| I-9 | 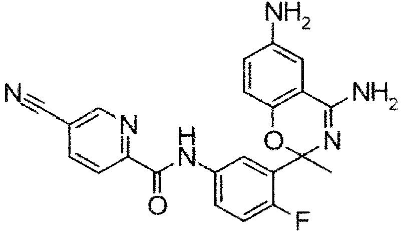 | | 417 | 1.14 | B |

**[Table 1-3]**

| **Comp. No.** | **Structure** | **NMR (solvent,shift value ascendong order)** | **MS [M+1]** | **I LC/MS RT** | **LC/MS Method** |
|---|---|---|---|---|---|
| **I-10** | 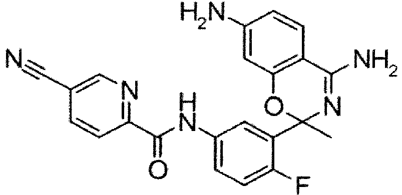 | | 417 | 1.13 | B |
| **I-11** | 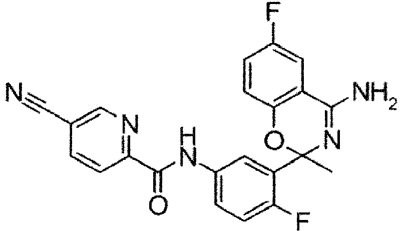 | 1H-NMR (CDCl3) δ :1.97(3H, s), 4.86(2H, bs), 6.96-7.25(4H, m), 7.69(1H, dd, J = 6.6, 2.7Hz), 7.80-7.83(1H, m), 8.18(1H, dd, J = 8.1, 2.1Hz),8.40(1H,dd.J = 8.1, 0.6Hz), 8.87(1H, J = 1.2Hz), 9.75(1H, bs). | 421 | 1.2 | A |
| I.12 | 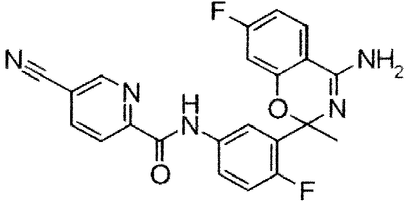 | 1H-NMR (CDCl3) δ :1.99(3H, d, J = 0.9Hz), 6.63(1H, dt, J = 2.7, 8.7Hz), 6.73(1H, dd, J = 9.9. 2.7Hz), 7.07 (1H, dd, J = 11.1, 9.0Hz), 7.30(1H, dd, J = 6.9, 2.7Hz), 7.71(1H, dd, J = 6.9, 2.7Hz), 7.80-7.86(1H, m). 8.19(1H, dd, J = 8. 1, 2.1Hz), 8.41(1H, d, J = 7.2Hz), 8.88(1H, s), 9.77(1H, bs). | 421 | 1.25 | A |

**[Table 1-4]**

| **Comp. No.** | **Structure** | **NMR (solvent shift value: ascendong order)** | **MS [M+1]** | **LC/MS RT** | **LC/MS Method** |
|---|---|---|---|---|---|
| I-13 | 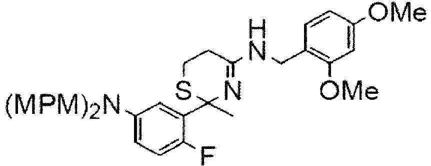 | ¹H-NMR (CDCl₃) δ : 1.95 (3H, d, *J* = 2.0 Hz), 2.00-2.11 (2H, m), 2.31-2.35 (2H, m), 3.78-3.81 (14H, m), 4.22-4.52 (5H, m), 6.41-6.49 (4H, m),6.79-6.83 (5H, m), 7.05 (4H, d, *J* = 8.7 Hz), 7.19-7.26 (1H, m). | | | |
| I-14 | 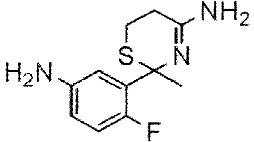 | 1H-NMR (CDCl₃) δ: 1.92 (3H, d, *J* = 1.7 Hz), 2.26-2.45 (2H, m), 2.57 (1H, ddd, *J* = 13.8, 9.1, 4.8 Hz), 2.70 (1H, ddd, *J* = 13.8, 5.7, 4.8 Hz), 3.50 (2H, s), 6.50 (1H, dt, *J* = 8.5, 3.3 Hz), 6.58 (1H, dd, *J* = 7.0, 3.3 Hz), 6.82 (1H, dd, *J* = 11.8, 8.5 Hz). | | | |
| I-15 | 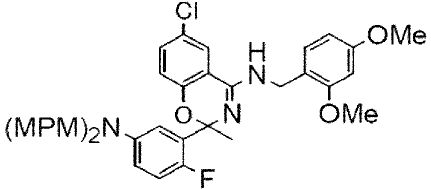 | ¹H-NMR (CDCl₃) δ: 1.97 (3H, s), 3.78-3.80 (12H, m), 4.35-4.45 (6H, m), 6.40-6.45 (4H, m), 6.73-6.88 (7H, m), 7.01-7.13 (6H, m). | | | |
| 1-16 | 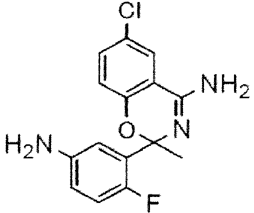 | ¹H-NMR (CDCl₃) *δ* : 1.94 (3H, s), 6.43-6.51 (1H, m), 6.73-6.79 (2H. m), 6.89 (1H, d, *J* = 8.7 Hz), 7.21-7.31 (2H, m). | | | |

**[Table 1-5]**

| **Comp. No.** | **Structure** | **NMR (solvent, shift value ascendong order)** | **MS [M+1]** | **LC/MS RT** | **LC/MS Method** |
|---|---|---|---|---|---|
| I-17 | 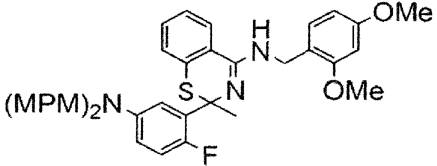 | ¹H-NMR (CDCl₃) δ : 1.88 (3H, s), 3.72-3.78 (12H, m), 4.33-4.38 (6H, m), 6.38-6.48 (4H, m), 6.78-6.82 (5H, m), 7.01-7.41 (10H, m). | | | |
| I-18 | 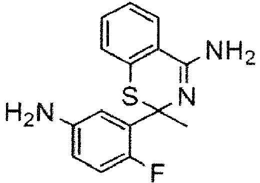 | ¹H-NMR (CDCl₃) δ : 1.91 (3H, d, *J* = 1.2 Hz), 6.47 (1H, ddd. *J* = 8.5, 3.7, 3.1 Hz), 6.81 (1H, dd, *J* = 11.7,8.5 Hz), 6.98 (1H, dd, *J* = 6.8, 3.0 Hz), 7.11-7.17 (1H, m), 7.28-7.30 (2H, m). 7.55-7.57 (1H, m). | | | |
| I-19 | 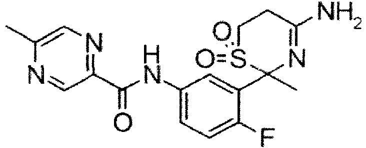 | | 392 | 0.7 | A |

The effect of the present compound is confirmed by the following test Examples.

### (Test Example 1: Assay of BACE1 inhibiting activity)

48.5 µL of substrate peptide solution (Biotin-XSEVNLDAEFRHDSGC-Eu: X=ε-amino-n-capronic acid, Eu=Europium cryptate) was added to each well of 96-hole half-area plate (a black plate: Costar), and after addition of 0.5 µl of the test compound (dissolved in N,N'-dimethylformaldehyde) and 1 µl of Recombinant human BACE1(R&D Systems), the reaction mixture was incubated at 30°C for 3 hours. The substrate peptide was synthesized by reacting Cryptate TBPCOOH mono SMP (CIS bio international) with Biotin-XSEVNLDAEFRHDSGC (Peptide Institute, Inc.). The final concentrations of the substrate peptide and Recombinant human BACK1 were adjusted to 18 nmol/L and 7.4 nmol/L, respectively, and the reaction was performed in sodium acetate buffer (50 mmol/L sodium acetate, pH 5.0, 0.008% Triton X-100). After the incubation for reaction, 50 µl of 8.0 µg/ml Streptavidin-XL665 (CIS bio international) dissolved in phosphate buffer (150 mmol/L K₂ HPO₄ -KH₂ PO₄, pH 7.0, 0.008 % Triton X-100, 0.8 mol/L KF) was added to each well and left stand at 30°C for an hour. After then, fluorescence intensity was measured (excitation wavelength: 320 nm, measuring wavelength: 620 nm and 665 nm) using Wallac 1420 multilabel counter (Perkin Elmer life sciences). Enzymatic activity was determined from counting ratio of each wavelength (10,000 x Count 665/Count 620) and 50% inhibitory concentration (IC₅₀) against the enzymatic activity was calculated.

### (Result)

**[Table 2]**

| **Comp. No.** | **Test example 1 IC50(*µ* M)** |
|---|---|
| I-2 | 0.010 |
| I-8 | 0.066 |
| I-12 | 0.061 |

Compounds I-1, 3 to 7 and 9 to 11 showed the IC ₅₀ value of 1µM or less.

### (Test Example 2: Measurement of (β-amyloid (Aβ) production inhibitory effect in cell)

Neuroblastoma SH-SY5Y cells (SH/APPwt) with human wild-type β-APP excessively expressed therein were prepared at 8 X 10⁵ cells/mL, and 150 µl portions thereof were inoculated into each well of a 96-well culture plate (Falcon). The cells were cultured for 2 hours at 37°C in a 5% gaseous carbon dioxide incubator. Then, a solution which had been preliminarily prepared by adding and suspending the test compound (DMSO (dimethyl sulfoxide) solution) so as to be 2 µl/50 µl medium was added to the cell sap. Namely, the final DMSO concentration was 1%, and the amount of the cell culture was 200 µl. After the incubation was performed for 24 hours from the addition of the test compound, 100 µl of the culture supernatant was collected from each fraction. The amount of the Aβ in each fraction was measured.

The Aβ amount was measured as follows. 10 µl of a homogeneous time resolved fluorescence (HTRF) measurement reagent (Amyloid β 1-40 peptide; IBA Molecular Holding, S.A.) and 10 µl of the culture supernatant were put into a 384-well half area microplate (black microplate, Costar) and mixed with each other, and then left standing overnight at 4°C while the light was shielded. Then, the fluorescence intensity (excitation wavelength: 337 nm, measurement wavelength: 620 nm and 665 nm) was measured with a Wallac 1420 multilabel counter (Perkin Elmer life sciences). The Aβ amount was determined from the count rate at each measurement wavelength (10000 X Count 665/Count 620), and the amount needed to inhibit Aβ production by 50 % (IC₅₀) was calculated from at least six different dosages. Table 3 shows the IC₅₀ value of each test compound.

### (Result)

**[Table 3]**

| **Comp. No.** | **Test example 2 IC50(*µ* M)** |
|---|---|
| I-2 | <0.001 |
| I-8 | 0.019 |
| I-12 | 0.015 |

Compounds I-1, 3 to 7, 9 to 11 and 19 showed the IC ₅₀ value of 1µM or less.

### (Test Example 3: Lowering effect on brain β amyloid in rats)

A test compound was suspended in 0.5% methylcellulose, the final concentration was adjusted to 2 mg/mL, and this was orally administered to male Crj:SD rat (7 to 9 weeks old) at 10 mg/kg. In a vehicle control group, only 0.5% methylcellulose was administered, and an administration test was performed at 3 to 8 animals per group. A brain was isolated 3 hours after administration, a cerebral hemisphere was isolated, a weight thereof was measured, the hemisphere was rapidly frozen in liquid nitrogen, and stored at -80°C until extraction date. The frozen cerebral hemisphere was transferred to a homogenizer manufactured by Teflon (registered trade name) under ice cooling, a 5-fold volume of a weight of an extraction buffer (containing 1% CHAPS ({3-[(3-chloroamidopropyl)dimethylammonio]-1-propanesulfonate}), 20 mmol/L Tris-HCl (pH 8.0), 150 mmol/L NaCl, Complete (Roche) protease inhibitor) was added, up and down movement was repeated, and this was homogenized to solubilize for 2 minutes. The suspension was transferred to a centrifugation tube, allowed to stand on an ice for 3 hours or more and, thereafter centrifuged at 100,000 x g, 4°C for 20 minutes. After centrifugation, the supernatant was transferred to an ELISA plate (product No. 294-62501, Wako Junyaku Kogyo) for measuring β amyloid 40. ELISA measurement was performed according to the attached instruction. The lowering effect was calculated as a ratio compared to the brain β amyloid 40 level of vehicle control group of each test.

### (Test Example 4: CYP3A4 fluorescent MBI test)

The CYP3A4 fluorescent MBI test is a test of investigating enhancement of CYP3A4 inhibition of a compound by a metabolism reaction, and the test was performed using, as CYP3A4 enzyme expressed in *Escherichia coli* and employing, as an index, a reaction in which 7-benzyloxytrifluoromethylchmarin (7-BFC) is debenzylated by the CYP3A4 enzyme to produce a metabolite, 7-hydroxytrifluoromethylchmarin (HFC) emitting fluorescent light.

The reaction conditions were as follows: substrate, 5.6 µmol/L 7-BFC; pre-reaction time, 0 or 30 minutes; reaction time, 15 minutes; reaction temperature, 25°C (room temperature); CYP3A4 content (expressed in *Escherichia coli*)*,* at pre-reaction 62.5 pmol/mL, at reaction 6.25 pmol/mL (at 10-fold dilution); test drug concentration, 0.625, 1.25, 2.5, 5, 10, 20 µmol/L (six points).

An enzyme in a K-Pi buffer (pH 7.4) and a test drug solution as a pre-reaction solution were added to a 96-well plate at the composition of the pre-reaction, a part of it was transferred to another 96-well plate so that it was 1/10 diluted by a substrate in a K-Pi buffer, NADPH as a cofactor was added to initiate a reaction as an index (without preincubation) and, after a predetermined time of a reaction, acetonitrile/0.5 mol/L Tris (trishydroxyaminomethane) = 4/1 was added to stop the reaction. In addition, NADPH was added to a remaining preincubation solution to initiate a preincubation (with preincubation) and, after a predetermined time of a preincubation, a part was transferred to another plate so that it was 1/10 diluted with a substrate and a K-Pi buffer to initiate a reaction as an index. After a predetermined time of a reaction, acetonitrile/0.5 mol/L Tris (trishydroxyaminomethane) = 4/1 was added to stop the reaction. For the plate on which each index reaction had been performed, a fluorescent value of 7-HFC which is a metabolite was measured with a fluorescent plate reader. (Ex = 420 nm, Em = 535 nm).

Addition of only DMSO which is a solvent dissolving a drug to a reaction system was adopted as a control (100%), remaining activity (%) was calculated at each concentration of a test drug added as the solution,and IC₅₀ was calculated by reverse-presumption by a logistic model using a concentration and an inhibition rate. When a difference between IC₅₀ values is 5 µM or more, this was defined as (+) and, when the difference is 3 µM or less, this was defined as (-).

### (Result)

### Compound I-4: (-)

### (Test Example 5: CYP inhibition test)

Using commercially available pooled human hepatic microsome, and employing, as markers, O-deethylation of 7-ethoxyresorufin (CYP1A2), methyl-hydroxylation of tolbutamide (CYP2C9), 4'-hydroxylation of mephenytoin (CYP2C19), O-demethylation of dextromethorphan (CYP2D6) and hydroxylation of terfenadine (CYP3A4) as typical substrate metabolism reactions of human main five CYP enzyme forms (CYP1A2, 2C9, 2C19, 2D6, 3A4), an inhibitory degree of each metabolite production amount by a test compound was assessed.

The reaction conditions were as follows: substrate, 0.5 µmol/L ethoxyresorufin (CYP1A2), 100 µmol/L tolbutamide (CYP2C9), 50 µmol/L S-mephenitoin (CYP2C19), 5 µmol/L dextromethorphan (CYP2D6), 1 µmo1/L terfenadine (CYP3A4); reaction time, 15 minutes; reaction temperature, 37°C; enzyme, pooled human hepatic microsome 0.2 mg protein/mL; test drug concentration, 1, 5, 10, 20 µmol/L (four points).

Each five kinds of substrates, human hepatic microsome, and a test drug in 50 mmol/L Hepes buffer as a reaction solution was added to a 96-well plate at the composition as described above, NADPH, as a cofactor was added to initiate metabolism reactions as markers and, after the incubation at 37°C for 15 minutes, a methanol/acetonitrile = 1/1 (v/v) solution was added to stop the reaction. After the centrifugation at 3000 rpm for 15 minutes, resorufin (CYP1A2 metabolite) in the supernatant was quantified by a fluorescent multilabel counter and tributamide hydroxide (CYP2CP metabolite), mephenytoin 4' hydroxide (CYP2C19 metabolite), dextromethorphan (CYP2D6 metabolite), and terfenadine alcohol (CYP3A4 metabolite) were quantified by LC/MS/MS.

Addition of only DMSO being a solvent dissolving a drug to a reaction system was adopted as a control (100%), remaining activity (%) was calculated at each concentration of a test drug added as the solution and IC₅₀ was calculated by reverse presumption by a logistic model using a concentration and an inhibition rate.

### (Result)

### Compound I-19: five kinds > 17 µM

### (Test Example 8: FAT Test)

Each 20 µL of freeze-stored *Salmonella typhimurium* (TA98 and TA100 strain) is inoculated in 10 mL of liquid nutrient medium (2.5% Oxoid nutrient broth No.2), and the cultures are incubated at 37°C under shaking for 10 hours. 9 mL of TA98 culture is centrifuged (2000 x g, 10 minutes) to remove medium, and the bacteria is suspended in 9 mL of Micro F buffer (K₂HPO₄: 3.5 g/L, KH₂PO₄: 1 g/L, (NH₄)₂SO₄: 1 g/L, trisodium citrate dihydrate: 0.25 g/L, MgSO₄ · 7H₂O: 0.1 g/L), and the suspension is added to 110 mL of Exposure medium (Micro F buffer containing Biotin: 8 µg/mL, histidine: 0.2 µg/mL, glucose: 8 mg/mL). 3.16 mL of TA100 culture is added to 120 mL of Exposure medium to prepare the test bacterial solution. 588 µL of the test bacterial solution (or mixed solution of 498 µl of the test bacterial solution and 90 µL of the S9 mix in the case with metabolic activation system) are mixed with each 12 µL of the following solution: DMSO solution of the test substance (eight dose levels from maximum dose 50 mg/mL at 2-fold ratio); DMSO as negative control; 50 µg/mL of 4-nitroquinoline-1-oxide DMSO solution as positive control for TA98 without metabolic activation system; 0.25 µg/mL of 2-(2-furyl)-3-(5-nitro-2-furyl)acrylamide DMSO solution as positive control for TA100 without metabolic activation system; 40 µg/mL of 2-aminoanthracene DMSO solution as positive control for TA98 with metabolic activation system; or 20 µg/mL of 2-aminoanthracene DMSO solution as positive control for TA100 with metabolic activation system. 12 µL of the solution and 588 µL of the test bacterial solution (a mixed solution of 498 µl of the test bacterial solution and 90 µL of S9 mix with metabolic activation condition) were mixed and incubated at 37°C under shaking for 90 minutes. 460 µL of the bacterial solution exposed to the test substance is mixed with 2300 µL of Indicator medium (Micro F buffer containing biotin: 8 µg/mL, histidine: 0.2 µg/mL, glucose: 8 mg/mL, Bromo Cresol Purple: 37.5 µg/mL), each 50 µL is dispensed into 48 wells per dose in the microwell plates, and is subjected to stationary cultivation at 37°C for 3 days. A well containing the bacteria, which has obtained the ability of proliferation by mutation in the gene coding amino acid (histidine) synthetase, turns the color from purple to yellow due to pH change. The number of the yellow wells among the 48 total wells per dose is counted and evaluated in comparing that of the negative control group. (-) means that mutagenicity is negative and (+) means that mutagenicity is positive.

### (Test Example 7(1): Solubility Test)

A 2-fold dilution series (12 points) of a 10 mM solution of a test compound in DMSO was added to a medium (JP-I, JP-II) (2%), and solubility was assessed by 3 stages (High; > 40 µM, Medium; 3-40 µM, Low; < 3 µM) from a turbidity after 4 hours (crystallization information).

### (Result)

### Compound I-9: High

### Test Example 7(2) Solubility test

The solubility of each compound is determined under 1% DMSO addition conditions. A 10 mM solution of the compound is prepared with DMSO, and 6 µL of the compound solution is added to 594 µL of an artificial intestinal juice (water and 118 mL of 0.2 mol/L NaOH reagent are added to 250 mL of 0.2 mol/L potassium dihydrogen phosphate reagent to reach 1000 mL) with a pH of 6.8. The mixture is left standing for 16 hours at 25°C, and the mixture is vacuum-filtered. The filtrate is two-fold diluted with methanol/water = 1/1, and the compound concentration in the filtrate is measured with HPLC or LC/MS/MS by the absolute calibration method.

### (Test Example 8: Metabolism Stability Test)

Using a commercially available pooled human hepatic microsomes, a test compound was reacted for a constant time, a remaining rate was calculated by comparing a reacted sample and an unreacted sample, thereby, a degree of metabolism in liver was assessed.

A reaction was performed (oxidative reaction) at 37°C for 0 minute or 30 minutes in the presence of 1 mmol/L NADPH in 0.2 mL of a buffer (50 mmol/L Tris-HCl pH 7.4, 150 mmol/L potassium chloride, 10 mmol/L magnesium chloride) containing 0.5 mg protein/mL of human liver microsomes. After the reaction, 50 µL of the reaction solution was added to 100 µL of a methanol/acetonitrile = 1/1 (v/v), mixed and centrifuged at 3000 rpm for 15 minutes. The test compound in the supernatant was quantified by LC/MS/MS, and a remaining amount of the test compound after the reaction was calculated, letting a compound amount at 0 minute reaction time to be 100%.

### (Result)

### Compound I-19: 97%

### (Test Example 9: hERG Test)

For the purpose of assessing risk of an electrocardiogram QT interval prolongation, effects on delayed rectifier K+ current (I_{Kr}), which plays an important role in the ventricular repolarization process, is studied using HEK293 cells expressing human ether-a-go-go related gene (hERG) channel.

After a cell is retained at a membrane potential of -80 mV by whole cell patch clamp method using an automated patch clamp system (PatchXpress 7000A, Axon Instruments Inc.), I_{Kr} induced by depolarization pulse stimulation at +40 mV for 2 seconds and, further, repolarization pulse stimulation at -50 mV for 2 seconds was recorded. After the generated current is stabilized, extracellular solution (NaCl: 135 mmol/L, KCl: 5.4 mmol/L, NaH₂PO₄: 0.3 mmol/L, CaCl₂·2H₂O: 1.8 mmol/L, MgCl₂·6H₂O: 1 mmol/L, glucose: 10 mmol/L, HEPES (4-(2-hydroxyethyl)-1-piperazine ethanesulfonic acid): 10 mmol/L, pH=7.4) in which the test compound had been dissolved at an objective concentration is applied to the cell under the room temperature condition for 10 minutes. From the recording I_{Kr}, an absolute value of the tail peak current is measured based on the current value at the resting membrane potential using an analysis software (DataXpress ver. 1, Molecular Devices Corporation). Further, the % inhibition relative to the tail peak current before application of the test substance is calculated, and compared with the vehicle-applied group (0.1% dimethyl sulfoxide solution) to assess influence of the test substance on I_{Kr}.

### (Test Example 9: Powder solubility test)

Appropriate amounts of the test substances are put into appropriate containers. To the respective containers are added 200 µL of JP-1 fluid (sodium chloride 2.0 g, hydrochloric acid 7.0 mL and water to reach 1000 mL), 200 µL of JP-2 fluid (phosphate buffer (pH 6.8) 500 mL and water 500 mL), and 200 µL of 20 mmol/L TCA (sodium taurocholate)/JP-2 fluid (TCA 1.08 g and water to reach 100 mL). In the case that the test compound is dissolved after the addition of the test fluid, the bulk powder is added as appropriate. The containers are sealed, and shaken for 1 hour at 37°C. The mixtures are filtered, and 100 µL of methanol is added to each of the filtrate (100 µL) so that the filtrates are two-fold diluted. The dilution ratio may be changed if necessary. The dilutions are observed for bubbles and precipitates, and then the containers are sealed and shaken. Quantification is performed by HPLC with an absolute calibration method.

### (Test Example 11: BA test)

Materials and methods for studies on oral absorption
(1) Animal: Mouse or Rats
(2) Breeding conditions: mouse or rats were allowed to freely take solid feed and sterilized tap water.
(3) Dose and grouping: orally or intravenously administered at a predetermined dose; grouping was as follows (Dose depends on the compound)
   Oral administration: 1 to 30 mg/kg (n=2 to 3)
   Intravenous administration: 0.5 to 10 mg/kg (n=2 to 3)
(4) Preparation of dosing solution: for oral administration, in a solution or a suspension state; for intravenous administration, in a solubilized state
(5) Administration method: in oral administration, forcedly administer into ventriculus with oral probe; in intravenous administration, administer from caudal vein with a needle-equipped syringe
(6) Evaluation items: blood was collected over time, and the plasma concentration of drug was measured by LC/MS/MS
(7) Statistical analysis: regarding the transition of the plasma concentration, the area under the plasma concentration-time curve (AUC) was calculated by non-linear least squares program WinNonlin (Registered trade name), and the bioavailability (BA) was calculated from the AUCs of the oral administration group and intravenous administration group

### (Result)

### Compound I-1: 93.4

### (Test Example 12: Brain distribution studies)

Intravenous administration is carried out to a rat by 0.5 mg/mL/kg dosage of a text compound. 30 minutes later, all blood is drawn from vena cava inferior under isoflurane anesthesia for death from exsanguination. Then, the brain was extracted and 20-25% of homogenate thereof was prepared with distilled water. On the other hand, the obtained blood is used as plasma after centrifuging. Then, to the brain sample was added the control plasma at 1:1. To the plasma samples was added the control brains at 1:1. Each sample was measured using LC/MS/MS. The obtained area ratio (a brain/plasma) was used for the brain Kp value.

### (Result)

### Compound I-11: 5.8.

### Formulation Example 1

A granule containing the following ingredients is produced.

| | | |
|---|---|---|
| Ingredient | Compound of formula (I) | 10 mg |
| | Lactose | 700 mg |
| | Corn starch | 274 mg |
| | HPC-L | 16 mg |
| | | 1000 mg |

The compound of formula (I), and lactose are passed through a 60 mesh sieve. Corn starch is passed through a 120 mesh sieve. These are mixed with a V-type mixer. A HPC-L (low viscosity hydroxypropylcellulose) aqueous solution is added. To the mixed powder is added a HPC-L (low viscosity hydroxypropylcellulose) aqueous solution, this is kneaded, granulated (extrusion granulation, pore diameter 0.5 to 1 mm), and dried. The resulting dry granule is passed through a vibration sieve (12/60 mesh) to obtain a granule.

### Formulation Example 2

A granule for filling a capsule containing the following ingredients is produced.

| | | |
|---|---|---|
| Ingredient | Compound of formula (I) | 15 mg |
| | Lactose | 90 mg |
| | Corn starch | 42 mg |
| | HPC-L | 3 mg |
| | | 150 mg |

The compound of formula (I), and lactose are passed through a 60 mesh sieve. Corn starch is passed through a 120 mesh sieve. These are mixed, a HPC-L solution is added to the mixed powder, this is kneaded, granulated, and dried. The resulting dry granule is adjusted in a size, and 150 mg of it is filled into a No.4 hard gelatin capsule.

### Formulation Example 3

A tablet containing the following ingredients is produced.

| | | |
|---|---|---|
| Ingredient | Compound of formula (I) | 10 mg |
| | Lactose | 90 mg |
| | Microcrystalline cellulose | w30 mg |
| | CMC-Na | 15 mg |
| | Magnesium stearate | 5 mg |
| | | 150 mg |

The compound of formula (I), lactose, microcrystalline cellulose, and CMC-Na (carboxymethylcellulose sodium salt) are passed through a 60 mesh sieve, and mixed. Magnesium stearate is mixed into the mixed powder to obtain a mixed powder for tabletting. The present mixed powder is directly compressed to obtain a 150 mg of a tablet.

### Formulation Example 4

The following ingredients are warmed, mixed, and sterilized to obtain an injectable.

| | | |
|---|---|---|
| Ingredient | Compound of formula (I) | 3 mg |
| | Nonionic surfactant | 15 mg |
| Purified water for injection | | 1 ml |

### [Industrial applicability]

The present compound can be a medicament useful as an agent for treating a disease induced by production, secretion and/or deposition of amyloid β protein.

## Claims

1. A compound of formula (I): 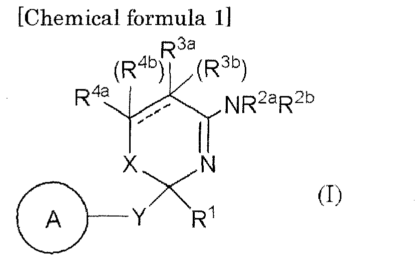 wherein
-X- is -O-, -S-, -SO-, -SO₂- or -N(R^{x})-,
R^{x} is hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl,
ring A is a substituted or unsubstituted carbocycle or a substituted or unsubstituted heterocycle, -Y- is substituted or unsubstituted alkenylene, substituted or unsubstituted alkynylene, (CR⁵R⁶)ₙ-, -(CR⁵R⁶)ₙO(CR⁵R⁶)ₘ-, -(CR⁵R⁶)ₙS(CR⁵R⁶)ₘ-, -(CR⁵R⁶)ₙSO₂(CR⁵R⁶)ₘ-, - (CR⁵R⁶)ₙSO(CR⁵R⁶)ₘ-, -(CR⁵R⁶)ₙC(=O)(CR⁵R⁶)ₘ-, -(CR⁵R⁶)ₙN(R⁷)(CR⁵R⁶)ₘ-, - (CR⁵R⁶)ₙ(C=O)N(R⁷)(CR⁵R⁶)ₘ-, or -(CR⁵R⁶)ₙN(R⁷)C(=O)(CR⁵R⁶)ₘ-,
R⁵ and R⁶ are each independently, hydrogen, halogen, hydroxy, nitro, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted carbamoyloxy, substituted or unsubstituted thiocarbamoyl, substituted or unsubstituted sulfamoyl, substituted or unsubstituted aminosulfinyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl,
R⁷ is hydrogen, substituted or unsubstituted alkyl, substituted or unsubstituted alkenyl, substituted or unsubstituted alkynyl or substituted or unsubstituted acyl,
n and m are each independently an integer of 0 to 3,
R¹ is substituted or unsubstituted alkyl, substituted or unsubstituted alkenyl, substituted or unsubstituted alkynyl, substituted or unsubstituted acyl, cyano, carboxy, substituted or unsubstituted alkoxycarbonyl, substituted or unsubstituted alkenyloxycarbonyl, substituted or unsubstituted alkynyloxycarbonyl, substituted or unsubstituted carbamoyl, substituted or unsubstituted thiocarbamoyl, a substituted or unsubstituted carbocyclic group or a substituted or unsubstituted heterocyclic group,
R^{2a} and R^{2b} are each independently hydrogen, substituted or unsubstituted alkyl, substituted or unsubstituted acyl, substituted or unsubstituted alkoxycarbonyl or substituted or unsubstituted carbamoyl,
R^{3a}, R^{3b}, R^{4a} and R^{4b} are each independently
hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl,
R^{3a} and R^{3b} together with the carbon atom to which they are attached may form a substituted or unsubstituted carbocycle or a substituted or unsubstituted heterocycle,
R^{4a} and R^{4b} together with the carbon atom to which they are attached may form a substituted or unsubstituted carbocycle or a substituted or unsubstituted heterocycle,
R^{3a} and R^{4a} together with the carbon atom to which they are attached may form a substituted or unsubstituted carbocycle or a substituted or unsubstituted heterocycle,
dashed line means the presence or absence of a bond,
when dashed line means the presence of a bond, then R^{3b} and R^{4b} are absent,
provided that when -X- is -O- or -N(R^{x})-, and R^{3a} and R^{4a} together with the carbon atom to which they are attached form a substituted or unsubstituted benzene ring, then ring A is not (i) phenyl substituted only with halogen or (ii) phenyl substituted with substituted or unsubstituted phenyl at m-position,
its pharmaceutically acceptable salt or a solvate thereof.

2. The compound according to claim 1 wherein Y is -(CR⁵R⁶)ₙ- and n is 0, its pharmaceutically acceptable salt or a solvate thereof.

3. The compound according to claim 2 wherein -X- is -O- or -S-, its pharmaceutically acceptable salt or a solvate thereof.

4. The compound according to any one of claims 1 to 3 wherein ring A is a group of formula: 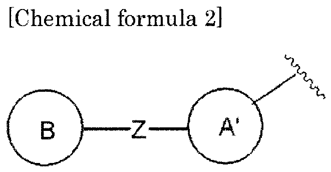
wherein ring A' and ring B are each independently a substituted or unsubstituted carbocycle or a substituted or unsubstituted heterocycle,
-Z- is -L¹-C(=O)N(R⁸)-L²-, -L¹-N(R⁸)C(=O)-L²- or -L¹-N(R⁸)-L²-,
L¹ and L² are each independently a bond, substituted or unsubstituted alkylene, substituted or unsubstituted alkenylene or substituted or unsubstituted alkynylene,
R⁸ is hydrogen, substituted or unsubstituted alkyl, substituted or unsubstituted alkenyl, substituted or unsubstituted alkynyl or substituted or unsubstituted acyl,
its pharmaceutically acceptable salt or a solvate thereof.

5. The compound according to claim 4 wherein -Z- is -C(=O)N(R⁸)-, its pharmaceutically acceptable salt or a solvate thereof.

6. The compound according to claim 4 or 5 wherein ring A' is substituted or unsubstituted benzene, its pharmaceutically acceptable salt or a solvate thereof.

7. The compound according to any one of claims 4 to 6 wherein ring B is substituted or unsubstituted pyridine, substituted or unsubstituted pyrimidine or substituted or unsubstituted pyrazine, its pharmaceutically acceptable salt or a solvate thereof.

8. The compound according to any one of claims 1 to 7 wherein R^{3a}, R^{3b}, R^{4a} and R^{4b} are all hydrogen, its pharmaceutically acceptable salt or a solvate thereof.

9. The compound according to any one of claims 1 to 7 wherein R^{3a} and R^{4a} together with the carbon atom to which they are attached form a substituted or unsubstituted benzene ring, its pharmaceutically acceptable salt or a solvate thereof.

10. The compound according to any one of claims 1 to 9 wherein R¹ is C1 to C3 unsubstituted alkyl, its pharmaceutically acceptable salt or a solvate thereof.

11. The compound according to any one of claims 1 to 10 wherein R^{2a} and R^{2b} are both hydrogen, its pharmaceutically acceptable salt or a solvate thereof.

12. A pharmaceutical composition comprising the compound according to any one of claims 1 to 11, its pharmaceutically acceptable salt or a solvate thereof as an active ingredient.

13. A pharmaceutical composition having BACE1 inhibitory activity comprising the compound according to any one of claims 1 to 11, its pharmaceutically acceptable salt or a solvate thereof as an active ingredient.

14. The pharmaceutical composition according to claim 12 or 13, which is a medicament for treating or preventing the diseases induced by production, secretion or deposition of amyloid-β proteins.

15. The pharmaceutical composition according to claim 12 or 13, which is a medicament for treating or preventing Alzheimer's disease.

16. A method for inhibiting BACE 1 activity comprising administering the compound according to any one of claims 1 to 11, its pharmaceutically acceptable salt or a solvate thereof.

17. The compound according to any one of claims 1 to 11, its pharmaceutically acceptable salt or a solvate thereof for use in a method for inhibiting BACK1 activity.

18. A method for treating or preventing diseases induced by production, secretion or deposition of amyloid-β proteins comprising administering the compound according to any one of claims 1 to 11, its pharmaceutically acceptable salt or a solvate thereof.

19. The compound according to any one of claims 1 to 11, its pharmaceutically acceptable salt or a solvate thereof for use in a method for treating or preventing diseases induced by production, secretion or deposition of amyloid-β proteins.

20. A method for treating or preventing Alzheimer's disease comprising administering the compound according to any one of claims 1 to 11, its pharmaceutically acceptable salt or a solvate thereof.

21. The compound according to any one of claims 1 to 11, its pharmaceutically acceptable salt or a solvate thereof for use in a method for treating or preventing Alzheimer's disease.
